# EUROPEAN PATENT APPLICATION

(11) **EP 4 501 942 A1**
(43) Date of publication of application: **05.02.2025**
(21) Application number: 23382808.6
(22) Date of filing: 02.08.2023
(51) Int. Cl.: C07K 5/062, C07K 5/068, A61K 38/00

(54) **PEPTIDOMIMETICS WITH ANTIVIRAL ACTIVITY**

(71) Applicant: Consejo Superior De Investigaciones Científicas (CSIC), 28006 Madrid (ES)
(72) Inventor: González Muñiz, Rosario, Madrid (ES); Martín Martínez, Mercedes, Madrid (ES); Bonache de Marcos, Mª Angeles, Madrid (ES); Algar Lizana, Sergio, Madrid (ES); Garaigorta de Dios, Urtzi, Madrid (ES); Gastaminza Landart, Pablo, Madrid (ES); Bueno Fernández, Paula, Madrid (ES); Castro Illana, Victoria, Madrid (ES)
(74) Representative: Pons

(57) **Abstract**

Peptidomimetics of formula **I**, wherein the meanings for the various substituents are as disclosed in the description. These compounds are useful for the treatment of viral infections, particularly for SARS-CoV-2 viral infections.

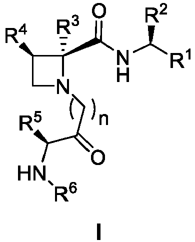

## Description

The invention relates to a peptidomimetic of formula I and to a pharmaceutical composition thereof for its use as a medicament and, particularly, for its use as antiviral, such as for the treatment of SARS-CoV-2 infections.

### BACKGROUND ART

COVID-19 disease caused by the severe acute respiratory syndrome coronavirus-2 (SARS-CoV-2), was declared a pandemic by the World Health Organization (WHO) at the beginning of 2020. The extent of the global pandemic due to COVID-19 has a significant impact on our lives, and the scientific community started looking for vaccines and antiviral to fait against the virus. The SARS-Cov-2 main protease (M^{pro}, or 3CL) plays a pivotal role in processing CoV-encoded polyproteins necessary for replication and transcription, and thus this protease is recognized as an attractive antiviral target (Chellapandi P, et al., Genomics insights of SARS-CoV-2 (COVID-19) into target-based drug discovery. Med. Chem. Res. 2020; 29(10):1777-1791; Gao K, et al., Perspectives on SARS-CoV-2 Main Protease Inhibitors. J. Med. Chem. 2021; 64 (23): 16922-16955; Sergio Algar, et al. SARS-CoV-2 main protease inhibitors: what is moving in the field of peptide and peptidomimetics? J. Pepetide Sci. 2023, e3467).

WO 2021/250648 A1 describes nirmatrelvir antiviral, WO 2021/207632 A1 discloses ritonavir antiviral and WO 2022/119756 A1 describes boceprevir antiviral, all of them conformationally restricted at the central part of the molecule (P2), and useful for the treatment of SARS-CoV-2.

WO 2022/020242 A1 describes new functionalized azepine peptide derivatives useful for the treatment and prevention of SARS-CoV-2 disease by inhibiting M^{pro}.

Accordingly, it would be desirable to have new conformationally restricted amino acid derivatives for P2 which were decorated with suitable moieties to mimic P1 and P3 residues in peptide substrates, which may have potent M^{pro} inhibitory activity and significantly reduced SARS-CoV-2 proliferation in A539-ACE2 cell cultures, with better properties than the antivirals described by the documents cited above.

All viruses, including SARS-CoV-2 that caused COVID-19, change over time, and some of these changes may affect the easy of spread, disease severity, efficacy of vaccines, as well as the development of therapeutic medicines and diagnostic devices. The SARS-CoV-2 virus is unceasingly mutating, especially in the Spike protein, crucial for viral entry, but also some mutants of the M^{pro} disturb Paxlovid effectiveness (Wang L, et al. Rebound after Paxlovid and Molnupiravir during January-June 2022. medRxiv 2022 (preprint); Service RF. Bad News for Paxlovid? Resistance May Be Coming. Science 2022; 377 (6602): 138-139).

Therefore, being able to advance in new antivirals with cross-resistance with nirmatrelvir is a need to address future combats against coronavirus outbreaks.

### SUMMARY OF THE INVENTION

The present invention relates to a series of peptidomimetic molecules able to inhibit the SARS-CoV-2 main protease (M^{pro}, or 3CL) in vitro activity and the viral replication in cell cultures.

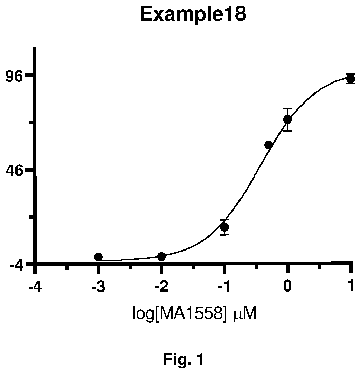

M^{pro} plays a pivotal role in processing CoV-encoded polyproteins necessary for replication and transcription, and thus this protease is recognized as an attractive antiviral target. In fact, one out of two approved drugs to date, nirmatrelvir (figure 1), is a potent inhibitor of SARS-CoV-2 M^{pro}. This drug has a conformationally restricted amino acid derivative at the central part of the molecule (P2).

Accordingly, a first aspect of the present invention relates to a compound of formula **I:** 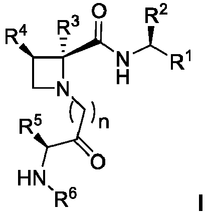 or a pharmaceutical salt thereof, wherein:
n is 0 or 1;
R¹ is a group selected from -CN, -CHO or -CH=CH-COOCH₂Ph;
R² is a -C₁-C₄ alkyl group which is optionally substituted by one -Cy₁;
Cy₁ is a 5 or 6 membered heterocycle, which is saturated, partially saturated, or aromatic, which comprises 1 or 2 heteroatoms in its structure selected from N, O or S, and which is optionally oxidized forming a -CO group in any available position.
R³ is selected from -CH₂-CH(Me)₂, -CH₂-Ph optionally substituted by -OH or -O-tBu, -C₁-C₄ alkyl substituted by -NHBoc or -NH₃Cl, 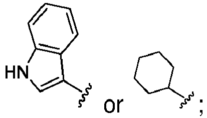
R⁴ is -C₁-C₄ alkyl;
R⁵ is a group selected from -CH(Me)₂, -C(Me)₃ or -C₁-C₄ alkyl substituted by -NHSO₂NHCO₂Me, -NHBoc or -NH₃Cl; and
R⁶ is selected from -COOMe, -COCH₂OPh-Me, -COOCH₂CHF₂, -COCF₃, -COOCH₂Ph, -COOC(Me)₃, 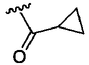 or -CONHC(Me)₃.

All possible stereoisomers and the therapeutically acceptable salts are included in the definition of the compounds of formula **I.**

In another embodiment the invention relates to the compound of formula **I** as defined above, wherein R₂ is selected from a group methyl, ethyl, n-propyl, n-butyl, 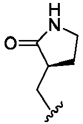 and

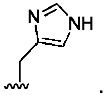

In another embodiment the invention relates to the compound of formula I as defined above, wherein R₄ is a methyl group.

In another embodiment the invention relates to the compound of formula I as defined above, wherein n is 0.

In another embodiment the invention relates to the compound of formula I as defined above, selected from: 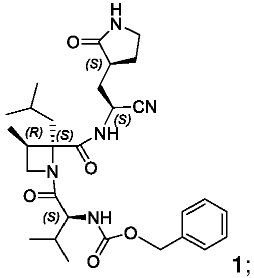 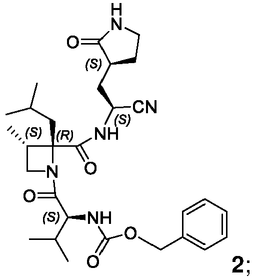 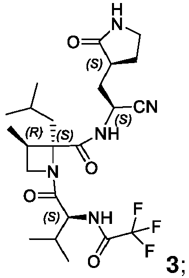 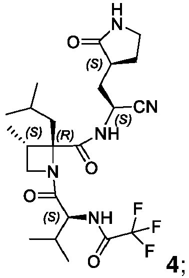 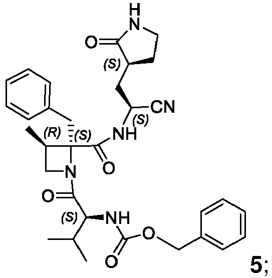 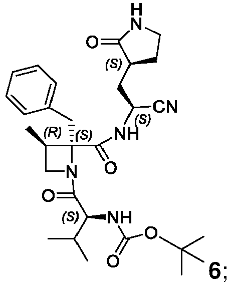 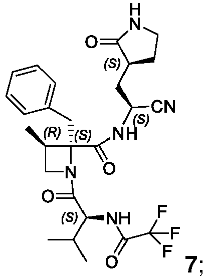 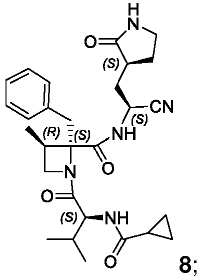 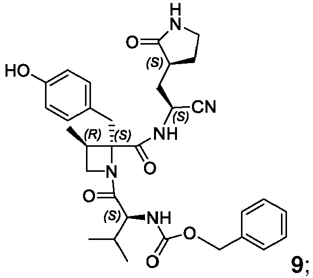 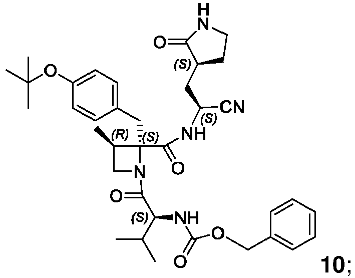 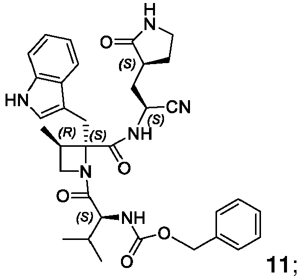 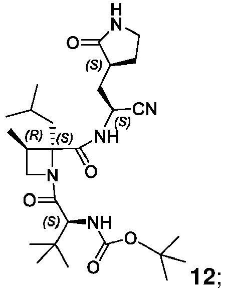 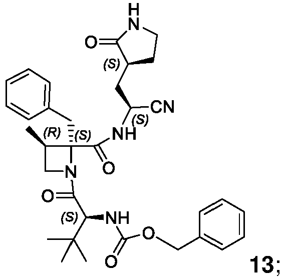 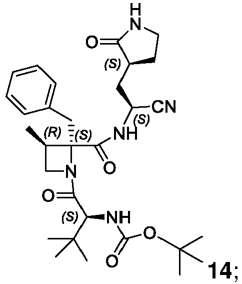 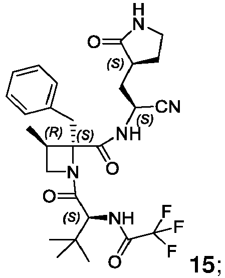 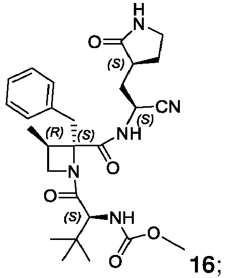 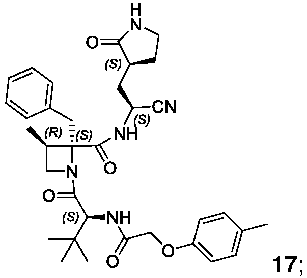 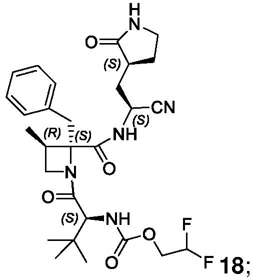 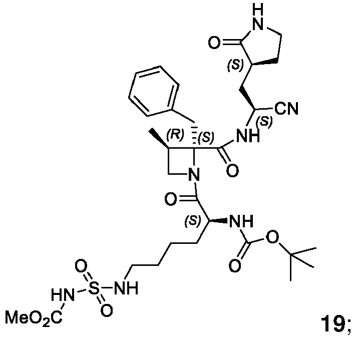 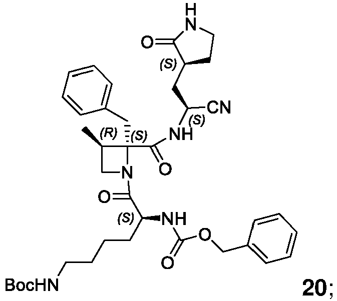 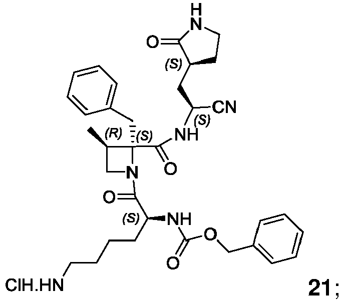 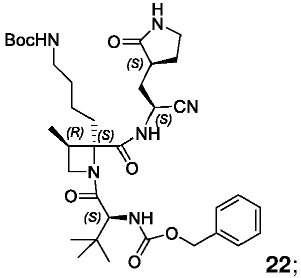 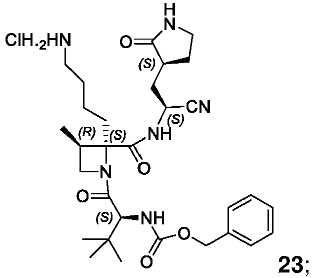 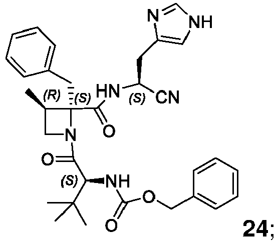 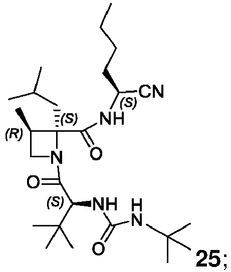 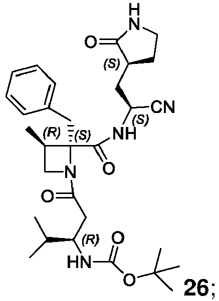 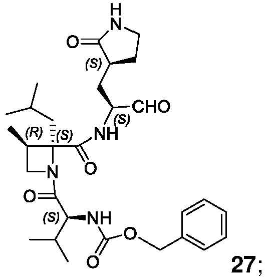 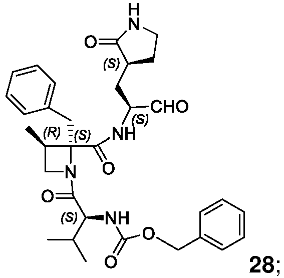 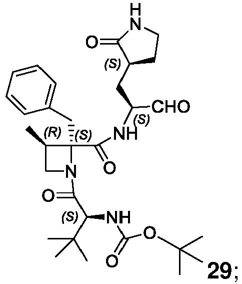 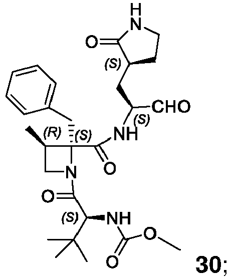 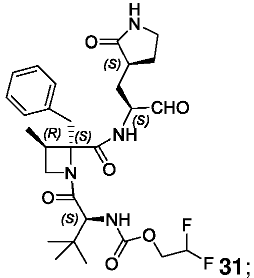 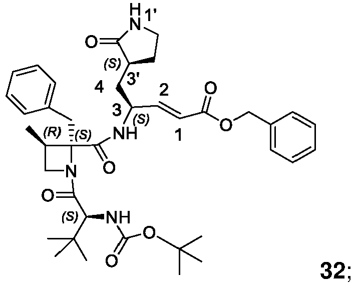 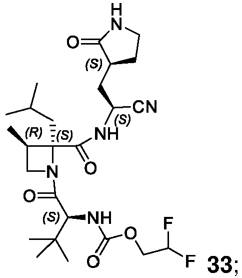 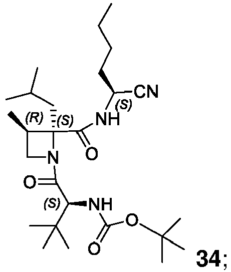 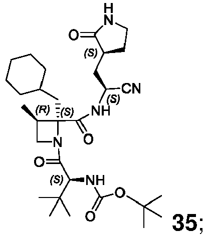 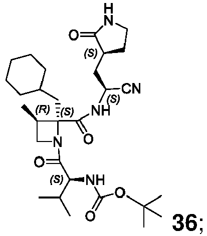 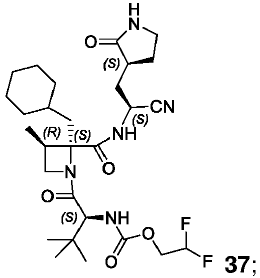 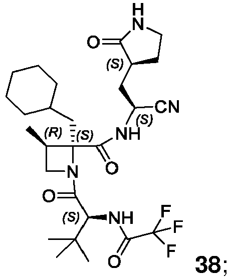 and

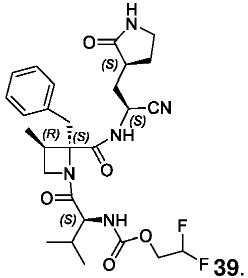

Another aspect of this invention relates to a pharmaceutical composition, which comprises a compound of formula I or a pharmaceutically acceptable salt thereof and one or more pharmaceutically acceptable excipients.

Another aspect of the invention relates to a compound of formula I as defined above, for use as medicament.

Another aspect of the invention relates to a compound of formula I as defined above, for use for the treatment of a viral infection, and preferably for the treatment of SARS-CoV-2 viral infection.

Another aspect of the present invention relates to the use of a compound of formula **I** or a pharmaceutically acceptable salt thereof for the manufacture of a medicament.

Another aspect of the present invention relates to the use of a compound of formula **I** or a pharmaceutically acceptable salt thereof for the manufacture of a medicament for the treatment of a viral infection, and preferably for the treatment of SARS-CoV-2 viral infection.

Another aspect of the present invention relates to a method of treating a disease, in a subject in need thereof, especially a human being, which comprises administering to said subject an effective amount of a compound of formula **I** or a pharmaceutically acceptable salt thereof.

Another aspect of the present invention relates to a method of treating a viral infection, preferably SARS-CoV-2 viral infection, in a subject in need thereof, especially a human being, which comprises administering to said subject an effective amount of a compound of formula **I** or a pharmaceutically acceptable salt thereof.

In the above definitions, the term C₁₋C₄ alkyl, as a group or part of a group, means a straight or branched alkyl chain which contains from 1 to 4 carbon atoms and includes the groups methyl, ethyl, propyl, isopropyl, butyl, isobutyl, *sec*-butyl and *tert*-butyl.

The compounds of the present invention may form salts. There is no limitation on the type of salt that can be used, provided that these are pharmaceutically acceptable when used for therapeutic purposes. The term pharmaceutically acceptable salt refers to those salts which are, according to medical judgment, suitable for use in contact with the tissues of humans and other mammals without undue toxicity, irritation, allergic response and the like. Pharmaceutically acceptable salts are well known in the art.

The salts of a compound of formula **I** can be obtained during the final isolation and purification of the compounds of the invention or can be prepared by treating a compound of formula **I** with a sufficient amount of the desired acid or base to give the salt in a conventional manner. The salts of the compounds of formula **I** can be converted into other salts of the compounds of formula **I** by ion exchange using ionic exchange resins.

The compounds of formula **I** and their salts may differ in some physical properties, but they are equivalent for the purposes of the present invention. All salts of the compounds of formula **I** are included within the scope of the invention.

The compounds of the present invention may form complexes with solvents in which they are reacted or from which they are precipitated or crystallized. These complexes are known as solvates. As used herein, the term solvate refers to a complex of variable stoichiometry formed by a solute (a compound of formula I or a salt thereof) and a solvent. Examples of solvents include pharmaceutically acceptable solvents such as water, ethanol and the like. A complex with water is known as a hydrate. Solvates of compounds of the invention (or salts thereof), including hydrates, are included within the scope of the invention.

The compounds of formula **I** may exist in different physical forms, i.e. amorphous and crystalline forms. Moreover, the compounds of the invention may have the ability to crystallize in more than one form, a characteristic which is known as polymorphism. Polymorphs can be distinguished by various physical properties well known in the art such as X-ray diffraction pattern, melting point or solubility. All physical forms of the compounds of formula **I,** including all polymorphic forms ("polymorphs") thereof, are included within the scope of the invention.

Some of the compounds of the present invention may exist as several diastereoisomers and/or several optical isomers. Diastereoisomers can be separated by conventional techniques such as chromatography or fractional crystallization. Optical isomers can be resolved by conventional techniques of optical resolution to give optically pure isomers. This resolution can be carried out on any chiral synthetic intermediate or on products of formula **I.** Optically pure isomers can also be individually obtained using enantiospecific synthesis. The present invention covers all individual isomers as well as mixtures thereof (for example racemic mixtures or mixtures of diastereomers), whether obtained by synthesis or by physically mixing them.

The present invention also relates to a pharmaceutical composition that comprises a compound of the present invention (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Solid compositions for oral administration include tablets, granulates and capsules. In any case the manufacturing method is based on a simple mixture, dry granulation or wet granulation of the active compound with excipients. These excipients can be, for example, diluents such as lactose, microcrystalline cellulose, mannitol or calcium hydrogenphosphate; binding agents such as for example starch, gelatin or povidone; disintegrants such as sodium carboxymethyl starch or sodium croscarmellose; and lubricating agents such as for example magnesium stearate, stearic acid or talc. Tablets can be additionally coated with suitable excipients by using known techniques with the purpose of delaying their disintegration and absorption in the gastrointestinal tract and thereby provide a sustained action over a longer period, or simply to improve their organoleptic properties or their stability. The active compound can also be incorporated by coating onto inert pellets using natural or synthetic film-coating agents. Soft gelatin capsules are also possible, in which the active compound is mixed with water or an oily medium, for example coconut oil, mineral oil or olive oil.

Powders and granulates for the preparation of oral suspensions by the addition of water can be obtained by mixing the active compound with dispersing or wetting agents; suspending agents and preservatives. Other excipients can also be added, for example sweetening, flavouring and colouring agents.

Liquid forms for oral administration include emulsions, solutions, suspensions, syrups and elixirs containing commonly-used inert diluents, such as purified water, ethanol, sorbitol, glycerol, polyethylene glycols (macrogols) and propylene glycol. Said compositions can also contain coadjuvants such as wetting, suspending, sweetening, flavouring agents, preservatives and buffers.

Injectable preparations, according to the present invention, for parenteral administration, comprise sterile solutions, suspensions or emulsions, in an aqueous or non-aqueous solvent such as propylene glycol, polyethylene glycol or vegetable oils. These compositions can also contain coadjuvants, such as wetting, emulsifying, dispersing agents and preservatives. They may be sterilized by any known method or prepared as sterile solid compositions, which will be dissolved in water or any other sterile injectable medium immediately before use. It is also possible to start from sterile materials and keep them under these conditions throughout all the manufacturing process.

For the rectal administration, the active compound can be preferably formulated as a suppository on an oily base, such as for example vegetable oils or solid semisynthetic glycerides, or on a hydrophilic base such as polyethylene glycols (macrogol).

The compounds of the invention can also be formulated for their topical application for the treatment of pathologies occurring in zones or organs accessible through this route, such as eyes, skin and the intestinal tract. Formulations include creams, lotions, gels, powders, solutions and patches wherein the compound is dispersed or dissolved in suitable excipients.

For the nasal administration or for inhalation, the compound can be formulated as an aerosol and it can be conveniently released using suitable propellants.

The dosage and frequency of doses will depend upon the nature and severity of the disease to be treated, the age, the general condition and body weight of the patient, as well as the particular compound administered and the route of administration, among other factors.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**Fig. 1****.** Shows isolated enzyme M^{pro} inhibition by compound **18.**
**Fig. 2****.** Shows isolated enzyme M^{pro} inhibition by compound **31.**

### Examples

### General procedures for the preparation of Key Intermediates (1-82)

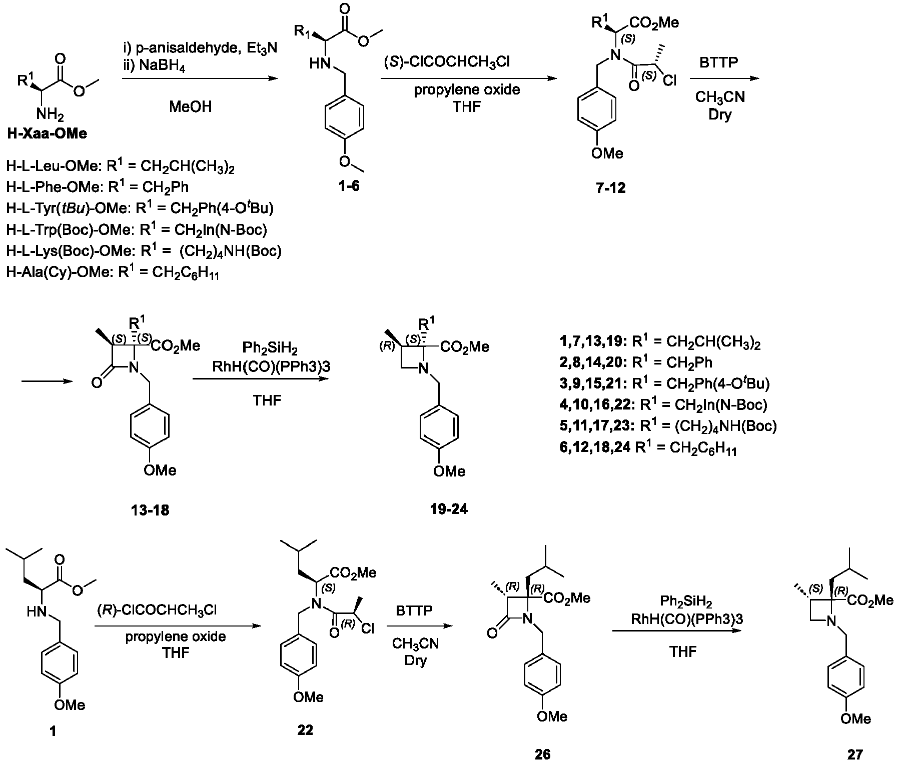

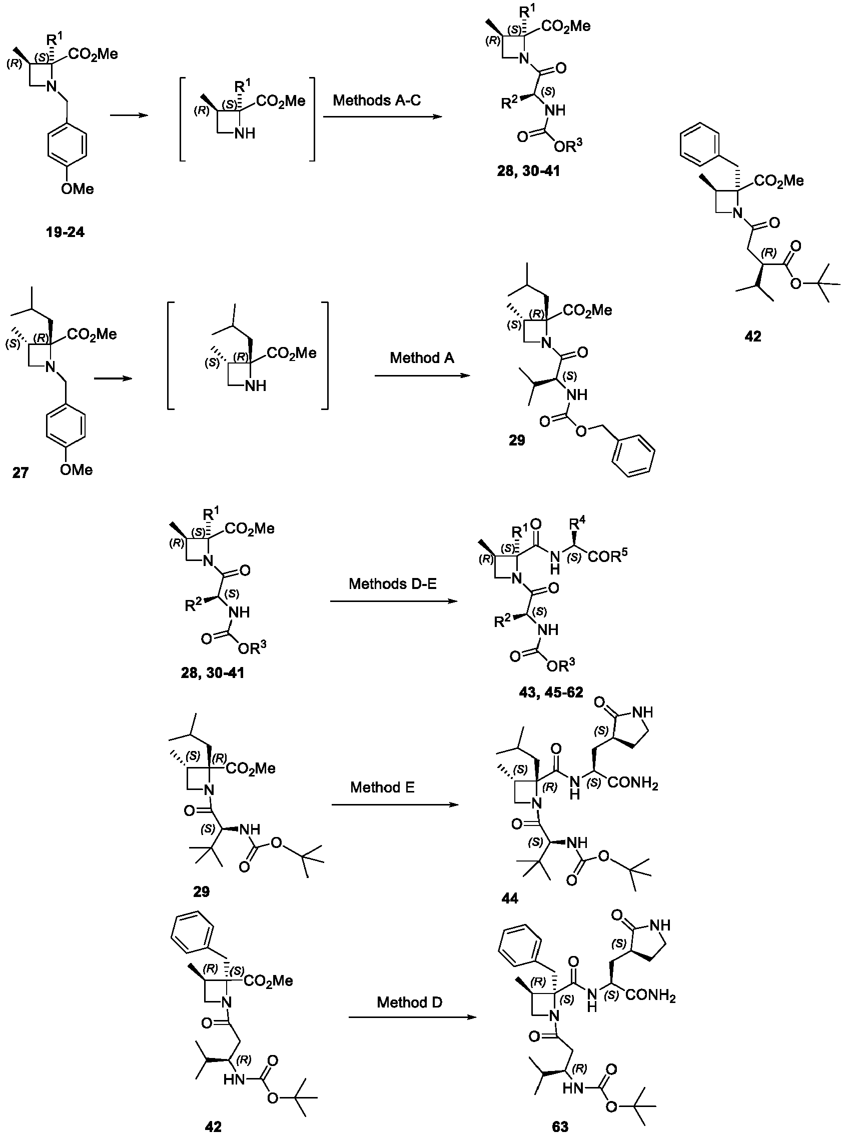

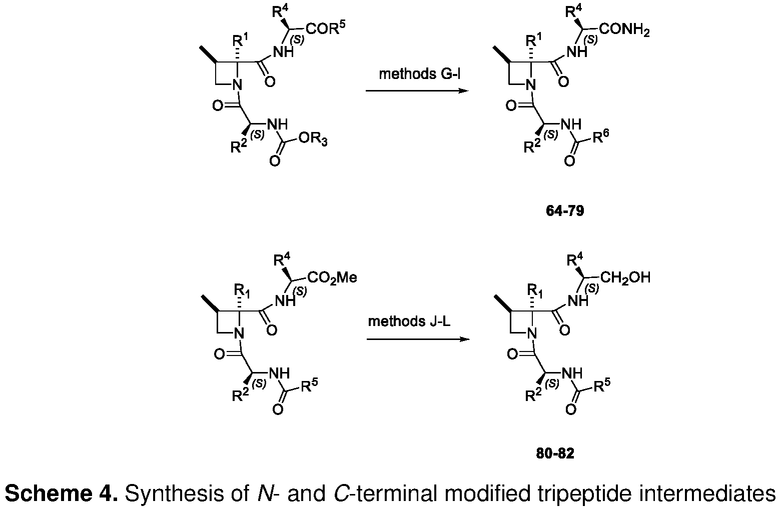

Scheme 5 illustrates the synthetic sequence followed for the preparation of compounds of general formula I. α-Cyano peptidomimetics may be obtained through the transformation of the corresponding C-terminal α-amides using POCl₃ or the Burgess reagent, while the C-terminal α-aldehydes may be achieved by partial reduction of α-methoxycarbonyl with DIBAL, or by oxidation of CN or Dess-martin oxidation of alcohols.

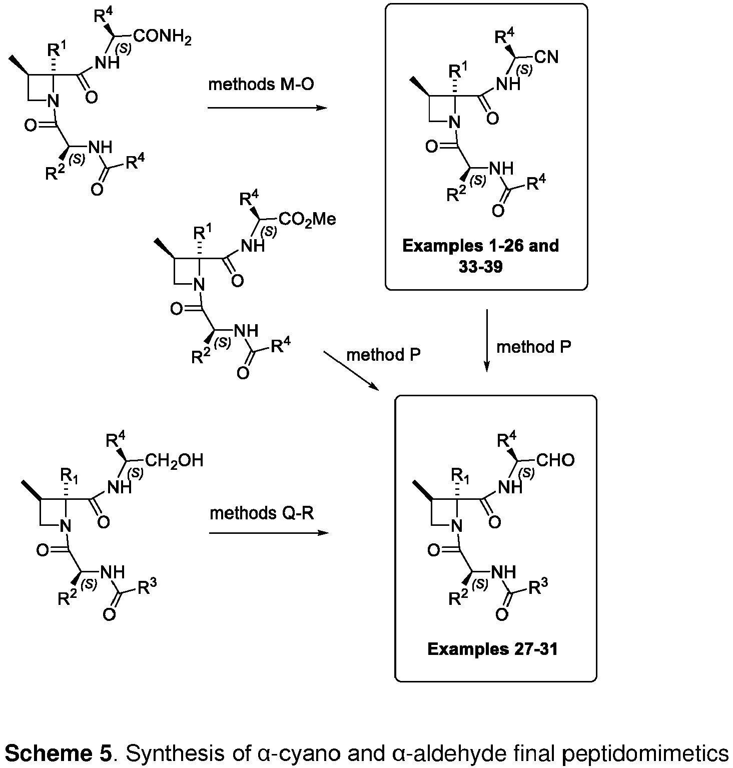

Reactions were monitored either by TLC and/or analytic HPLC (UV, detection, 220 nm). Flash columns, filled with silica gel Merck 60 (230-400), solid-phase extraction cartridges, or centrifugal circular chromatography (CCC), plates coated with silica gel Merck 60 gipshating, were used for chromatographic separations. A reversed-phase column, Sunfire C18 (4.6 × 50 mm, 3.5 µm), a flux of 1 mL/min, and mixtures of CH₃CN, phase A, and H₂O, phase B, both containing 0.01% formic acid, were used for analytical HPLCs. Mass spectra, in electrospray, positive mode, were obtained on a Waters Micro mass ZQ spectrometer. High resolution mass spectrum (ESI-HRMS) was recorded on an Agilent 6520 Q-TOF instrument. Optical rotations for final compounds were measured in a polarimeter Perkin Elmer 141 apparatus. NMR spectra were recorded in a Brucker-400 (400 MHz) spectrometer, operating at 400 and 100 MHz for ¹H and ¹³C experiments, respectively or a Varian System 500, operating at 500 and 125 MHz for ¹H and ¹³C experiments. Chemical shifts are expressed in ppm and coupling constants in Hz).

**General Procedure for the Synthesis of *N*-*p*-methoxybenzyl Amino Acid** Derivatives. A solution of H-L-Leu-OMe·HCl, H-L-Phe-OMe·HCl, H-L-Tyr(*t*Bu)-OMe·HCl, H-L-Trp(Boc)-OMe (1.0 eq) or H-Lys(Boc)-OMe in MeOH (2mL/mmol) was successively treated with TEA (1.0 eq) and p-methoxybenzaldehyde (1.2 eq). After the mixture was stirred at rt for 1.5 h, NaBH₄ (2.0 eq) was added in portions, and the stirring continued for 30 min. Then, the solvent was evaporated under vacuum, the residue was dissolved in EtOAc, and washed with H₂O and brine. The organic layer was dried over Na₂SO₄, and, after evaporation, the resulting residue was purified on a silica gel column as specified in each case.

***N*-(*p*-Pmb)-L-Leu-OMe (1)** and ***N*-(*p*-Pmb)-L-Phe-OMe (2)** were prepared as described (Guillermo Gerona Navarro, et al. Entry to new conformationally constrained amino acids. First synthesis of 3-unsubstituted 4-alkyl-4-carboxy-2-azetidinone derivatives via an intramolecular N-α-C-α -cyclization strategy. J. Org. Chem. 2001, 66, pp. 3538 - 3547).

### N-(p-Pmb)-L-Tyr(tBu)-OMe (3)

Syrup. Yield: 76% (from H-L-Tyr(*t*Bu)-OMe·HCl). Eluents: EtOAc:hexane (2:8). HPLC (t_{R}, min): 4.95 (gradient from 20 to 95% of A, in 10 min). ¹H NMR (400 MHz, CDCl₃): δ 7.14 (m, 2H, C₆H₄), 7.05 (d, *J* = 8.4 Hz, 2H, C₆H₄), 6.90 (d, *J* = 8.4 Hz, 2H, C₆H₄), 6.81 (d, *J=* 8.6 Hz, 2H, C₆H₄), 3.81-3.74 (m, 4H, NHCH₂, OCH₃), 3.65-3.57 (m, 4H, NHCH₂, COOCH₃), 3.52 (m, 1H, α-H Tyr), 2.94-2.91 (m, 2H, β-HTyr), 1.33 (s, 9H, C(CH₃)₃). ¹³C (100 MHz, CDCl₃): δ 174.8 (CO), 158.9, 154.2, 132.1, 131.0, 129.8, 129.7, 124.3, 113.9 (Ar), 78.5 (C(CH₃)₃), 62.0 (Cα Tyr), 55.4 (OCH₃), 51.8, 51.4 (NHCH₂, CO₂CH₃), 39.0 (Cβ Tyr), 29.0 (C(CH₃)₃). MS (ES)⁺: 372.3 [M+H]⁺.

### N-(p-Pmb)-L-Trp(Boc)-OMe (4)

Syrup. Yield: 68% (from HCl.H-Trp(Boc)-OMe). Eluents: EtOAc:hexane (3:7). HPLC (t_{R}, min): 6.17 (gradient from 20 to 95% of A, in 10 min). ¹H NMR (400 MHz, CDCl₃): δ 8.04 (d, *J* = 7.4 Hz, 1H, CH In), 7.42-7.38 (m, 2H, CH In), 7.25-7.21 (m, 1H, H In), 7.16-7.10 (m, 3H, C₆H₄, CH Ind), 6.72 (d, *J* = 8.6 Hz, 2H, C₆H₄), 3.73-3.70 (m, 4H, α-H Trp, OCH₃), 3.60-3.56 (m, 5H, NHCH₂, CO₂CH₃), 3.08-2.97 (m, 2H, β-H Trp), 1.59 (s, 9H, C(CH₃)₃). ¹³C (100 MHz, CDCl₃): δ 174.7 (CO), 159.0 (C Ar), 149.8 (CO Boc), 135.5, 130.9, 130.5 (C Ar), 129.8 (CH Ar), 124.6, 124.3, 122.6, 119.1 (CH In), 116.0 (C In), 115.4 (CH In), 113.9 (CH Ar), 83.7 (C(CH₃)₃), 60.4 (Cα Trp), 55.4 (OCH₃), 52.1 (CO₂CH₃), 51.5 (NHCH₂), 28.9 (Cβ Trp), 28.4 (C(CH₃)₃). MS (ES)⁺: 439.3 [M+H]⁺.

### N-(p-Pmb)-L-Lys(Boc)-OMe (5)

Syrup. Yield: 76% (from H-L-Lys(Boc)-OMe·HCl). Eluents: EtOAc:hexane (1:2). HPLC (t_{R}, min): 5.96 (gradient from 20 to 95% of A, in 10 min). ¹H NRM (400 MHz, CDCl₃): δ 7.22 (d, *J* = 8.6 Hz, 2H, C₆H₄), 6.84 (d, *J* = 8.6 Hz, 2H, C₆H₄), 4.54 (br s, 1H, NHBoc), 3.78 (s, 3H, OMe), 3.71 (d, *J=* 12.7 Hz, 1H, NHCH₂), 3.70 (s, 3H, OMe), 3.54 (d, *J* = 12.7 Hz, 1H, NHCH₂), 3.23 (t, *J* = 6.7 Hz, 1H, α-H Lys), 3.08 (m, 2H, ε-H Lys), 1.62 (m, 2H, β-H Lys), 1.43 (s, 9H, C(CH₃)₃), 1.42-1.33 (m, 4H, γ,δ-H Lys). ¹³C (100 MHz, CDCl₃): δ 176.1 (CO), 158.8 (Ar), 156.1 (CO, NHBoc), 132.0, 129.6, 113.9 (Ar), 79.2 (C(CH₃)₃), 60.5 (Cα Lys), 55.4 (OCH₃), 51.8, 51.7 (NHCH₂, CO₂CH₃), 40.4 (Cε Lys), 33.2 (Cβ Lys), 29.9 (Cδ Lys), 28.5 (C(CH₃)₃), 28.1 (Cγ Lys). MS (ES)⁺: 381,2 [M+H]⁺.

### N-(p-Pmb)-L-Ala(Cy)-OMe (6)

Syrup. Yield: 58% (from H-L-Ala(Cy)-OMe.HCl). Eluents: EtOAc:hexane (1:9). HPLC (t_{R}, min): 5.80 (gradient from 10 to 95% of A, in 10 min). ¹H NMR (400 MHz, CDCl₃): δ 7.23 (d, *J* = 8.6 Hz, 2H, C₆H₄), 6.84 (d, *J* = 8.6 Hz, 2H, C₆H₄),
3.79 (s, 3H, OCH₃), 3.74 (d, *J=* 12.8 Hz, 1H, NHCH₂), 3.71 (s, 3H, OCH₃), 3.55 (d, *J* = 12.8 Hz, 1H, NHCH₂), 3.32 (t, *J* = 7.0 Hz, 1H, α-H), 1.72-1.56 (m, 5H, 2, 3, 4, 5, 6-H Cy), 1.48-1.44 (m, 3H, β-H, 1-H Cy), 1.27-1.08 (m, 3H, 3, 4, 5-H Cy), 0.93-0.77 (m, 2H, 2, 3-H Cy). ¹³C (100 MHz, CDCl₃): δ 176.7 (CO), 158.8, 132.1, 129.6, 113.8 (Ar), 58.4 (Cα), 55.4 (OCH₃), 51.7, 51.6 (NHCH₂, CO₂CH₃), 41.4 (Cβ), 34.3 (C1 Cy), 33.6, 32.9 (C2-, C6- Cy), 26.6, 26.3, 26.2 (C3-, C4-, C5- Cy). MS (ES)⁺: 306.3 [M+H]⁺.

**General Procedure for the Synthesis of N-Chloropropionyl Amino Acid Derivatives.** PPh₃ (2.2 eq) was added to a solution of (S)-2-chloropropionic acid (2.0 eq) or (R)-2-chloropropionic acid (2.0 eq) and Cl₃CCN (3.1 mL, 30.66 mmol) in THF (6mL/mmol) at 0 ºC, and the reaction mixture was stirred for 30 min. Then, it was added dropwise to the reaction mixture of *N*-p-methoxybenzyl-L-Xaa-OMe (1.0 eq) and propylene oxide (20 eq) in THF (2 mL/mmol). After stirring for 24 h, the solvent was evaporated to dryness. The residue was purified by column chromatography on silica gel using the solvent system specified.

### N-[(S)-2-Chloropropionyl]-N-(p-Pmb)-L-Leu-OMe (7)

Syrup. Yield: 70% (from *N*-(*p*-Pmb)-L-Leu-OMe, 1). Eluents: Acetone:DCM (1:200). HPLC: t_{R}= 8.40 min (gradient from 30 to 95% of A, in 10 min). ¹H NMR (400 MHz, CDCl₃, two rotamers, Mr/mr = 1:0.14): δ major rotamer 7.14 (d, *J =* 8.7 Hz, 2H, C₆H₄), 6.87 (d, *J* = 8.7 Hz, 2H, C₆H₄), 4.99 (dd, *J* = 8.1, 6.6 Hz, 1H, α-H), 4.69 (d, *J* = 17.1 Hz, 1H, NCH₂), 4.53 (d, *J* = 17.1 Hz, 1H, NCH₂), 4.49 (q, *J* = 6.5 Hz, 1H, 2-H), 3.80 (s, 3H, OCH₃), 3.52 (s, 3H, CO₂CH₃), 1.85 (m, 1H, β-H), 1.62 (d, *J* = 6.6 Hz, 3H, 3-H), 1.59-1.52 (m, 2H, β-H, γ-H), 0.89 (d, *J =* 6.4 Hz, 3H, δ-H), 0.85 (d, *J =* 6.4 Hz, 3H, δ-H). ¹³C NMR (100 MHz, CDCl₃, major rotamer): δ 171.7, 170.5 (CO), 159.3, 128.4, 127.8, 114.3 (Ar), 55.7 (Cα), 55.4 (OCH₃), 52.1 (CO₂CH₃), 50.3 (C2), 48.6 (NCH₂), 38.5 (Cβ), 25.0 (Cy), 22.9, 22.3 (Cδ ), 21.0 (C3). MS (ES)⁺: 378.2 [M+Na]⁺.

***N*-[(*S*)-2-Chloropropionyl]-*N*-(*p*-Pmb)-L-Phe -OMe (8)** was prepared as described (Paula Pérez-Faginas, et al. Exceptional Stereoselectivity in the Synthesis of 1,3,4-Trisubstituted 4-Carboxy β-Lactam Derivatives from Amino Acids. Org. Lett. 2007, 9, 1593-1596).

### N-[(S)-2-Chloropropionyl]-N-(p-Pmb)-L-Tyr(^{t}Bu)-OMe (9)

Syrup. Yield: 81% (from *N*-(*p*-Pmb)-L-Tyr(*t*Bu)-OMe, 3). Eluents: EtOAc:hexane (15:85). HPLC (t_{R}, min): 5.52 (gradient from 60 to 95% of A, in 10 min). ¹H NMR (400 MHz, CDCl₃): δ 7.03-6.98 (m, 4H, C₆H₄), 6.91 (d, *J* = 8.5 Hz, 2H, C₆H₄), 6.81 (d, *J* = 8.7 Hz, 2H, C₆H₄), 4.51 (d, *J=* 16.1 Hz, 1H, NCH₂), 4.47 (q, *J* = 6.4 Hz, 1H, 2-H), 4.36 (dd, *J* = 9.5, 5.9 Hz, 1H, α-H), 3.89 (d, *J* = 16.1 Hz, 1H, NCH₂), 3.79 (s, 3H, OCH₃), 3.61 (s, 3H, CO₂CH₃), 3.29 (dd, *J* = 14.1, 5.9 Hz, 1H, β-H), 3.19 (dd, *J* = 14.1, 9.5 Hz, 1H, β-H), 1.66 (d, *J* = 6.5 Hz, 3H, 3-H), 1.33 (s, 9H, C(CH₃)₃). ¹³C (100 MHz, CDCl₃): δ 170.5, 169.6 (COO, CO), 159.4, 154.3, 132.6, 129.9, 129.0, 127.3, 124.5, 114.2 (Ar), 78.6 (OC(CH₃)₃), 60.2 (Cα), 55.4 (OCH₃), 52.3 (CO₂CH₃), 51.3 (NCH₂), 50.0 (C2), 34.4 (Cβ), 29.0 (C(CH₃)₃), 21.2 (C3). MS (ES)⁺: 484.2 [M+Na]⁺.

### N-[(S)-2-Chloropropionyl]-N-(p-Pmb)-L-Trp(Boc)-OMe (10)

Syrup. Yield: 81% (from *N*-(*p*-Pmb)-L-Trp(Boc)-OMe, **4**). Eluents: EtOAc:hexane (15:85). HPLC (t_{R}, min): 6.55 (gradient from 60 to 95% of A, in 10 min). ¹H NMR (400 MHz, CDCl₃): δ 8.11 (d, *J* = 7.5 Hz, 1H, In), 7.36-7.28 (m, 3H, In), 7.20-7.16 (m, 1H, In), 6.86 (d, *J=* 8.8 Hz, 2H, C₆H₄), 6.67 (d, *J=* 8.7 Hz, 2H, C₆H₄), 4.51 (q, *J* = 6.5 Hz, 1H, 2-H), 4.43 (m, 1H, α-H), 4.40 (d, *J* = 16.0 Hz, 1H, NCH₂), 4.24 (d, *J* = 16.2, 1H, NCH₂), 3.75 (s, 3H, OCH₃), 3.64 (s, 3H, CO₂CH₃), 3.40 (d, *J* = 7.4 Hz, 2H, β-Trp), 1.71 (d, *J* = 6.5 Hz, 3H, 3-H), 1.66 (s, 9H, C(CH₃)₃). ¹³C (100 MHz, CDCl₃): δ 170.5, 169.6 (COO, CO), 159.3 (C₆H₄), 149.7 (CO Boc), 135.5, 130.5 (In), 128.9, 127.0 (C₆H₄), 124.6, 124.4, 122.6, 118.9, 116.4, 115.4 (In), 113.9 (C₆H₄), 83.7 (C(CH₃)₃), 59.3 (Cα), 55.3 (OCH₃), 52.4 (CO₂CH₃), 51.8 (NCH₂), 50.1 (C2), 28.4 (C(CH₃)₃), 24.4 (Cβ), 21.2 (C3). MS (ES)⁺: 551.0 [M+H]⁺.

### N-[(S)-2-Chloropropionyl]-N-(p-Pmb)-L-Lys-OMe (11)

Syrup. Yield: 94% (from *N*-(*p-*Pmb)-L-Lys-OMe, 5). Eluents: EtOAc:hexane (1:2). HPLC: t_{R}= 9.88 min (gradient from 10 to 95% of A, in 10 min). ¹H NMR (400 MHz, CDCl₃, two rotamers, Mr/mr = 1:0.16): δ major rotamer 7.09 (d, *J =* 8.7 Hz, 2H, C₆H₄), 6.82 (d, *J* = 8.7 Hz, 2H, C₆H₄), 4.75 (dd, *J* = 8.7, 6.1 Hz, 1H, α-H), 4.64 (d, *J* = 17.0 Hz, 1H, NCH₂), 4.46 (m, 1H, 2-H), 4.44 (d, *J=* 12.7 Hz, 1H, NCH₂), 3.73 (s, 3H, OMe), 3.45 (s, 3H, CO₂Me), 2.99 (m, 2H, ε-H), 1.92 (m, 1H, β-H), 1.72 (m, 1H, β-H), 1.56 (d, *J* = 6.5 Hz, 3H, 3-H), 1.37 (m, 11H, C(CH₃)₃, δ-H), 1.22 (m, 2H, γ-H). ¹³C NMR (100 MHz, CDCl₃, major rotamer): δ 171.0, 170.3 (CO), 159.2 (Ar), 155.9 (CO, NHBoc), 128.0, 127.8, 114.1 (Ar), 78.8 (C(CH₃)₃, 57.1 (Cα), 55.2 (OCH₃), 51.8 (CO₂CH₃), 50.0 (C2), 48.8 (NCH₂), 40.1 (Cε), 29.4 (Cβ), 28.9 (Cδ), 28.4 (C(CH₃)₃), 23.1 (Cγ), 20.8 (C3). MS (ES)⁺: 493.1 [M+H]⁺.

### N-[(S)-2-Chloropropionyl]-N-(p-Pmb)-L-Ala(Cy)-OMe (12)

Syrup. Yield: 98% (from *N*-(*p*-Pmb)-L-Ala(Cy)-OMe **6**). Eluents: EtOAc:hexane (13:87). HPLC: t_{R}= 5.04 min (gradient from 60 to 95% of A, in 10 min). ¹H NMR (400 MHz, CDCl₃, two rotamers, Mr/mr = 1:0.14): δ major rotamer 7.14 (d, *J* = 8.6 Hz, 2H, C₆H₄), 6.88 (d, *J* = 8.6 Hz, 2H, C₆H₄), 5.03 (dd, *J* = 8.6, 6.2 Hz, 1H, α-H), 4.69 (d, *J* = 17.1 Hz, 1H, NCH₂), 4.51 (d, *J* = 17.1 Hz, 1H, NCH₂), 4.49 (q, *J* = 6.5 Hz, 1H, 2-H), 3.80 (s, 3H, OCH₃), 3.52 (s, 3H, CO₂CH₃), 1.86 (ddd, *J* = 14.5, 8.3, 6.2 Hz, 1H, β-H), 1.75-1.52 (m, 9H, 3-H, 2, 3, 4, 5, 6-H Cy, β-H), 1.25-1.05 (m, 4H, 1, 3, 4, 5 -H Cy), 0.92-0.77 (m, 2H, 2, 6-H Cy). ¹³C NMR (100 MHz, CDCl₃, major rotamer): δ 171.8, 170.5 (CO), 159.3, 128.5, 127.8, 114.3 (Ar), 55.5 (OCH₃), 55.1 (Cα), 52.1 (CO₂CH₃), 50.3 (C2), 48.6 (NCH₂), 37.1 (Cβ), 34.3 (C1 Cy), 33.6, 32.9 (C2-, C6- Cy), 26.5, 26.4, 26.1 (C3-, C4-, C5- Cy), 20.9 (C3). MS (ES)⁺: 418.2 [M+Na]⁺.

### N-[(R)-2-Chloropropionyl]-N-(p-Pmb)-L-Leu-OMe (25)

Syrup. Yield: 72% (from *N*-(*p*-Pmb)-L-Leu-OMe, **1**). Eluents: Acetone:DCM (1:200). HPLC: t_{R}= 8.41 min (gradient from 30 to 95% of A, in 10 min).. ¹H NMR (400 MHz, CDCl₃, two rotamers, Mr/mr = 1:0.4): δ major rotamer 7.19 (d, *J* = 8.7 Hz, 2H, C₆H₄), 6.90 (d, *J* = 8.7 Hz, 2H, C₆H₄), 4.85 (d, *J* = 17.3 Hz, 1H, NCH₂), 4.58 (t, *J* = 6.8 Hz, 1H, α-H), 4.47 (q, *J* = 6.6 Hz, 1H, 2-H), 4.42 (d, *J* = 17.3 Hz, 1H, NCH₂), 3.81 (s, 3H, OCH₃), 3.69 (s, 3H, CO₂CH₃), 1.90 (m, 1H, β-H), 1.62 (d, *J =* 6.6 Hz, 3H, 3-H), 1.59-1.50 (m, 2H, β-H, γ-H), 0.90 (d, *J* = 6.5 Hz, 3H, δ-H), 0.78 (d, *J* = 6.4 Hz, 3H, δ-H). ¹³C NMR (100 MHz, CDCl₃, major rotamer): δ 171.8, 170.3 (CO), 159.4, 128.7, 127.8, 114.5 (Ar), 57.7 (Cα), 55.5 (OCH₃), 52.3 (CO₂CH₃), 50.4 (NCH₂), 50.1 (C2), 38.2 (CP), 25.4 (Cy) 22.6, 22.5 (Cδ), 20.9 (C3). MS (ES)⁺: 378.2 [M+Na]⁺.

### General Procedure for the Synthesis of 2-Oxoazetidine Amino Acid Derivatives.

A solution of the corresponding *N*-*p*-methoxybenzyl-*N*-chloropropionyl derivative (1.0 eq) in dry CH₃CN (2mL/mmol) was treated with BTPP (1.5 eq) and stirred at rt until disappearance of the starting material. After evaporation of the solvent, the residue was extracted with EtOAc, and the extract was washed with 1M HCl, H₂O and brine. The organic layer was dried over Na₂SO₄ and then, after evaporation, the resulting residue was purified on a silica gel column as specified in each case.

### (3S,4S)-4-isobutyl-1-(p-methoxy)benzyl-4-methoxycarbonyl-3-methyl-2-oxoazetidine (13)

Syrup. Yield: 77% (from **7**). Eluents: EtOAc:hexane (1:2). HPLC: t_{R}= 8.28 min (gradient from 10 to 95% of A, in 10 min). [α]_{D} = -39.36 (c 0.76, CHCl₃). ¹H NMR (400 MHz, CDCl₃): δ 7.23 (d, *J =* 8.7 Hz, 2H, C₆H₄), 6.84 (d, *J =* 8.7 Hz, 2H, C₆H₄), 4.67 (d, *J =* 15.6 Hz, 1H, NCH₂), 4.22 (d, *J =* 15.6 Hz, 1H, NCH₂), 3.78 (s, 3H, OCH₃), 3.70 (s, 3H, CO₂CH₃), 3.15 (q, *J =* 7.5 Hz, 1H, 3-H), 1.81 (m, 1H, CH, *ⁱ*Bu), 1.61 (m, 2H, CH₂, *ⁱ*Bu), 1.19 (d, *J=* 7.5 Hz, 3H, 3-CH₃), 0.76 (d, *J =* 6.5 Hz, 6H, CH₃, *ⁱ*Bu). ¹³C NMR (100 MHz, CDCl₃): δ 172.1 (CO), 170.0 (C2), 159.0, 129.55, 129.5, 114.0 (Ar), 68.4 (C4), 55.4 (OCH₃), 54.6 (1-CH₂), 52.0 (CO₂CH₃), 44.9 (CH₂, *ⁱ*Bu), 44.5 (C3), 24.9, 24.7 (CH₃, *ⁱ*Bu), 22.4 (CH, *ⁱ*Bu) 10.7 (3-CH₃). MS (ES)⁺: 320.1 [M+H]⁺.

### (3R,4R)-4-isobutyl-1-p-methoxybenzyl-4-methoxycarbonyl-3-methyl-2-oxoazetidine (26)

Syrup. Yield: 78% (from **25**). Eluents: EtOAc:hexane (1:2). HPLC: t_{R}= 8.28 min (gradient from 10 to 95% of A, in 10 min). [α]_{D} = 39.68 (c 0.75, CHCl₃). ¹H NMR (400 MHz, CDCl₃ and ¹³C NMR (100 MHz, CDCl₃): δ identical to its enantiomer **13** MS (ES)⁺: 320.1 [M+H]⁺.

**(35.45) -4-Benzyl-4-methoxycarbonyl-1-p-methoxybenzyl-3-methyl-2-oxoazetidine (14)** was prepared from **8** as described in Paula Pérez-Faginas, et al. Exceptional Stereoselectivity in the Synthesis of 1,3,4-Trisubstituted 4-Carboxy β-Lactam Derivatives from Amino Acids. Org. Lett. 2007, 9, 1593-1596.

### (3S,4S)-4-(tert-Butoxy)benzyl-1-p-methoxybenzyl-4-methoxycarbonyl-3-methyl-2-oxoazetidine (15)

Syrup. Yield: 77% (from **9**). Eluents: EtOAc:hexane (3:7). HPLC (t_{R}, min): 8.74 (gradient from 40 to 95% of A, in 10 min). ¹H NMR (400 MHz, CDCl₃): δ 7.12 (d, *J* = 8.6 Hz, 2H, C₆H₄), 6.86-6.80 (m, 6H, C₆H₄), 4.38 (d, *J=* 15.4 Hz, 1H, 1-CH₂), 4.10 (d, *J* = 15.4 Hz, 1H, 1-CH₂), 3.79 (s, 3H, OCH₃), 3.69 (s, 3H, CO₂CH₃), 3.31 (d, *J* = 14.5 Hz, 1H, 4-CH₂), 3.15 (q, *J* = 7.5 Hz, 1H, H3), 3.04 (d, *J* = 14.5 Hz, 1H, 4-CH₂), 1.32 (s, 9H, OC(CH₃)₃), 1.14 (d, *J* = 7.5 Hz, 3H, 3-CH₃), ¹³C (100 MHz, CDCl₃): δ 171.9 (CO), 169.8 (C2), 160.0, 154.7, 130.6, 130.1, 129.9, 129.1, 124.2, 113.9 (Ar), 78.6 (OC(CH₃)₃), 69.3 (C4), 55.4 (OCH₃), 53.9 (1-CH₂), 52.2 (CO₂CH₃), 45.6 (C3), 40.2 (4-CH₂), 29.0 (C(CH₃)₃), 10.2 (3-CH₃). MS (ES)⁺: 426.3 [M+Na]⁺.

### (35,4 S)-1- p-Meth oxybenzyl-4-methoxycarbonyl-3-methyl-4-[(1- tert-butoxycarbonyl)indol-3-yl]methyl-2-oxoazetidine (16)

Syrup. Yield: 73% (from **10**). Eluents: EtOAc:hexane (3:7). HPLC (t_{R}, min): 9.07 (gradient from 40 to 95% of A, in 10 min). ¹H NMR (400 MHz, CDCl₃): δ 8.07 (d, *J* = 8.4 Hz, 1H, 7-In), 7.35 (dt, *J* = 7.7, 1.0 Hz, 1H, 4-In), 7.29 (ddd, *J* = 8.5, 7.3, 1.3 Hz, 1H, 6-In), 7.19 (td, *J* = 7.5, 1.1 Hz 1H, 5-In), 7.13 (d, *J* = 8.6 Hz, 2H, C₆H₄), 7.09 (s, 1H, 2-In), 6.76 (d, *J* = 8.7 Hz, 2H, C₆H₄), 4.51 (d, *J* = 15.2 Hz, 1H, 1-CH₂), 4.22 (d, *J* = 15.2 Hz, 1H, 1-CH₂), 3.76 (s, 3H, OCH₃), 3.74 (s, 3H, CO₂CH₃), 3.29 (d, *J* = 15.5 Hz, 1H, 4-CH₂), 3.19-3.14 (m, 2H, 4-CH₂, H3), 1.67 (s, 9H, C(CH₃)₃). ¹³C (100 MHz, CDCl₃): δ 172.1 (CO), 169.8 (C2), 159.1 (Ar), 149.6 (CO₂*^{t}*Bu), 135.1, 131.1 (In), 130.0, 128.5 (C₆H₄), 124.7, 124.6, 122.8, 118.5, 115.4 (In), 114.0 (C₆H₄), 113.9 (In), 83.9 (OC(CH₃)₃), 68.5 (C4), 55.3 (OCH₃), 53.7 (1-CH₂), 52.4 (CO₂CH₃), 45.2 (C3), 29.0 (4-CH₂), 28.3 (C(CH₃)₃), 10.2 (3-CH₃). MS (ES)⁺: 515.1 [M+Na]⁺.

### (3S,4S)-1-(p-Methoxy)benzyl-4-[(tert-butoxycarbonyl)amino]butyl-4-methoxy-carbonyl-3-methyl-2-oxoazetidine (17)

Syrup. Yield: 72% (from **11**). Eluents: EtOAc:hexane (1:2). HPLC: t_{R}= 5.25 min (gradient from 40 to 95% of A, in 10 min). ¹H NMR (400 MHz, CDCl₃): δ 7.20 (d, *J* = 8.6 Hz, 2H, C₆H₄), 6.83 (d, *J* = 8.6 Hz, 2H. C₆H₄), 4.74 (d, *J* = 15.3 Hz, 1H, NCH₂), 4.35 (br s, 1H, NHBoc), 4.08 (d, *J* = 15.4 Hz, 1H, NCH₂), 3.77 (s, 3H, OMe), 3.72 (s, 3H, CO₂Me), 3.11 (q, *J* = 7.5 Hz, 1H, 3-H), 2.85 (m, 2H, 4'-H), 1.69 (m, 2H, 1'-H), 1.41 (s, 9H, C(CH₃)₃), 1.13 (d, *J* = 7.6 Hz, 3H, 3-CH₃), 1.10-1.05 (m, 3H, 3'-H, 2'-H), 0.96 (m, 1H, 2'-H). ¹³C NMR (100 MHz, CDCl₃): δ 171.9 (CO), 169.5 (C2), 159.2, 129.8, 128.1, 114.0 (Ar), 79.2 (C(CH₃)₃), 68.8 (OCH₂), 55.4 (OCH₃), 53.0 (3-H), 52.2 (CO₂CH₃), 44.7 (NCH₂), 40.1 (C4'), 34.1 (C1'), 29.9 (C3'), 44.5 (C3), 28.5 (C(CH₃)₃), 21.4 (C2'), 10.3 (3-CH₃). MS (ES)⁺: 435.1 [M+H]⁺.

### (3S,4S)-1-(p-Methoxy)benzyl-4-cyclohexylmethyl-4-methoxycarbonyl-3-methyl-2-oxoazetidine (18)

Syrup. Yield: 78% (from **12**). Eluents: EtOAc:hexane (21:79). HPLC: t_{R}= 7.89 min (gradient from 40 to 95% of A, in 10 min). ¹H NMR (400 MHz, CDCl₃): δ 7.22 (d, *J* = 8.7 Hz, 2H, C₆H₄), 6.84 (d, *J* = 8.7 Hz, 2H, C₆H₄), 4.71 (d, *J* = 15.6 Hz, 1H, 1-CH₂), 4.18 (d, *J* = 15.6 Hz, 1H, 1-CH₂), 3.78 (s, 3H, OCH₃), 3.70 (s, 3H, CO₂CH₃), 3.13 (q, *J* = 7.5 Hz, 1H, 3-H), 1.70 (d, *J* = 6.3 Hz, 2H, 4-CH₂), 1.59-1.47 (m, 3H, 3, 4, 5-H Cy), 1.38-1.27 (m, 2H, 2, 6-H Cy), 1.18 (m, 1H, 1-H Cy), 1.17 (d, *J* = 7.5 Hz, 3H, 3-CH₃), 1.06-0.97 (m, 3H, 3, 4, 5-H Cy), 0.84-0.70 (m, 2H, 2, 6-H Cy). ¹³C NMR (100 MHz, CDCl₃): δ 172.2 (CO), 170.1 (C2), 159.0, 129.7, 129.5, 114.0 (Ar), 68.3 (C4), 55.4 (OCH₃), 54.6 (C3), 52.0 (CO₂CH₃), 44.8 (1-CH₂), 43.1 (4-CH₂), 35.2 (C2-, C6-Cy), 34.1 (C1 Cy), 33.2 (C2-, C6- Cy), 26.35, 26.3, 26.1 (C3-, C4-, C5- Cy), 10.6 (3-CH₃). MS (ES)⁺: 360.1 [M+H]⁺.

**General method for the β-lactam reduction.** A solution of the corresponding azetidinone (1.0 eq) in dry THF (2mL/mmol) was added, under Ar atmosphere, tris(triphenyl-phosphine)rhodium(I) carbonyl hydride (0.01 eq) and diphenylsilane (2.5 eq). After 16 h of reaction at rt, the solvent was evaporated to give a residue that was dissolved in Et₂O and washed twice with 1 M HCl. The aqueous layer was treated with 2 N NaOH to pH 10 and extracted with EtOAc. The organic layer was washed with brine and dried over Na₂SO₄. The solvent was evaporated under reduced pressure and the resulting residue was purified on a silica gel column as specified in each case.

### p-Methoxybenzyl-(αS, β'R)-Azl-OMe (MA-1474) (19)

Syrup. Yield: 86% (from **13**). Eluents: EtOAc:hexane (1:10). HPLC: t_{R}= 3.32 min (gradient from 10 to 95% of A, in 10 min). [α]_{D} = -6.23 (c 1.55, CHCl₃).¹H NMR (400 MHz, CDCl₃): δ 7.24 (d, *J* = 8.6 Hz, 2H, C₆H₄), 6.84 (d, *J* = 8.7 Hz, 2H, C₆H₄), 3.99 (d, *J* = 13.3 Hz, 1H, NCH₂), 3.79 (s, 3H, OCH₃), 3.75 (s, 3H, CO₂CH₃), 3.66 (d, *J* = 13.3 Hz, 1H, NCH₂), 3.25 (dd, *J* = 7.5, 5.8 Hz, γ'-H), 2.85 (dd, *J* = 7.5, 5.8 Hz, γ'-H), 2.51 (m, 1H, β'-H), 1.91-1.81 (m, 3H, β-H, γ-H), 1.05 (d, *J* = 7.1 Hz, 3H, β'-CH₃), 0.95 (d, *J =* 6.3 Hz, 3H, δ-H), 0.90 (d, *J =* 6.3 Hz, 3H, δ-H). ¹³C NMR (100 MHz, CDCl₃): δ 174.4 (CO), 158.6, 131.8, 129.6, 113.7 (Ar), 75.7 (Cα), 56.7 (Cγ'), 55.5 (OCH₃), 55.4 (CO₂CH₃), 50.9 (NCH₂), 46.3 (Cβ'), 37.1 (Cβ), 22.9 (Cγ), 24.6, 23.3 (Cδ), 15.3 (β'-CH₃). MS (ES)⁺: 306.2 [M+H]⁺.

### p-Methoxybenzyl- (αR, β'S)-Azl-OMe (27)

Syrup. Yield: 82% (from **26**). Eluents: EtOAc:hexane (1:2). HPLC: t_{R}= 3.32 min (gradient from 10 to 95% of A, in 10 min). [α]_{D} = 5.73 (c 0.71, CHCl₃). ¹H NMR (400 MHz, CDCl₃) and and ¹³C NMR (100 MHz, CDCl₃): δ identical to those of its enantiomer 16. MS (ES)⁺: 306.2 [M+H]⁺.

### p-Methoxybenzyl-(αR, β'S)-Azf-OMe (20)

Syrup. Yield: 86% (from **14**). Eluents: EtOAc:hexane (8:1). HPLC: t_{R}= 6.19 min (gradient from 10 to 95% of A, in 10 min). ¹H NMR (400 MHz, CDCl₃): δ 7.30-7.18 (m, 7H, C₆H₄, Ph), 6.84 (d, *J* = 8.9 Hz, 2H, C₆H₄), 3.84 (d, *J* = 13.3 Hz, 1H, NCH₂), 3.82 (s, 3H, OCH₃), 3.79 (s, 3H, CO₂CH₃), 3.78 (d, *J* = 13.3 Hz, 1H, NCH₂), 3.30-3.27 (m, 2H, γ'-H, β-H), 3.07 (d, *J* = 13.3 Hz, 1H, β-H), 2.90 2.90 (dd, *J* = 8.2, 5.6 Hz, 1H, γ'-H), 2.64 (m, 1H, β'-H), 1.00 (d, *J=* 6.9 Hz, 3H, β'-CH₃), ¹³C NMR (100 MHz, CDCl₃): δ 173.6 (CO), 158.5, 137.4, 131.7, 130.6, 129.5, 128.1, 126.5 113.7 (Ar), 77.0 (Cα), 57.0 (Cγ'), 55.4 (OCH₃), 55.1 (NCH₂), 51.0 (OCH₃), 43.9 (Cβ'), 36.6 (Cβ), 22.9 (Cγ), 14.7 (β'-CH₃). MS (ES)⁺: 340.3 [M+H]⁺.

### p-Methoxybenzyl-(αS,β'R)-Azy(tBu)-OMe (21).

Syrup. Yield: 60% (from **15**) Eluents: EtOAc:hexane (1:9). HPLC (t_{R}, min): 3.89 (gradient from 30 to 95% of A, in 10 min). ¹H NMR (400 MHz, CDCl₃): δ 7.17-7.14 (m, 4H, C₆H₄), 6.87 (d, *J=* 8.5 Hz, 2H, C₆H₄), 6.81 (d, *J=* 8.7 Hz, 2H, C₆H₄), 3.81 (s, 3H, OCH₃), 3.80 (d, *J* = 13.2 Hz, 1H, NCH₂), 3.79 (s, 3H, CO₂CH₃), 3.31-3.26 (m, NCH₂, y'-H), 3.21 (d, *J* = 13.4 Hz, 1H, β-H), 2.99 (d, *J* = 13.4 Hz, 1H, β-H), 2.88 (dd, *J* = 8.2, 5.6 Hz, 1H, γ'-H), 2.60 (h, J= 7.2 Hz, 1H, β'-H), 1.30 (s, 9H, CH₃ *^{t}*Bu), 0.97 (d, J= 7.1 Hz, 3H, β'-CH₃), ¹³C (100 MHz, CDCl₃): δ 173.7 (CO), 158.5, 154.0, 132.3 131.8, 130.9, 129.5, 123.9, 113.7 (Ar), 78.3 (OC(CH₃)₃), 77.4 (Cα), 57.1 (Cγ'), 55.4 (OCH₃), 55.2 (NCH₂), 51.0 (CO₂CH₃), 43.5 (Cβ'), 36.8 (Cβ), 29.0 (OC(CH₃)₃), 14.7 (β'-CH₃). MS (ES)⁺: 412.4 [M+H]⁺.

### p-Methoxybenzyl-(αS, β'R)-Azw(Boc)-OMe (22)

Syrup. Yield: 80% (from **16**). Eluents: EtOAc:hexane (8:92). HPLC (t_{R}, min): 6.97 (gradient from 30 to 95% of A, in 10 min). ¹H NMR (400 MHz, CDCl₃): δ 8.11 (d, *J* = 8.2 Hz, 1H, 7-In), 7.62 (d, *J=* 7.7 Hz, 1H, 4-In), 7.47 (s, 1H, 2-In), 7.28 (m, 1H, 6In), 7.20 (m, 1H, 5-In), 7.13 (d, *J=* 8.5 Hz, 2H, C₆H₄), 6.79 (d, *J=* 8.6 Hz, 2H, C₆H₄), 3.86 (d, *J* = 13.1 Hz, 1H, NCH₂), 3.80 (s, 3H, OCH₃), 3.78 (s, 3H, CO₂CH₃), 3.45 (d, *J* = 13.1 Hz, 1H, NCH₂), 3.29 (dd, *J* = 7.4, 5.8 Hz, 1H, γ'-H), 3.22 (m, 2H, β-H), 2.92 (dd, *J* = 7.9, 5.7 Hz, 1H, γy'-H), 2.64 (q, *J* = 7.2 Hz, 1H, β'-H), 1.64 (s, 9H, C(CH₃)₃), 1.01 (d, *J=* 7.0 Hz, 3H, β'-CH₃), ¹³C (100 MHz, CDCl₃): δ 173.5 (CO), 158.5 (C₆H₄), 149.9 (NCOO), 135.2, 131.7, 131.4 (C₆H₄, In), 129.7 (C₆H₄), 124.8, 124.3, 122.5, 119.6, 116.2, 115.2 (In), 113.6 (C₆H₄), 83.4 (C(CH₃)₃), 77.4 (Cα), 56.9 (Cγ), 55.4 (OCH₃), 55.0 (NCH₂), 51.1 (CO₂CH₃), 36.1 (Cβ'), 32.2 (Cβ), 28.4 (C(CH₃)₃), 14.8 (β'-CH₃). MS (ES)⁺: 497.3 [M+H]⁺.

### p-Methoxybenzyl-(αS, β'R)-Azk(Boc)-OMe (23)

Syrup. Yield: 53% (from **17**). Eluents: EtOAc:hexane (1:2). HPLC: t_{R}= 6.27 min (gradient from 5 to 95% of A, in 10 min).

¹H NMR (400 MHz, CDCl3): δ 7.23 (m, 2H, C₆H₄), 6.83 (d, J = 8.6 Hz, 2H, C₆H₄), 4.55 (m, 1H, ε-NH), 3,87 (m, 1H, *N*-CH₂), 3.79-3,77 (m, 6H, OMe), 3.61 (m, 1H, *N*-CH₂), 3.27 (m, 1H, y'-H), 3.10 (m, 2H, ε-H), 2.92 (m, 1H, y'-H), 2.49 (m, 1H, β'-H), 1.81 (m, 2H, β-H), 1.44 (m, 12H, C(CH₃)₃, γ-H, δ-H), 1.28 (m, 1H, γ-H), 1.03 (d, J = 7.0 Hz, 3H, β'-CH₃), ¹³C NMR (100 MHz, CDCl₃): 173.8 (CO), 158.1 (Ar), 156.1 (NHCOO), 133.4, 129.7, 127.7, 113.7 (Ar), 79.2 (C(CH₃)₃), 77.2 (Cα), 56.6 (Cy'), 55.4 (OMe, N-CH₂), 51.1 (CO₂Me), 40.4 (Cε), 36.6 (Cβ), 35,7 (Cβ'), 30.6 (Cδ), 28.6 (C(CH₃)₃), 21,7 (Cγ), 15.1 (β'-CH₃). MS (ES)⁺: 421.2 [M+H]⁺.

### p-Methoxybenzyl-(αS, β'R)-Aza(Cy)-OMe (24)

Syrup. Yield: 70% (from **18**). Eluents: EtOAc:hexane (22:78). HPLC: t_{R}= 6.93 min (gradient from 5 to 95% of A, in 10 min). ¹H NMR (400 MHz, CDCl₃): δ 7.24 (d, *J* = 8.6 Hz, 2H, C₆H₄), 6.84 (d, *J* = 8.6 Hz, 2H, C₆H₄), 3.97 (d, *J* = 13.2 Hz, 1H, NCH₂), 3.79 (s, 3H, OCH₃), 3.75 (s, 3H, CO₂CH₃), 3.63 (d, *J* = 13.2 Hz, 1H, NCH₂), 3.25 (dd, *J* = 7.5, 5.8 Hz, 1H, y'-H), 2.85 (dd, *J* = 7.5, 5.8 Hz, 1H, γ'-H), 2.50 (m, 1H, β'-H), 1.84 (dd, *J* = 13.9, 7.6 Hz, 1H, β-H), 1.76 (dd, *J* = 13.9, 4.6 Hz, 1H, β-H), 1.71-1.57 (m, 5H, 2, 3, 4, 5, 6-H Cy), 1.54 (m, 1H, 1-H Cy), 1.28-1.09 (m, 3H, 3, 4, 5-H Cy), 1.04 (d, *J* = 7.1 Hz, 3H, β'-CH₃), 1.00-0.83 (m, 2H, 2, 6-H Cy). ¹³C NMR (100 MHz, CDCl₃): δ 174.5 (CO), 158.6, 131.8, 129.7, 113.8 (Ar), 75.6 (Cα), 56.7 (Cy'), 55.5 (NCH₂), 55.4 (OCH₃), 50.9 (CO₂CH₃), 44.9 (Cβ), 37.1 (Cβ' Aza), 35.2, 34.1 (C2-, C6- Cy), 34.1 (C1 Cy), 26.6, 26.55, 26.5 (C3-, C4-, C5- Cy), 15.2 (β'-CH₃).MS (ES)⁺: 346.2 [M+H]⁺.

### General Procedures for the Synthesis of Dipeptide Derivatives

***Method A.*** A solution of the corresponding 1-(*p*-methoxy)benzylazetidine (1.0 eq) in MeOH (10 mL/mmol) was successively added HCl 12 M (1.0 eq) and Pd(OH)₂ (20%w/w). The obtained suspension was hydrogenated at 50 °C and 40 psi of pressure for 16 h. After filtration of the catalyst, the solvent was evaporated to dryness and the final azetidine hydrochloride was lyophilized. To a solution of the resulting azetidine hydrochloride (1.0 eq) in THF (5mL/mmol), TEA (2.2 eq), PyBrop (1.2 eq) and Z-Val-OH were added and the reaction mixture was stirred at rt for 24-48 h. After that time, the solvent was evaporated under reduced pressure and the residue was redissolved in EtOAc, washed with a 10% citric acid solution, a 10% NaHCO₃ solution and brine. The organic layer was dried over Na₂SO₄, filtered and the solvent was evaporated under reduced pressure. The resulting residue was purified on a silica gel column as specified in each case.

***Method B.*** The suspension containing 1-(*p*-methoxy)benzylazetidine (1.0 eq), HCl 12 M (1.0 eq) and Pd(OH)₂ (20%w/w) in MeOH (10 mL/mmol) was hydrogenated as described in method A above. To a solution of Z-Val-OH, Z-Tbg-OH, Boc-Tbg-OH, Boc-β-Val-OH , Z-Lys(Boc)-OH or Boc-Lys(Z)-OH (1.2 eq), HATU (1.2 eq) was added, at 0 ºC, and the mixture was stirred for 10 min at this temperature. Then, the corresponding azetidine hydrochloride (1.0 eq) was added and finally, DIPEA (2.2 eq) was added dropwise and the reaction was stirred overnight at rt. Then, the workup was carried out as in method A above.

***Method C.*** Pd(OH)₂ (20%w/w) was added a solution of the corresponding 1-(*p-*methoxy)benzylazetidine (1.0 eq) in MeOH (10 mL/mmol). The obtained suspension was hydrogenated as specified in method A above, but without HCl. To a solution of the corresponding Z-Val-OH or Z-Tbg-OH (1.2 eq) in THF (5mL/mmol), HATU (1.2 eq) was added, at 0 ºC, and the mixture was stirred for 10 min at this temperature. Then, the corresponding azetidine (1.0 eq) was added and finally, DIPEA (1.2 eq) was added dropwise and the reaction was stirred overnight at rt. Then, the workup was carried out as in method A above.

### Z-L-Val-(αS,β'R)-Azl-OMe (28)

Syrup. Yield: 70% (method A, from **19** and Z-L-Val-OH). Eluents: EtOAc:hexane (3:1). HPLC: t_{R}= 9.29 min (gradient from 10 to 95% of A, in 10 min). ¹H NMR (400 MHz, CDCl₃): δ 7.33 (s, 5H, Ph), 5.34 (d, *J =* 9.5 Hz, 1H, α-NH Val), 5.08 (s, 2H, OCH₂), 4.34 (t, *J =* 8.3 Hz, 1H, γ'-H Azl), 3.96 (dd, *J =* 9.5, 7.1 Hz, 1H, α-H Val), 3.74 (s, 3H, CO₂CH₃), 3.70 (m, 1H, γ'-H Azl), 2.79 (m, 1H, β'-H Azl), 2.12-1.85 (m, 4H, β-H Azl, γ-H Azl, β-H Val), 1.05 (d, *J =* 7.1 Hz, 3H, β'-CH₃), 1.04 (d, *J =* 6.8 Hz, 3H, γ-H Val), 0.97 (d, *J =* 6.8 Hz, 3H, γ-H Val), 0.96 (d, *J* = 6.7 Hz, 3H, δ-H Azl), 0.88 (d, *J =* 6.7 Hz, 3H, δ-H Azl). ¹³C NMR (100 MHz, CDCl₃): δ 170.91, 170,9 (CO), 156.5 (NHCOO), 136.6, 128.6, 128.2, 128.0 (Ar), 75.6 (Cα Azl), 66.9 (OCH₂), 55.9 (Cα Val), 54.0 (Cγ' Azl), 52.0 (OCH₃), 42.3 (Cβ Azl), 32.2 (Cβ' Azl), 30.8 (Cβ Val), 25.2 (Cγ' Azl), 24.6, 23.3 (Cδ Azl), 19.3, 17.8 (Cγ Val), 14.9 (β'-CH₃). MS (ES)⁺: 419.3 [M+H]⁺.

### Z-L-Val-(αR,β'S)-Azl-OMe (29)

Syrup. Yield: 81% (method A, from **27** and Z-L-Val-OH). Eluents: EtOAc:hexane (3:1). HPLC: t_{R}= 9.57 min (gradient from 10 to 95% of A, in 10 min). ¹H NMR (400 MHz, CDCl₃): δ 7.37-7.28 (m, 5H, Ph), 5.40 (d, *J =* 9.3 Hz, 1H, α-NH Val), 5.11 (s, 2H, OCH₂), 4.12-4.05 (m, 2H, α-Val, γ'-H Azl), 3.90 (t, *J =* 7.6 Hz, 1H, γ'-H Azl), 3.66 (s, 3H, CO₂CH₃), 2.79 (m, 1H, β'-H Azl), 2.08-1.86 (m, 4H, β-H Azl, γ'-H Azl, β-H Val), 1.01 (d, *J =* 7.1 Hz, 3H, β'-CH₃), 0.98 (d, *J =* 6.4 Hz, 3H, γ-H Val), 0.97 (d, *J =* 6.4 Hz, 3H, γ-H Val), 0.95 (d, *J =* 6.8 Hz, 3H, δ-H Azl), 0.91 (d, *J =* 6.8 Hz, 3H, δ-H Azl). ¹³C NMR (100 MHz, CDCl₃): δ 172.2 (COO), 170.8 (CON), 156.5 (NHCOO), 136.7, 128.6, 128.2, 128.0 (Ar), 76.1 (Cα Azl), 66.9 (OCH₂), 55.8 (Cα Val), 54.3 (Cγ' Azl), 51.9 (OCH₃), 43.1 (Cβ Azl), 32.7 (Cβ' Azl), 31.3 (Cβ Val), 25.0 (Cγ Azl), 24.9, 23.8 (Cδ Azl), 19.5, 17.6 (Cγ Val), 14.3 (β'-CH₃).MS (ES)⁺: 419.3 [M+H]⁺.

### Z-L-Val-(αS,β'R)-Azf-OMe (30)

Syrup. Yield: 60% (method B, from **20** and Z-L-Val-OH). Eluents: EtOAc:hexane (4:6). HPLC (t_{R}, min): 9.37 (gradient from 10 to 95% of A, in 10 min). ¹H NMR (400 MHz, CDCl₃): δ 7.41-7.32 (m, 5H, Ph Z), 7.21-7.13 (m, 5H, Ph), 5.46 (d, *J* = 9.6 Hz, 1H, α-NH Val), 5.12 (m, 2H, OCH₂), 3.86 (dd, *J* = 9.6, 7.4 Hz, 1H, α-H Val), 3.82 (s, 3H, OCH₃), 3.70 (d, *J* = 14.2 Hz, 1H, β-H Azf), 3.53 (t, *J* = 8.2 Hz, 1H, γ'-H Azf) 3.47 (m, 1H, γ'-H Azf), 3.10 (d, *J* = 14.3 Hz, 1H, β-H Azf), 2.63 (m, 1H, β'-H Azf), 2.03 (dq, *J* = 13.7, 6.8 Hz, 1H, β-H Val), 1.05 (d *J* = 6.8 Hz, 3H, β'-CH₃), 1.03 (d, *J* = 6.8 Hz, 3H, γ-H Val), 0.97 (d, *J* = 6.8 Hz, 3H, γ-H Val). ¹³C (100 MHz, CDCl₃): δ 171.3, 170.6 (CO), 156.2 (NHCOO), 136.6, 135.6, 131.0, 128.7, 128.34, 128.29, 128.1, 127.1 (Ar), 74.9 (Cα Azf), 67.0 (OCH₂), 55.8 (Cα Val), 53.8 (Cγ' Azf), 52.4 (OCH₃), 37.7 (Cβ Azf), 31.1 (Cβ Val), 29.9 (Cβ' Azf), 19.1, 17.9 (Cγ Val), 14.6 (β'-CH₃). MS (ES)⁺: 453.2 [M+H]⁺; 475.1 [M+Na]⁺.

### Z-L-Val-(αS, β'R)-Azy-OMe (31)

Syrup. Yield: 73% (method B, from **21** and Z-L-Val-OH). Eluents: EtOAc:hexane (35:65). HPLC (t_{R}, min): 8.65 (gradient from 15 to 95% of A, in 10 min). ¹H NMR (400 MHz, CDCl₃): δ 7.46-7.34 (m, 5H, Ph), 6.98 (d, *J=* 8.5Hz, 2H, C₆H₄), 6.50 (d, *J* = 8.5 Hz, 2H, C₆H₄), 5.47 (d, *J* = 9.6 Hz, 1H, α-NH Val), 5.20 (d, *J* = 12.4 Hz, 1H, OCH₂), 5.07 (d, *J* = 12.3 Hz, 1H, OCH₂), 3.82 (m, 1H, α-H Val), 3.80 (s, 3H, OCH₃), 3.61 (d, *J* = 14.4 Hz, 1H, β-H Azy), 3.53 (t, *J* = 8.2 Hz, 1H, γ'-H Azy), 3.45 (dd, *J* = 7.8, 6.0 Hz, 1H, γ'-H Azy), 3.00 (d, *J =* 14.4 Hz, 1H, β-H Azy), 2.60 (dq, *J* = 13.9, 7.0 Hz, 1H, β'-H Azy), 2.00 (m, 1H, β-H Val), 1.04 (d, *J* = 7.0 Hz, β'-CH₃), 1.03 (d, *J* = 6.7 Hz, 3H, γ-H Val), 0.97 (d, *J* = 6.7 Hz, 3H, γ-H Val). ¹³C (100 MHz, CDCl₃): δ 171.3, 170.7 (CO), 156.2 (NHCOO), 154.7, 137.0, 132.1, 128.8, 128.4, 128.3, 127.5, 115.2 (Ar), 75.1 (Cα Azy), 66.9 (OCH₂), 55.9 (Cα Val), 53.8 (Cγ' Azy), 52.3 (OCH₃), 36.8 (Cβ Azy), 31.2 (Cβ Val), 29.8 (Cβ' Azy), 19.1, 18.0 (Cγ Val), 14.7 (β'-CH₃). MS (ES)⁺: 469.24 [M+H]⁺; 491.1 [M+Na]⁺.

### Z-L-Val-(αS, β'R)-Azy(tBu)-OMe (32)

Syrup. Yield: 83% (method C, from **21** and Z-L-Val-OH). Eluents: EtOAc:hexane (35:65). HPLC (t_{R}, min): 9.10 (gradient from 40% to 95% of A, in 10 min. ¹H NMR (400 MHz, CDCl₃): δ 7.39-7.31 (m, 5H, Ph), 7.05 (d, *J* = 8.4 Hz, 2H, C₆H₄), 6.86 (d, *J* = 8.5 Hz, 2H, C₆H₄), 5.43 (d, *J* = 9.6 Hz, 1H, α-NH Val), 5.09 (d, *J* = 12.4 Hz, 2H, OCH₂), 5.03 (d, *J* = 12.4 Hz, 2H, OCH₂), 3.82 (dd, *J* = 9.6, 7.1 Hz, 1H, α-H Val), 3.81 (s, 3H, OCH₃), 3.66 (d, *J =* 14.3 Hz, 1H, β-H Azy), 3.53 (t, *J =* 8.2 Hz, 1H, γ'-H Azy), 3.45 (dd, *J* = 7.8, 6.1 Hz, 1H, γ'-H Azy), 3.07 (d, *J* = 14.4 Hz, 1H, β-H Azy), 2.62 (m, *J* = 1H, β'-H Azy), 2.02 (m, 1H, β-H Val), 1.31 (OC(CH₃)₃), 1.04 (m, 6H, γ-H Val, β'-CH₃), 0.96 (d, *J* = 6.8 Hz, 3H, γ-H Val). ¹³C (100 MHz, CDCl₃): δ 171.3, 170.7 (CO), 156.1 (NHCOO), 154.5, 136.6, 131.4, 130.4, 128.7, 128.3, 127.9, 124.0 (Ar), 78.5 (OC(CH₃)₃), 75.0 (Cα Azy), 67.0 (OCH₂), 55.8 (Cα Val), 53.8 (Cγ' Azy), 52.3 (OCH₃), 37.0 (Cβ Azy), 31.2 (Cβ Val), 29.9 (Cβ' Azy), 29.0 (OC(CH₃)₃), 19.1, 17.9 (Cγ Val), 14.7 (β'-CH₃). MS (ES)⁺: 525.21 [M+H]⁺.

### Z-L-Val-(αS,β'R)-Azw(Boc)-OMe (33)

Syrup. Yield: 50% (method B, from **22** and Z-L-Val-OH). Eluents: EtOAc:hexane (1:3). HPLC: t_{R}= 9.04 min (gradient from 50 to 95% of A, in 10 min). ¹H NMR (400 MHz, CDCl₃): δ 7.53-7.12 (m, 10H, Ph, In), 5.45 (d, *J =* 9.3 Hz, 1H, α-NH Val), 5.13 (d, *J =* 12.3 Hz, 1H, OCH₂), 5.00 (d, *J =* 12.3 Hz, 1H, OCH₂), 3.93 (m, 1H, α-H Val), 3.84 (s, 3H, OCH₃), 3.80 (d, *J* = 15.3 Hz, 1H, β-H Azw), 3.74 (m, 1H, γ'-H Azw), 3.54 (m, 1H, γ'-H Azw), 2.73 (m, 1H, β'-H Azw), 2.05 (m,1H, β-H Val), 1.65 (s, 9H, C(CH₃)₃), 1.04 (d, *J =* 7.2 Hz, 3H, β'-CH₃), 1.02 (d, *J =* 6.1 Hz, 3H, γ-H Val), 0.96 (d, *J =* 6.8 Hz, 3H, γ-H Val). ¹³C NMR (100 MHz, CDCl₃): δ 171.1, 170.7 (CO), 156.1 (NHCOO Z), 149.8 (NHCOO, Boc), 136.7, 131.8, 128.8, 128.6, 128.2, 128.1, 125.8, 124.5, 122.7, 119.2, 115.3, 114.7 (Ar), 83.9 (C(CH₃)₃), 75.3 (Cα Azw), 67.0 (OCH₂), 55.7 (Cα Val), 54.0 (Cγ' Azw), 52.4 (OCH₃), 31.2 (Cβ Val), 30.9 (Cβ' Azw), 28.3 (C(CH₃)₃), 27.8 (Cβ Azw), 19.3, 17.5 (Cγ Val), 14.6 (β'-CH₃).MS (ES)⁺: 592.2 [M+H]⁺.

### Boc-L-Tbg-(αS,β'R)-Azl-OMe (34)

Syrup. Yield: 83% (method B, from **19** and Boc-Tbg-OH). Eluents: EtOAc:hexane (1:9). HPLC: t_{R}= 8.10 min (gradient from 40 to 95% of A, in 10 min). ¹H NMR (400 MHz, CDCl₃): δ

5.15 (d, *J =* 10.2 Hz, 1H, α-NH Tbg), 4.39 (t, *J =* 8.3 Hz, 1H, γ'-H Azl), 3.87 (d, *J* = 10.2 Hz, 1H, α-H Tbg), 3.73 (s, 3H, OMe), 3.70 (m, 1H, γ'-H Azl), 2.78 (m, 1H, β'-H Azl), 2.16 (m, 1H, β-H Azl), 1.90 (m , 2H, β-H Azl, γ-H Azl), 1.40 (s, 9H, C(CH₃)₃), 1.04 (d, *J* = 7.4 Hz, 3H, β'-CH₃), 1.03 (s, 9H, γ-H Tbg), 0.97 (d, *J* = 6.6 Hz, 3H, δ-H Azl), 0.89 (d, *J* = 6.6 Hz, 3H, δ-H Azl). ¹³C NMR (100 MHz, CDCl₃): δ, 171.2, 171.1 (CO), 155.9 (NHCO), 79.5 (C(CH₃)₃), 75.3 (Cα Azl), 56.5 (Cα Tbg), 54.6 (Cγ' Azl), 51.9 (OCH₃), 41.5 (Cβ Azl), 35.0 (Cβ Tbg), 31.3 (Cβ' Azl), 28.5 (C(CH₃)₃), 26.4 (Cγ Tbg), 25.3 (Cδ Azl), 24.5 (Cy, Azl), 23.4 (Cδ Azl), 15.1 (β'-CH₃). MS (ES)⁺: 399.0 [M+H]⁺.

### Z-L-Tbg-(αS,β'R)-Azf-OMe (35)

Syrup. Yield: 82% (method B, from **20** and Z-L-Tbg-OH). Eluents: EtOAc:hexane (4:6). HPLC (t_{R}, min): 8.52 (gradient from 40 to 95% of A, in 10 min). ¹H NMR (400 MHz, CDCl₃): δ 7.44-7.33 (m, 5H, Ph Z), 7.15-7.07 (m, 5H, Ph), 5.56 (d, *J* = 10.0 Hz, 1H, α-NH Tbg), 5.17 (d, *J* = 12.2 Hz, 1H, OCH₂), 5.11 (d, *J* = 12.2 Hz, 1H, OCH₂), 3.83 (m, 1H, α-H Tbg), 3.82 (s, 3H, OCH₃), 3.74 (d, *J* = 14.1 Hz, 1H, β-H Azf), 3.51-3.44 (m, 2H, γ'-H Azf), 3.08 (d, *J* = 14.2 Hz, 1H, β-H Azf), 2.59 (m, 1H, β'-H Azf), 1.05 (d, *J* = 6.5 Hz, 3H, β'-CH₃), 1.04 (s, 9H, γ-H Tbg). ¹³C (100 MHz, CDCl₃): δ 170.9, 170.6 (CO), 156.1 (NHCO), 130.9, 128.8, 128.4, 128.33, 128.28, 127.1 (Ar), 77.4 (Cα Azf), 67.1 (OCH₂), 57.1 (Cα Tbg), 54.3 (Cγ' Azf), 52.3 (OCH₃), 37.5 (Cβ Azf), 35.6 (Cβ Tbg), 29.6 (Cβ' Azf), 26.3 (Cγ Tbg), 14.7 (β'-CH₃). MS (ES)⁺: 467.2 [M+H]⁺; 489.2 [M+Na]⁺.

### Boc-L-Tbg-(αS, β'R)-Azf-OMe (36)

Syrup. Yield: 10% (method A), 91% (method B) (from **20** and Boc-Tbg-OH). Eluents: EtOAc:hexane (5:1). HPLC: t_{R}= 9.91 min (gradient from 30 to 95% of A, in 10 min). ¹H NMR (400 MHz, CDCl₃): δ 7.29-7.15 (m, 5H, Ph), 5.34 (d, *J =* 9.9 Hz, 1H, α-NH Tbg), 3.82 (s, 3H, OCH₃), 3.80 (d, *J =* 9.7 Hz, 1H, α-H Tbg), 3.77 (d, *J =* 14.1 Hz, 1H, β-H Azf), 3.48 (m, 2H, γ'-H Azf), 3.10 (d, *J =* 14.2 Hz, 1H, β-H Azf), 2.56 (m, 1H, β'-H Azf), 1.48 (s, 9H, C(CH₃)₃ Boc), 1.04 (d, *J =* 7.0 Hz, 3H, β'-CH₃), 1.03 (s, 9H, γ-H Tbg). ¹³C NMR (100 MHz, CDCl₃): δ 171.2, 170.7 (CO), 156.5 (NHCOO), 135.8, 131.0, 128.4, 127.1 (Ar), 79.5 (C(CH₃)₃), 74.6 (Cα Azf), 56.5 (Cα Tbg), 54.3 (Cγ' Azf), 52.3 (OCH₃), 37.4 (Cβ Azf), 35.8 (Cβ Tbg), 29.6 (Cβ' Azf), 28.6 (C(CH₃)₃), 26.3 (Cγ Tbg), 14.8 (β'-CH₃). MS (ES)⁺: 455.3 [M+Na]⁺.

### Boc-L-Lys(Z)-(αS,β'R)-Azf-OMe (37)

Syrup. Yield: 46% (method HATU, from **20** and Boc-Lys(Z)-OH). Eluents: EtOAc:hexane (1:2). HPLC: t_{R}= 9.43 min (gradient from 30 to 95% of A, in 10 min). ¹H NMR (400 MHz, CDCl₃): δ 7.34-7.16 (m, 10H, Ar), 5.25 (d, *J* = 8.7 Hz, 1H, α-NH Lys), 5.12 (br s, 1H, ε-NH Lys), 5.08 (s, 2H, OCH₂), 4.04 (m, 1H, α-H Lys), 3.74 (s, 3H, OMe), 3.68 (d, *J =* 14.3 Hz, 1H, β-H Azf), 3.51 (t, *J* = 8.2 Hz, 1H, γ'-H Azf), 3.43 (t, *J=* 7.0 Hz, 1H, γ'-H Azf), 3.21 (m, 2H, ε-H Lys), 3.10 (d, *J* = 14.3 Hz, 1H, β-H Azf), 2.63 (q, *J* = 8.2, 7.0 Hz, 1H, β'-H Azf), 1.74 (m, 1H, β-H Lys), 1.61-1.49 (m, 3H, β, δ-H Lys), 1.45 (s, 9H, C(CH₃)₃), 1.41 (m, 2H, γ-H Lys), 1.02 (d, *J* = 7.1 Hz, 3H, β'-CH₃). ¹³C NMR (100 MHz, CDCl₃): δ 171.8, 170.7 (CO), 156.6, 155.5 (NHCOO, Boc,Z), 136.8, 135.7, 131.0, 128.6, 128.4, 128.35, 128.3, 127.1 (Ar), 79.8 (C(CH₃)₃), 75.3 (Cα Azf), 66.7 (OCH₂), 53.7 (Cγ' Azf), 52.5 (OMe), 49.9 (Cα Lys), 40.8 (Cε Lys), 37.8 (Cβ Azf), 32.0 (Cβ Lys), 30.1 (Cβ' Azf), 29.7 (Cδ Lys), 28.5 (C(CH₃)₃), 22.0 (Cγ Lys), 14.5 (β'-CH₃). MS (ES)⁺: 582.4 [M+H]⁺.

### Z-L-Lys(Boc)-(αS,β'R)-Azf-OMe (38)

Syrup. Yield: 55% (method B, from **20** and Z-Lys(Boc)-OH). Eluents: EtOAc:hexane (1:1). HPLC: t_{R}= 8.15 min (gradient from 40 to 95% of A, in 10 min). ¹H NMR (400 MHz, CDCl₃): δ 7.38-7.13 (m, 10H, Ar), 5.52 (d, *J* = 8.7 Hz, 1H, α-NH Lys), 5.14 (d, *J* = 12.3 Hz, 1H, OCH₂), 5.09 (d, *J* = 12.3 Hz, OCH₂), 4.73 (br s, 1H, ε-NH Lys), 4.08 (m,2H, α-H Lys), 3.85 (s, 3H, OMe), 3.68 (d, *J* = 14.2 Hz, 1H, β-H Azf), 3.52 (t, *J* = 7.1 Hz, 1H, γ'-H Azf), 3.45 (t, *J* = 7.1 Hz, 1H, γ'-H Azf), 3.11 (m, 2H, ε-H Lys), 2.64 (m, 1H, β'-H Azf), 1.79 (m, 1H, β-H Lys), 1.61 (m, 1H, β-H Lys), 1.51-1.39 (m, 13H, C(CH₃)₃, γ,δ-H Lys), 1.04 (d, *J* = 7.2 Hz, 3H, β'-CH₃), ¹³C NMR (100 MHz, CDCl₃): δ 171.3, 170.6 (CO), 156.2, 155.9 (NHCOO, Boc,Z), 136.5, 135.6, 131.0, 128.7, 128.4, 128.3, 128.1, 127.1 (Ar), 79.2 (C(CH₃)₃), 75.2 (Cα Azf), 67.0 (OCH₂), 53.4 (Cγ' Azf), 52.5 (OMe), 50.5 (Cα Lys), 40.3 (Cε Lys), 37.8 (Cβ Azf), 31.9 (Cβ Lys), 30.0 (Cβ' Azk), 29.7 (Cδ Lys), 28.6 (C(CH₃)₃), 22.0 (Cγ Lys), 14.5 (β'-CH₃). MS (ES)⁺: 604.3 [M+Na]⁺.

### Z-L-Tbg-(αS,β'R)-Azk(Boc)-OMe (39)

Syrup. Yield: 84% (method C, from **23** and Z-Tbg-OH). Eluents: EtOAc:hexane (1:3). HPLC: t_{R}= 9,26 min (gradient from 30 to 95% of A, in 10 min). ¹H NMR (400 MHz, CDCl₃): δ 7.35-7.32 (m, 5H, Ph), 5.49 (br s, 1H, ε-NH Azk), 5.12 (d, *J* = 12.5 Hz, 1H, OCH₂), 5.08 (d, *J=* 12.3 Hz, 1H, OCH₂), 4.82 (brs, 1H, α-NH Tbg), 4.34 (t, *J* = 8.3 Hz, 1H, γ'-H Azk), 3.95 (d, *J* = 9.7 Hz, 1H, α-H Tbg), 3.75 (s, 3H, OMe), 3.71 (m, 1H, γ'-H Azk), 3.13 (m, 1H, ε-H Azk), 3.03 (m, 1H, ε-H Azk), 2.69 (q, *J* = 7.3 Hz, 1H, β'-H Azk), 2.24 (m, 1H, β-H Azk), 1.94-1.85 (m, 2H, β-H Azk, δ-H Azk), 1.51 (m, 1H, γ-H Azk), 1.46-1.42 (m, 10H,C(CH₃)₃, δ-H Azk), 1.22 (m, 1H, γ-H Azk), 1.05 (m, 10H, γ-H Tbg, β'-CH₃), ¹³C NMR (100 MHz, CDCl₃): δ 170.9, 170.8 (CO), 156.6, 156.2 (NHCOO, Boc, Z), 136.5, 128.7, 128.2, 128.1 (Ar), 79.2 (C(CH₃)₃), 75.1 (Cα Azk), 67.0 (OCH₂), 57.3 (Cα Tbg), 54.6 (Cγ' Azk), 52.1 (OMe), 39.8 (Cε Azk), 35.1 ( Cβ Tbg), 32.5 (Cβ Azk), 31.2 (Cβ' Azk), 29.4 (Cδ Azk), 28.5 (C(CH₃)₃), 26.3 (Cγ Tbg), 20.1 (Cγ Azk), 15.0 (β'-CH₃). MS (ES)⁺: 548.2 [M+H]⁺.

### Boc-L-Val-(αS, β'R)- Aza(Cy)-OMe (40)

Syrup. Yield: 81% (method B, from **24** and Boc-L-Val-OH). Eluents: EtOAc:hexane (1:4). HPLC (t_{R}, min): 4.86 (gradient from 60 to 95% of A, in 10 min). ¹H NMR (400 MHz, CDCl₃): δ 5.14 (d, *J* = 9.6 Hz, 1H, α-NH Val), 4.33 (t, *J* = 8.3 Hz, 1H, γ'-H Aza), 3.89 (dd, *J* = 9.6, 7.2 Hz, 1H, α-H Val), 3.73 (s, 3H, OCH₃), 3.68 (dd, *J* = 8.0, 6.7 Hz, 1H, γ'-H Aza), 2.77 (m, 1H, β'-H Aza), 2.10 (dd, *J* = 15.0, 6.7 Hz, 1H, β-H Aza), 1.98 (m, 1H, β-H Val), 1.90 (dd, *J* = 15.0, 4.5 Hz, 1H, β-H Aza), 1.73-1.61 (m, 2H, 2, 6-H Cy), 1.58 (m, 1H, 1-H Cy), 1.42 (s, 9H, C(CH₃)₃), 1.31-1.07 (m, 5H, 2, 3, 4, 5, 6-H Cy), 1.04 (d, *J* = 7.4 Hz, 3H, β'-CH₃), 1.01 (d, *J* = 6.7 Hz, 3H, γ-H Val), 0.95 (d, *J* = 6.8 Hz, 3H, γ-H Val). ¹³C (100 MHz, CDCl₃): δ 171.4, 171.0 (CO), 155.9 (NHCOO), 79.5 (C(CH₃)₃), 75.5 (Cα Aza), 55.3 (Cα Val), 54.0 (Cγ' Aza), 52.0 (OCH₃), 41.1 (Cβ Aza), 35.6, 34.1 (C2-, C6- Cy), 33.8 (C1 Cy), 32.3 (CP'Aza), 30.8 (Cβ Val), 28.5 (C(CH₃)₃), 26.5, 26.4, 26.3 (C3-, C4-, C5- Cy), 19.3, 17.9 (Cγ Val), 14.9 (β'-CH₃). MS (ES)⁺: 425.4 [M+H]⁺.

### Boc-L-Tbg-(αS, β'R)-Aza(Cy)-OMe (41)

Syrup. Yield: 58% (method B, from **24**) and Boc-L-Tbg-OH). Eluents: EtOAc:hexane (1:4). HPLC (t_{R}, min): 10.14 (gradient from 40 to 95% of A, in 10 min). ¹H NMR (400 MHz, CDCl₃): δ 5.19 (d, *J* = 9.6 Hz, 1H, α-NH Tbg), 4.35 (t, *J* = 8.3 Hz, 1H, γ'-H Aza), 3.90 (d, *J* = 10.0 Hz, 1H, α-H Tbg), 3.74 (s, 3H, OMe), 3.71 (m, 1H, γ'-H Aza), 2.76 (m, 1H, β'-H Aza), 2.15 (dd, *J* = 15.0, 6.5 Hz, 1H, β-H Aza). 1.89 (dd, *J* = 15.0, 4.5 Hz, 1H, β-H Aza), 1.79-1.59 (m, 6H, 1, 2, 3, 4, 5, 6-H Cy), 1.29-1.09 (m, 3H, 3, 4, 5-H Cy), 1.08-1.07 (m 2H, 2, 6-H Cy), 1.04 (d, *J* = 7.8 Hz, 3H, β'-CH₃), 1.02 (s, 9H, γ-H Tbg). ¹³C (100 MHz, CDCl₃): δ 171.1, 170.9 (CO), 155.8 (NHCOO), 79.5 (C(CH₃)₃), 75.3 (Cα Aza), 56.5 (Cα Tbg), 54.6 (Cγ' Aza), 51.9 (OCH₃), 40.3 (Cβ Aza), 35.7, 35.3 (C2- , C6- Cy), 34.1 (C1 Cy), 33.7 (Cβ Tbg), 31.6 (CP'Aza), 28.5 (C(CH₃)₃), 26.6, 26.5, 26.4 (C3-, C4-, C5- Cy), 26.3 (Cγ Tbg), 15.1 (β'-CH₃).MS (ES)⁺: 439.3 [M+H]⁺.

### Boc-L-β-Val-(αS,β'R)-Azf-OMe (42)

Syrup. Yield: 64% (method B, from **20** and (*R*)-3-((tert-butoxycarbonyl)amino)-4-methylpentanoic acid). Eluents: EtOAc:hexane (1:5). HPLC: t_{R}= 8.87 min (gradient from 30 to 95% of A, in 10 min). ¹H NMR (400 MHz, CDCl₃): δ 7.35-7.18 (m, 5H, Ph), 5.54 (br s, 2H, 1-H, β-NH β-Val), 3.8 (s, 3H, OMe), 3.64 (d, *J* = 14.2 Hz, 1H, β-H Azf), 3.46 (t, *J* = 7.0 Hz, 1H, γ'-H Azf), 3.33 (t, *J* = 8.0 Hz, 1H, γ'-H Azf), 3.11 (d, *J* = 14.2 Hz, 1H, β-H Azf), 2.60 (q, *J =* 7.3 Hz, 1H, β'-H Azf), 2.33 (dd, *J* = 15.4, 5.0 Hz, 1H, α-H β-Val), 2.16 (dd, *J* = 15.3, 5.2 Hz, 1H, α-H β-Val), 2.03 (m, 1H, β-H β-Val), 1.44 (s, 9H, C(CH₃)₃), 1.03 (d, *J* = 7.1 Hz, 3H, β'-CH₃), 0.94 (d, *J* = 7.1 Hz, 3H, γ-H β-Val), 0.93 (d, *J* = 7.1 Hz, 3H, γ-H β-Val). ¹³C NMR (100 MHz, CDCl₃): δ 171.1, 170.8 (CO), 155.9 (NHCOO), 136.1, 131.0, 128.4, 127.0 (Ar), 78.9 (C(CH₃)₃), 74.9 (Cα Azf), 53.8 (Cγ' Azf), 53.7 (Cβ β-Val), 52.3 (OMe), 37.9 (Cβ Azf), 33.1 (Cα β-Val), 31.1 (Cγ β-Val), 29.6 (Cβ' Azf), 28.6 (C(CH₃)₃), 19.8 (Cδ β-Val), 19.2 (Cδ β-Val), 14.3 (β'-CH₃). MS (ES)⁺: 433.3 [M+H]⁺.

### General Procedure for the Synthesis of tripeptides Derivatives.

A solution of the corresponding 2-methoxycarbonyl dipeptide derivatives (1.0 eq) in MeOH/THF (1:2) (5/10 mL/mmol) was treated with 2 N NaOH (1.5 eq) and stirred at rt until disappearance of the starting material (24-72h). Then, the solvent was evaporated to dryness, and the residue was dissolved in H₂O and washed with EtOAc. The aqueous phase was acidified with 1 M HCl to pH 3 and extracted with EtOAc. This second organic phase was separated, dried over Na₂SO₄ and evaporated to dryness to afford the corresponding carboxylic acid, which was used in the following coupling without further purification.

***Method D.*** The corresponding dipeptide carboxylic acid (1.0 eq) was dissolved in THF (5 mL/mmol) and H-L-Ala(3*S*-2-oxopyrrolidin-3-yl)-NH₂.HCl or H-L-Ala(3*S*-2-oxopyrrolidin-3-yl)-OMe.HCl (1.2 eq), PyBrop (1.2 eq) and TEA (2.4 eq) were added. After being stirred for 24 h at rt, the solvent was evaporated under vacuum, the residue was dissolved in CH₂Cl₂, and washed successively with a solution of citric acid (10%), NaHCOs (10%), H₂O, and brine. The organic phase was dried over Na₂SO₄ and evaporated to dryness. The resulting residue was purified by column chromatography on silica gel as specified in each case.

***Method E.*** To a solution of the corresponding carboxylic acid (1.0 eq) in DMF (5 mL/mmol) or THF (5mL/mmol), HATU (1.2 eq) was added, at 0 ºC, and the mixture was stirred for 5 min at this temperature. Then, H-L-Ala(3S-2-oxopyrrolidin-3-yl)-NH₂.HCl, H-L-Ala(3S-2-oxopyrrolidin-3-yl)-OMe.HCl, H-His(Trt)-OMe.HCl or H-Nle-OMe.HCl (1.2 eq) was added and stirred 10 min at this temperature, and finally, DIPEA (2.4 eq) was added dropwise and the reaction was stirred overnight at rt. The solvent was evaporated under reduced pressure and the residue was redissolved in EtOAc or CH₂Cl₂, washed with 10% citric acid, 10% NaHCOs solution and brine. The organic layer was dried over Na₂SO₄, filtered and the solvent was evaporated under reduced pressure. The resulting residue was purified on a silica gel column, as specified in each case.

***Method F.*** The corresponding methoxycarbonyl tripeptide derivative (0.15 mmol) was treated with excess 7N NH₃/MeOH (4 mL) and stirred ar rt until disapearance of the starting material. Then, the reaction was evaporated to dryness and the crude liophylized (ACN/H₂O, 1:2). Purification as specified in each case.

### Z-L-Val-(αS,β'R)-Azl-L-Ala(3S-2-oxopyrrolidin-3-yl)-NH₂ (43)

Syrup. Yield: 72% (method D, from **28**). Eluents: MeOH:DCM (1:20). HPLC: t_{R}= 6.76 min (gradient from 10 to 95% of A, in 10 min). ¹H NMR (400 MHz, CDCl₃): δ 8.26 (d, *J =* 7.0 Hz, 1H, α-NH Ala), 7.49 (br s, 1H, CONH₂), 7.25 (s, 5H, Ph), 6.87 (br s, 1H, CONH₂), 6.78 (br s, 1H, 1-H), 6.38 (d, *J =* 9.2 Hz, α-NH Val), 5.07 (d, *J =* 12.4 Hz, 1H, OCH₂), 5.03 (d, *J =* 12.4 Hz, 1H, OCH₂), 4.55 (t, *J =* 8.8 Hz, 1H, γ'-H Azl), 4.47 (m, 1H, α-H Ala), 4.04 (m, 1H, α-H Val), 3.77 (m, 1H, γ'-H Azl), 3.32 (m, 2H, 5-H), 2.65 (m, 1H, 3-H), 2.39-2.34 (m, 2H, β'-H Azl, 4-H), 2.16-1.96 (m, 1H, β-H Ala, β-H Val), 1.91-1.74 (m, 3H, β-H Ala, γ-H Azl, 4-H), 1.12 (d, *J =* 7.1 Hz, 3H, β'-CH₃), 0.97 (d, *J =* 6.8 Hz, 3H, γ-H Val), 0.93 (d, *J =* 6.8 Hz, 3H, γ-H Val), 0.90 (d, *J =* 6.8 Hz, 3H, δ-H Azl), 0.80 (d, *J =* 6.6 Hz, 3H, δ-H Azl). ¹³C NMR (100 MHz, CDCl₃): δ 180.5, 175.3, 173.0, 170.8 (CO), 156.0 (NHCOO), 136.6, 128.5, 128.1, 127.9 (Ar), 77.9 (Cα Azl), 66.9 (OCH₂), 56.4 (Cα Val), 54.6 (Cγ' Azl), 53.9 (Cα Ala), 42.1 (Cβ Azl), 40.8 (C5), 39.7 (C3), 32.7 (Cβ Ala), 31.5 (Cβ' Azl), 29.6 (Cβ Val), 29.3 (C4), 25. 25.4 (Cδ Azl), 24.0 (Cγ Azl), 23.9 (Cδ Azl), 19.6 (Cγ Val), 17.9 (Cγ Val), 15.5 (β'-CH₃).. MS (ES)⁺: 558.3 [M+H]⁺.

### Z-L-Val-(αS,β'R)-Azl-L-Ala(3S-2-oxopyrrolidin-3-yl)-NH₂ (44)

Syrup. Yield: 70% (method D, from **29**). Eluents: MeOH:DCM (1:20). HPLC: t_{R}= 6.78 min (gradient from 10 to 95% of A, in 10 min). ¹H NMR (400 MHz, CDCl₃): δ 8.15 (d, *J =* 7.4 Hz, 1H, α-NH Ala), 7.38-7.28 (m, 5H, Ph), 7.09 (br s, 1H, CONH₂), 5.99 (br s, 1H, CONH₂), 5.97 (brs, α-NH Val), 5.96 (br s, 1H, 1-NH), 5.16 (d, *J =* 12.2 Hz, 1H, OCH₂), 5.11 (d, *J =* 12.2 Hz, 1H, OCH₂), 4.55 (m, 1H, α-H Ala), 4.19 (t, *J =* 8.5 Hz, 1H, α-H Val), 4.04 (m, 2H, γ'-H Azl), 3.23 (m, 2H, H5), 2.66 (m, 1H, H3), 2.50 (m, 1H, β'-H Azl), 2.31-1.71 (m, 8H, 4-H, β-H Ala, β-Azl, γ-Azl), 1.17 (d, *J =* 7.1 Hz, 3H, β'-CH₃), 1.01 (d, *J =* 7.0 Hz, 3H, γ-H Val), 0.98 (d, *J =* 7.0 Hz, 3H, γ-H Val), 0.96 (d, *J =* 7.0 Hz, 3H, δ-H Azl), 0.95 (d, *J =* 7.0 Hz, 3H, δ-H Azl). ¹³C NMR (100 MHz, CDCl₃): δ 179.9, 174.3, 172.3, 170.9 (CO), 157.2 (NHCO), 136.4, 128.7, 128.3, 128.1 (Ar), 77.9 (Cα Azl), 67.3 (OCH₂), 56.6 (Cα Val), 54.0 (Cγ' Azl), 51.8 (Cα Val), 44.9 (CβAzl), 40.4 (C5), 37.5 (C3), 34.3 (Cβ Ala), 33.1 (Cβ' Azl), 30.5 (Cβ Val), 28.0 (C4), 25.1, 25.0 (Cδ Azl), 23.6 (Cγ Azl), 19.5, 18.3 (Cγ Val), 16.0 (β'-CH₃). MS (ES)⁺: 580.3 [M+Na]⁺.

### Z-L-Val-(αS, β'R)-Azf-Ala(3S-2-oxopyrrolidin-3-yl)-NH₂ (45)

Syrup. Yield: 70% (method E, from **30**). Eluents: MeOH:DCM: (1:9). HPLC (t_{R}, min): 6.88 (gradient from 10 to 95% of A, in 10 min). ¹H NMR (400 MHz, CDCl₃): δ 8.55 (d, *J* = 6.5 Hz, 1H, α-NH Ala), 7.55 (s, 1H, α-CONH₂), 7.41-7.30 (m, 5H, Ph), 7.18-7.08 (m, 5H, Ph), 5.88 (s, 1H, 1-H), 5.82 (s, 1H, α-CONH₂), 5.68 (d, *J* = 9.3 Hz, α-NH Val), 5.17 (d, *J =* 12.4 Hz, 1H, OCH₂), 5.12 (d, *J=* 12.3 Hz, 1H, OCH₂), 4.52 (ddd, *J* = 11.4, 6.4, 4.7 Hz, 1H, α-H Ala), 4.03 (dd, *J* = 9.3, 5.5 Hz, 1H, α-H Val), 3.55 (d, *J* = 14.2 Hz, 1H, β-H Azf), 3.47 (dd, *J* = 8.4, 4.8 Hz, 1H, γ'-H Azf), 3.37 (m, 2H, 5-H), 3.31 (t, *J* = 8.4 Hz, 1H, γ'-H Azf), 3.22 (d, *J* = 14.3 Hz, 1H, β-H Azf), 2.60 (m, 1H, β'-H Azf), 2.48-2.39 (m, 2H, 3-H, 4-H), 2.24-2.16 (m, 2H, β-H Val, β-H Ala), 1.98-1.88 (m, 2H, β-H Ala , 4-H), 1.12 (d, *J* = 7.2 Hz, 3H, β'-CH₃), 0.98 (d, *J* = 6.8 Hz, 3H, γ-H Val), 0.89 (d, *J* = 6.7 Hz, 3H, γ-H Val). ¹³C (100 MHz, CDCl₃): δ 180.3, 174.7, 173.3, 170.3 (CO), 156.4 (COO), 136.6, 135.7, 130.9, 128.7, 128.5, 128.3, 128.2, 127.2 (Ar), 77.4 (Cα Azf), 67.2 (OCH₂), 56.2 (Cα Val), 54.1 (Cα Ala), 53.8 (Cγ' Azf), 40.9 (C5), 39.6 (C3), 37.7 (Cβ Azf), 32.7 (Cβ Ala), 30.2 (Cβ Val), 30.1 (Cβ' Azf), 29.4 (C4), 19.9, 17.2 (Cγ Val), 15.2 (β'-CH₃). MS (ES)⁺: 592.3 [M+H]⁺.

### Z-L-Val-(αS, β'R)-Azy-LAla(3S-2-oxopyrrolidin-3-yl)-NH₂ (46)

Syrup. Yield: 73% (method E, from 31). Eluents: MeOH:DCM (1:9). HPLC (t_{R}, min): 7.06 (gradient from 10 to 95% of A, in 10 min). ¹H NMR (400 MHz, CDCl₃): δ 8.58 (d, *J* = 6.5 Hz, 1H, α-NH Ala), 7.55 (s, 1H, α-CONH₂ Ala), 7.40-7.28 (m, 5H, Ph), 6.90 (d, *J* = 8.4 Hz, 2H, C₆H₄), 6.55 (d, *J* = 8.4 Hz, 2H, C₆H₄), 6.43 (s, 1H, 1-H), 6.19 (s, 1H, α-CONH₂ Ala), 6.04 (d, *J* = 9.1 Hz, α-NH Val), 5.15 (m, 2H, OCH₂), 4.58 (m, 1H, α-H Ala), 4.06 (dd, *J* = 9.5, 6.0 Hz, 1H, α-H Val), 3.50-3.30 (m, 5H, β-H Azy, γ'-H Azy, 5-H), 3.10 (d, *J* = 14.5 Hz, 1H, β-H Azy), 2.50 (m, 1H, β'H Azy), 2.43-2.36 (m, 2H, H3, 4-H), 2.21-2.17 (m, 2H, β-H Val, β-H Ala), 1.93-1.84 (m, 2H, β-H Ala, 4-H), 1.09 (d, *J* = 7.2 Hz, 3H, β'-CH₃), 0.98, 0.89 (d, *J* = 6.7 Hz, 3H, γ-H Val). ¹³C (100 MHz, CDCl₃): δ 180.5, 175.1, 173.4, 170.5 (CO), 156.5 (NHCOO), 155.0, 136.9, 131.9, 128.7, 128.31, 128.26, 127.3, 115.6 (Ar), 76.9 (Cα Azy), 67.1 (OCH₂), 56.0 (Cα Val), 54.4 (Cα Ala), 54.0 (Cγ' Azy), 41.0 (C5), 39.9 (C3), 37.0 (Cβ Azy), 32.6 (Cβ Ala), 30.1 (Cβ Val), 30.0 (Cβ' Azy), 29.5 (C4), 19.8, 17.2 (Cγ Val), 15.1 (β'-CH₃). MS (ES)⁺: 608.4 [M+H]⁺.

### Z-L-Val-(αS, β'R)-Azy(tBu)-L-Ala(3S-2-oxopyrrolidin-3-yl)-NH₂ (47)

Syrup. Yield: 72% (method E, from 32). Eluents: MeOH:DCM (17:83). HPLC (t_{R}, min): 8.91 (gradient from 10 to 95% of A, in 10 min). ¹H NMR (400 MHz, CDCl₃): δ 8.47 (d, *J* = 6.7 Hz, 1H, α-NH Ala), 7.50 (s, 1H, α-CONH₂ Ala), 7.40-7.28 (m, 5H, Ph), 7.02 (d, *J* = 8.4 Hz, 2H, C₆H₄), 6.85 (d, *J* = 8.4 Hz, 2H, C₆H₄), 5.89 (s, 1H, 1-H), 5.87 (s, 1H, α-CONH₂ Ala), 5.76 (d, *J* = 9.2 Hz, α-NH Val), 5.16 (s, 2H, OCH₂), 4.52 (ddd, *J* = 11.5, 6.7, 4.7 Hz, 1H, α-H Ala), 4.04 (dd, *J* = 9.2, 5.5 Hz, 1H, α-H Val), 3.54 (d, *J* = 14.4 Hz, 1H, β-H Azy), 3.45 (d, *J* = 8.3, 4.6 Hz, 1H, γ'-H Azy), 3.38-3.29 (m, 3H, γ'-H Azy, 5-H), 3.19 (d, *J* = 14.5 Hz, 1H, β-H Azy), 2.60 (dq, *J* = 11.9, 7.1 Hz, 1H, β'-H Azy), 2.46-2.37 (m, 2H, 3-H, 4-H), 2.23-2.15 (m, 2H, β-H Val, β-H Ala), 1.99-1.86 (m, 2H, β-H Ala, 4-H), 1,31 (s, 9H, C(CH₃)₃), 1.11 (d, *J* = 7.2 Hz, 3H, β'-CH₃), 0.97 (d, *J* = 6.8 Hz, 3H, γ-H Val), 0.88 (d, *J* = 6.7 Hz, 3H, γ-H Val). ¹³C (100 MHz, CDCl₃): δ 180.2, 174.5, 173.3, 170.4 (CO), 156.4 (NHCOO), 154.6, 136.7, 131.4, 130.4, 128.7, 128.2, 127.9, 124.2 (Ar), 78.5 (C(CH₃)₃), 77.0 (Cα Azy), 67.1 (OCH₂), 56.4 (Cα Val), 54.0, 53.9 (Cα Ala, Cy' Azy), 40.8 (C5), 39.5 (C3), 37.0 (Cβ Azy), 32.8 (Cβ Ala), 30.2 (Cβ Val), 30.0 (Cβ' Azy), 29.2 (C4), 19.0 (OC(CH₃)₃), 19.9, 17.2 (Cγ Val), 15.2 (β'-CH₃). MS (ES)⁺: 664.35 [M+H]⁺.

### Z-L-Val-(αS,β'R)-Azw-L-Ala(3S-2-oxopyrrolidin-3-yl)-NH₂ (48)

Syrup. Yield: 50% (method E, from 33). Eluents: MeOH:DCM (1:15). HPLC: t_{R}= 5.56 min (gradient 30 to 95% of A, in 10 min). ¹H NMR (400 MHz, CDCl₃): δ 8.48 (d, *J* = 6.9 Hz, 1H, α-NH Ala), 7.58-7.03 (m, 11H, Ph, NH In), 6.49 (br s, 1H, α-CONH₂ Ala), 6.37 (d, *J* = 9.9 Hz, α-NH Val), 6.32 (br s, 1H, α-CONH₂ Ala), 5.83 (br s, 1H,1-H), 5.32 (d, *J* = 12.5 Hz, 1H, OCH₂), 5.05 (d, *J* = 12.5 Hz, 1H, OCH₂), 4.56 (m, 1H, α-H Ala), 4.09 (m, 1H, α-H Val), 3.58 (d, *J* = 15.2 Hz, 1H, β-H Azw), 3.52 (d, *J* = 15.2 Hz, 1H, β-H Azw), 3.39-3.28 (m, 4H, 5-H, γ'-H Azl), 2.59 (m, 1H, H3), 2.39-2.34 (m, 2H, β'-H Azl, 4-H), 2.27-2.19 (m, 2H, β-H Val, β-H Ala), 1.94-1.86 (m, 2H, 4-H, β-H Ala), 1.08 (d, *J* = 7.1 Hz, 3H, β'-CH₃), 0.98, 0.90 (d, *J* = 6.8 Hz, 3H, γ-H Val). ¹³C NMR (100 MHz, CDCl₃): δ 180.3, 174.8, 174.1, 170.6 (CO), 156.8 (NHCOO), 137.5, 135.8. 129.0, 128.7, 128.55, 128.5, 125.0, 121.8, 119.6, 118.7, 111.4, 108.9 (Ar), 78.6 (Cα Azw), 67.0 (OCH₂), 56.1 (Cα Val), 54.2 (Cα Ala), 54.0 (Cγ' Azl), 40.8 (C5), 39.5 (C3), 32.6 (Cβ Ala), 30.1 (Cβ Val), 29.4 (C4), 27.4 (Cβ Azw), 19.9, 16.9 (Cγ Val), 15.3 (β'-CH₃). MS (ES)⁺: 631.4 [M+H]⁺.

### Boc-L-Tbg-(αS,β'R)-Azl-L-Ala(3S-2-oxopyrrolidin-3-yl)-NH₂ (49)

Syrup. Yield: 55% (method E, from **34**). Eluents: MeOH:DCM (1:20). HPLC: t_{R}= 7.43 min (gradient from 15 to 95% of A, in 10 min). ¹H NMR (400 MHz, CDCl₃): δ 8.00 (d, *J* = 7.7 Hz, 1H, α-NH Ala), 7.35 (br s, 1H, CONH₂), 5.67 (br s, 1H, 1-H), 5.42 (br s, 1H, CONH₂), 5.11 (d, *J* = 9.6 Hz, 1H, α-NH Tbg), 4.64 (q, *J* = 7.8 Hz, 1H, α-H Ala), 4.51 (t, *J* = 8.5 Hz, 1H, γ'-H Azl), 3.93 (d, *J* = 9.7 Hz, 1H, , α-H Tbg), 3.65 (m, 1H, γ'-H Azl), 3.36 (M, 2H, 5'-H), 2.66 (m, 1H, 3-H), 2.47-2.36 (m, 3H, β'-H Azl, 4-H, γ-H Azl), 2.15-1.98 (m, 2H, β-H Azl), 1.86 (m, 1H, 4-H), 1.43 (s, 9H, C(CH₃)₃), 1.19 (d, *J* = 7.1 Hz, 3H, β'-CH₃), 1.04 (s, 9H, Cγ Tbg), 0.96, 0.92 (d, *J* = 6.6 Hz, 3H, δ-H Azl). ¹³C NMR (100 MHz, CDCl₃): δ 179.8, 173.8, 173.2, 171.1 (CO), 156.0 (NHCOO), 80.1 (C(CH₃)₃), 77.7 (Cα Azl), 57.4 (Cα Tbg), 54.9 (Cγ' Azl), 52.0 (Cα Ala), 42.8 (Cβ Azl), 40.7 (C5), 38.4 (C3), 34.5 ( Cβ Tbg), 33.6 (Cβ Ala), 32.2 (Cβ' Azl), 28.5 (C(CH₃)₃), 28.4 (C4), 26.5 (Cγ Tbg), 25.2 (Cδ Azl), 24.4 (Cy, Azl), 23.7 (Cδ Azl), 16.4 (β'-CH₃). MS (ES)⁺: 538.3 [M+H]⁺.

### Z-L-Tbg-(αS, β'R)-Azf-L-Ala(3S-2-oxopyrrolidin-3-yl)-NH₂ (50)

Syrup. Yield: 75% (method E, from **35**). Eluents: MeOH:DCM (1:9). HPLC (t_{R}, min): 7.92 (gradient from 15 to 95% of A, in 10 min). ¹H NMR (400 MHz, CDCl₃): δ 8.00 (d, *J* = 6.5 Hz, 1H, α-NH Ala), 7.51 (s, 1H, α-CONH₂ Ala), 7.43-7.32 (m, 5H, Ph), 7.15-7.08 (m, 5H, Ph), 6.07 (s, 1H, 1-H), 5.71 (s, 1H, α-CONH₂ Ala), 5.57 (d, *J* = 9.5 Hz, α-NH Tbg), 5.15 (s, 2H, OCH₂), 4.69-4.64 (m, 1H, α-H Ala), 3.91 (d, *J* = 9.6, 1H, α-H Tbg), 3.61 (d, *J* = 14.1 Hz, 1H, β-H Azf), 3.42-3.31 (m, 4H, γ'-H Azf, 5-H), 3.18 (d, *J* = 14.2 Hz, 1H, β-H Azf), 2.60 (m, 1H, β'-H Azf), 2.50-2.40 (m, 2H, 3-H, 4-H), 2.20-1.88 (m, 3H, 4-H, β-H Ala), 1.15 (d, *J* = 6.9 Hz, 3H, γ'-CH₃ Azf), 1.03 (s, 9H, γ-H Tbg). ¹³C (100 MHz, CDCl₃): δ 180.0, 174.0, 172.8, 170.5 (CO), 156.1 (NHCOO), 136.4, 135.5, 130.9, 128.8, 128.4, 128.3, 127.2 (Ar), 77.5 (Cα Azf), 67.3 (OCH₂), 58.0 (Cα Tbg), 54.5 (Cα Ala), 52.8 (Cγ' Azf), 40.8 (C5), 39.8 (C3), 38.1 (Cβ Azf), 35.5 (Cβ Tbg), 33.5 (Cβ Ala), 30.0 (Cβ' Azf), 28.7 (C4), 26.5 (Cγ Tbg), 15.7 (β'-CH₃). MS (ES)⁺: 606.4 [M+H]⁺.

### Boc-L-Tbg-(αS,β'R)-Azf -L-Ala(3S-2-oxopyrrolidin-3-yl)-NH₂ (51)

Syrup. Yield: 37% (method D), 72% (method E) (from **36**). Eluents: MeOH:DCM (1:20). HPLC: t_{R}= 7.07 min (gradient from 10 to 95% of A, in 10 min). ¹H NMR (400 MHz, CDCl₃): δ 8.09 (d, *J* = 7.4 Hz, 1H, α-NH Ala), 7.48 (br s, 1H, CONH₂), 7.31-7.17 (m, 5H, Ph), 5.69 (br s, 1H,1-H), 5.46 (br s, 1H, CONH₂), 5.20 (d, *J* = 9.7 Hz, 1H, α-NH Tbg), 4.65 (m, 1H, α-H Ala), 3.88 (d, *J* = 9.7 Hz, 1H, α-H Tbg), 3.64 (d, *J* = 14.2 Hz, 1H, β-H Azf), 3.42-3.30 (m, 4H, 5-H, γ'-H Azf), 3.21 (d, *J* = 14.2 Hz, 1H, β-H Azf), 2.57 (m, 1H, 3-H), 2.49-2.37 (m, 2H, β'-H Azf, 4-H), 2.06 (m, 2H, β-H Ala), 1.90 (m, 1H, 4-H), 1.47 (s, 9H, C(CH₃)₃ Boc), 1.15 (d, *J* = 7.1 Hz, 3H, β'-CH₃). 1.02 (s, 9H, γ-H Tbg). ¹³C NMR (100 MHz, CDCl₃): δ 179.7, 173.9, 173.0, 170.5 (CO), 155.4 (NHCOO), 135.8, 131.0, 128.5, 127.2 (Ar), 80.0 (C(CH₃)₃ Boc), 77.3 (Cα Azf), 57.4 (Cα Tbg), 54.5 (Cγ' Azf), 52.9 (Cα Ala), 40.5 (C5), 38.7 (C3), 38.1 (Cβ' Azf), 35.6 (Cβ Tbg), 33.5 (Cβ Ala), 30.1 (Cβ Azf), 28.8 (C4), 28.6 (C(CH₃)₃ Boc), 26.5 (Cγ Tbg), 15.7 (β'-CH₃). MS (ES)⁺: 572.3 [M+H]⁺.

### Boc-L-Lys(Z)-(αS,β'R)-Azf-L-Ala(3S-2-oxopyrrolidin-3-yl)-NH₂ (52)

Syrup. Yield: 45% (method E, from **37**). Eluents: MeOH:DCM (1:20). HPLC: t_{R}= 8.53 min (gradient from 15 to 95% of A, in 10 min). ¹H NMR (400 MHz, CDCl₃): δ 8.89 (br s, 1H, α-NH Ala), 7.71 (br s, 1H, α-CONH₂ Ala), 7.37-7.12 (m, 10H, Ar), 6.71 (br s, 1H, α-CONH₂ Ala), 5.83 (br s, 1H,1-H), 5.22 (d, *J* = 8.7 Hz, 1H, ε-NH Lys), 5.21 (brs, 1H, α-NH Lys), 5.07 (s, 2H, OCH₂), 4.52 (m, 1H, α-H Ala), 4.07 (m, 1H, α-H Lys), 3.56 (d, *J* = 14.3 Hz, 1H, β-H Azf), 3.41 (dd, *J* = 8.2, 4.5 Hz, 1H, γ'-H Azf), 3.32-3.15 (m, 6H, 5-H, γ'-H Azf, β-H Azf, ε-H Lys), 2.60 (m, 1H, β'-H Azf), 2.46-2.36 (m, 2H, 3-H, 4-H), 2.19(m, 1H, β-H Ala), 2.08-1.80 (m, 4H, β-H Ala, β-H Lys, 4-H), 1.64-1.49 (m, 4H, δ-H Lys, γ-H Lys), 1.48 (s, 9H, C(CH₃)₃), 1.12 (d, *J* = 7.1 Hz, 3H, β'-CH₃), ¹³C NMR (100 MHz, CDCl₃): δ 175.2, 175.1, 173.6, 170.1 (CO), 156.5, 156.4 (NHCOO, Boc, Z), 136.6, 136.0, 131.0, 128.7, 128.5, 128.4, 128.2, 127.2 (Ar), 80.0 (C(CH₃)₃), 76.4 (Cα Azf), 66.9 (OCH₂), 54.2 (Cα Ala), 53.8 (Cγ' Azf), 50.5 (Cα Lys), 41.0 (C5), 40.6 (Cε Lys), 39.5 (C3), 37.5 (Cβ Azf), 32.4 (Cβ Ala), 30.1 (Cβ' Azf), 29.55 (Cβ Lys), 29.5 (Cδ Lys), 28.5 (C(CH₃)₃), 28.5 (C4), 22.2 (Cγ Lys), 15.2 (β'-CH₃). MS (ES)⁺: 721.4 [M+H]⁺.

### Z-L-Lys(Boc)-(αS,β'R)-Azf-L-Ala(3S-2-oxopyrrolidin-3-yl)-NH₂ (53)

Syrup. Yield: 77% (method E, from **38**). Eluents: MeOH:DCM (1:20). HPLC: t_{R}= 8.42 min (gradient from 15 to 95% of A, in 10 min). ¹H NMR (400 MHz, CDCl₃): δ 9.32 (br s, 1H, α-NH Ala), 7.49 (br s, 1H, α-CONH₂ Ala), 7.42-7.10 (m, 10H, Ar), 5.83 (br s, 1H, α-CONH₂ Ala), 5.81 (br s, 2H,1-H, α-NH Lys), 5.18 (d, *J* = 12.3 Hz, 1H, OCH₂), 5.11 (d, *J* = 12.3 Hz, 1H, OCH₂), 4.70 (br s, 1H, ε-NH Lys), 4.47 (m, 1H, α-H Ala), 4.09 (m, 1H, α-H Lys), 3.54 (d, *J* = 14.3 Hz, 1H, β-H Azf), 3.42 (dd, *J* = 8.2, 4.7 Hz, 1H, γ'-H Azf). 3.37 (m, 2H, 5-H), 3.27 (d, *J* = 14.3 Hz, 1H, β-H Azf), 3.27 (m, 1H, γ'-H Azf), 3.12 (m, 2H, ε-H Lys), 2.59 (m, 1H, β'-H Azf), 2.45-2.37 (m, 2H, 3-H, 4-H), 2.23 (m, 1H, β-H Ala), 2.12-1.82 (m, 4H, β-H Ala, β-H Lys, 4-H), 1.57-1.42 (m, 4H, δ-H Lys, γ-H Lys), 1.41 (s,9H, C(CH₃)₃), 1.11 (d, *J* = 7.2 Hz, 3H, β'-CH₃), ¹³C NMR (100 MHz, CDCl₃): δ 180.8, 175.4, 173.7, 170.1 (CO), 156.5, 156.4 (NHCOO, Boc, Z), 136.8, 135.8, 130.9, 128.6, 128.4, 128.2, 128.0, 127.1 (Ar), 79.5 (C(CH₃)₃), 76.6 (Cα Azf), 66.9 (OCH₂), 54.5 (Cα Ala), 53.8 (Cγ' Azf), 51.0 (Cα Lys), 41.3 (C5), 40.1 (Cε Lys), 39.7 (C3), 37.4 (Cβ Azf), 32.6 (Cβ Ala), 30.2 (Cβ' Azf), 29.9 (Cβ Lys), 29.7 (Cδ Lys), 28.5 (C(CH₃)₃), 28.5 (C4), 22.5 (Cγ Lys), 15.1 (β'-CH₃). MS (ES)⁺: 721.3 [M+H]⁺.

### Z-L-Tbg-(αS,β'R)-Azk(Boc)-L-Ala(3S-2-oxopyrrolidin-3-yl)-NH₂ (54)

Syrup. Yield: 63% (method E, from 39). Eluents: MeOH:DCM (1:15). HPLC: t_{R}= 6.36 min (gradient from 30 to 95% of A, in 10 min). ¹H NMR (400 MHz, CDCl₃): δ 8.00 (brs, 1H, α-NH Ala), 7.35 (br s, 1H, CONH₂), 7.34 (m, 5H, Ph), 5.76 (br s, 1H, CONH₂), 5.54 (brs, 1H, ε-NH Azk), 5.50 (br s, 1H, 1-H), 5.15 (d, *J* = 12.5 Hz, 1H, OCH₂), 5.12 (d, *J* = 12.3 Hz, 1H, OCH₂), 4.77 (brs, 1H, α-NH Tbg), 4.59 (q, *J* = 7.6 Hz, 1H, α-H Ala), 4.40 (t, *J* = 8.6 Hz, 1H, γ'-H Azk), 4.02 (d, *J* = 9.3 Hz, 1H, α-H Tbg), 3.71 (m, 1H, γ'-H Azk), 3.36 (m, 2H, 5-H), 3.06 (m, 2H, ε-Azk), 2.58 (m, 1H, β'-H Azk), 2.46-2.34 (m, 2H, 3-H, 4-H), 2.13 (m, 1H, β-H Ala), 2.12-1.96 (m, 3H, β-H Ala, δ-H Azk), 1.89 (m, 1H, 4-H), 1.46 (m, 2H, β-H Azk), 1.42 (s, 9H, C(CH₃)₃), 1.17 (m, 2H, γ-H Azk), 1.14 (d, *J* = 7.0 Hz, 3H, β'-CH₃), 1.06 (s, 9H, γ-H Tbg), ¹³C NMR (100 MHz, CDCl₃): δ 179.9, 174.0, 173.0, 170.9 (CO), 156.7 (NHCO)O, 136.4, 128.7, 128.4, 128.2 (Ar), 79.4 (C(CH₃)₃), 76.4 (Cα Azk), 67.3 (OCH₂), 58.4 (Cα Tbg), 55.0 (Cγ' Azk), 52.6 (Cα Ala), 40.7 (C5), 39.9 (Cε Azk), 38.7 (C3), 34.6 (Cβ Tbg), 33.2 (Cβ Ala), 33.0 (Cδ Azk), 31.1 ( Cβ' Azk), 29.7 (Cβ Azk), 28.8 (C4), 28.6 (C(CH₃)₃), 26.6 (Cγ Tbg), 19.9 (Cγ Azk), 15.9 (β'-CH₃). MS (ES)⁺: 687.4 [M+H]⁺.

### Z-L-Val-(αS,β'R)-Azf-L-Ala(3S-2-oxopyrrolidin-3-yl)-OMe (55)

Syrup. Yield: 44% (method E, from **30**)**.** Eluents: MeOH:DCM (1:9). HPLC (t_{R}, min): 7.83 (gradient from 10 to 95% of A, in 10 min). ¹H NMR (400 MHz, CDCl₃): δ 8.33 (d, *J* = 7.7 Hz, 1H, α-NH Ala), 7.43-7.29 (m, 5H, Ph), 7.20-7.09 (m, 5H, Ph), 5.90 (s, 1H, 1-NH), 5.54 (d, *J* = 9.4 Hz, 1H, α-NH Val), 5.19 (d, *J* = 12.3 Hz, 2H, OCH₂), 5.12 (d, *J* = 12.4 Hz, 2H, OCH₂), 4.67 (ddd, *J* = 10.9, 7.7, 4.0 Hz, 1H, α-H Ala), 3.88 (dd, *J* = 9.4, 6.6 Hz, 1H, α-H Val), 3.77 (OCH₃), 3.66 (d, *J* = 13.9 Hz, 1H, β-H Azf), 3.40-3.29 (m, 2H, γ'-H Azf), 3.20-3.13 (m, 2H, 5-H), 3.02 (d, *J* = 13.9 Hz, 1H, β-H Azf), 2.67-2.57 (m, 1H, β'-H Azf), 2.53-2.28 (m, 2H, 3-H, 4-H), 2.22 (ddd, *J* = 14.1, 10.5, 3.8 Hz, 1H, β-H Ala), 2.01-1.85 (m, 3H, β-H Val, β-H Ala, 4-H), 1.22 (d, *J* = 7.1 Hz, 3H, β'-CH₃), 0.97 (d, *J* = 6.8 Hz, 3H, γ-H Val), 0.95 (d, *J* = 6.7 Hz, 3H, γ-H Val). ¹³C (100 MHz, CDCl₃): δ 179.2, 173.9, 172.12, 172.08 (CO), 156.1 (NHCOO), 136.6, 135.0, 130.8, 128.7, 128.6, 128.3, 128.2, 127.4 (Ar), 77.9 (Cα Azf), 67.1 (OCH₂), 56.0 (Cα Val), 53.8 (Cα Ala), 52.7 (Cγ' Azf), 51.1 (OCH₃), 40.5 (C5), 39.1 (C3), 38.4 (Cβ Azf), 34.5 (Cβ Ala), 31.6 (Cβ Val), 30.8 (Cβ' Azf), 29.8 (C4), 19.3, 17.7 (Cγ Val), 17.0 (β'-CH₃). MS (ES)⁺: 607.4 [M+H]⁺.

### Boc-L-Tbg-(αS,β'R)-Azf-L-Ala(3S-2-oxopyrrolidin-3-yl)-OMe (56)

Syrup. Yield: 86% (method E, from **36**)**.** Eluents: MeOH:DCM (1:40). HPLC: t_{R}= 9.13 min (gradient from 10 to 95% of A, in 10 min). ¹H NMR (400 MHz, CDCl₃): δ 8.39 (d, *J* = 7.8 Hz, 1H, α-NH Ala), 7.29-7.17 (m, 5H, Ph), 5.79 (br s, 1H, 1-H), 5.42 (d, *J* = 9.9 Hz, 1H, α-NH Tbg), 4.66 (m, 1H, α-H Ala), 3.81 (d, *J* = 9.9 Hz, 1H, α-H Tbg), 3.77 (s, 3H, OCH₃), 3.71 (d, *J* = 14.0 Hz, 1H, β-H Azf), 3.32 (m, 2H, 5-H), 3.19 (m, 2H, γ'-H Azf), 3.02 (d, *J* = 14.0 Hz, 1H, β-H Azf), 2.58-2.33 (m, 3H, 3-H, β'-H Azf, 4-H), 2.23 (m, 1H, β-H Ala), 1.93-1.82 (m, 2H, β-H Ala, 4-H), 1.44 (s, 9H, C(CH₃)₃ Boc), 1.20 (d, *J* = 7.1 Hz, 3H, β'-CH₃). 1.00 (s, 9H, γ-H Tbg). ¹³C NMR (100 MHz, CDCl₃): δ 179.0, 173.6, 172.3, 172.1 (CO), 155.3 (NHCOO), 135.2, 130.8, 128.6, 127.3 (Ar), 79.7 (C(CH₃)₃ Boc), 77.7 (Cα Azf), 56.9 (Cα Tbg), 54.4 (Cγ' Azf), 52.7 (OCH₃), 51.1 (Cα Ala), 40.2 (C5), 39.0 (C3), 38.2 (Cβ Azf), 35.9 (Cβ Tbg), 34.6 (Cβ Ala), 30.5 (CP' Azf ), 28.6 (C(CH₃)₃), 28.2 (C4), 26.4 (Cγ Tbg), 17.1 (β'-CH₃). MS (ES)⁺: 587.4 [M+H]⁺.

### Z-L-Tbg-(αS,β'R)-Azf-L-His(Trt)-OMe (57)

Syrup. Yield: 65% (method E, from **35**)**.** Eluents: MeOH:DCM (1:60). HPLC: t_{R}= 7.68 min (gradient from 40 to 95% of A, in 10 min). ¹H NMR (400 MHz, CDCl₃): δ 8.03 (d, *J* = 7.5 Hz, 1H, α-NH His), 7.44-7.02 (m, 26H, Ar, His), 6.62 (br s, 1H, 2-H His), 5.59 (d, *J* = 9.7 Hz, 1H, α-NH Tbg), 5.19 (d, *J* = 12.3 Hz, 1H, OCH₂), 5.13 (d, *J* = 12.4 Hz, OCH₂), 4.88 (m, 1H, α-H His), 3.78 (d, *J* = 8.2 Hz, 1H, α-H Tbg), 3.66 (d, *J* = 14.0 Hz, 1H, β-H Azf), 3.60 (s, 3H, OMe), 3.16 (m, 4H, γ'-H Azf, β-H His), 3.05 (d, *J* = 13.9 Hz, 1H, β-H Azf), 2.54 (m, 2H, β'-H Azf), 1.19 (d, *J* = 7.1 Hz, 3H, β'-CH₃), 0.91 (s, 9H, γ-H Tbg). ¹³C NMR (100 MHz, CDCl₃): δ 172.7, 171.5, 170.7 (CO), 155.9 (NHCOO), 142.2, 138.3, 136.5, 135.5, 130.8, 129.8, 128.8, 128.4, 128.3, 128.1, 127.2, 119.7 (Ar), 77.0 (Cα Azf), 67.1 (OCH₂), 57.4 (Cα Tbg), 54.3 (Cγ' Azf), 53.1 (Cα His), 52.3 (OMe), 38.3 (Cβ Azf), 35.8 (Cβ Tbg), 30.45, (Cβ His), 30,4 (Cβ' Azf), 26.4 (Cγ Tbg), 16.3 (β'-CH₃). MS (ES)⁺: 847.4 [M+H]⁺.

### Z-L-Tbg-(αS,β'R)-Azf-L-His(Trt)-NH₂ (58)

Syrup. Yield: 86% (method F, from **57**). The crude was lyophylized in ACN/H₂O, 1:2. HPLC: t_{R}= 6.17 min (gradient from 40 to 95% of A, in 10 min). ¹H NMR (400 MHz, CDCl₃): δ 8.10 (d, *J* = 8.3 Hz, 1H, α-NH His), 7.42-7.07 (m, 26H, Ar, 5-H His), 6.70 (s, 1H, 2-H His), 5.63 (brs, 1H, CONH₂), 5.60 (brs, 1H, CONH₂), 5.34 (br s, 1H, α-NH Tbg), 5.18 (d, *J* = 12.3 Hz, 1H, OCH₂), 5.14 (d, *J* = 12.3 Hz, 1H, OCH₂), 4.96 (m , 1H, α-H His), 3.84 (d, *J* = 9.8 Hz, 1H, α-H Tbg), 3.61 (d, *J* = 14.2 Hz, 1H, β-H Azf), 3.90-3.16 (m, 4H, β-H Azf, β-H His, γ'-H Azf), 2.95 (dd, *J* = 14.8, 5.2 Hz, 1H, β-H His), 2.57 (m,1H, β'-H Azf), 1.17 (d, *J* = 7.1 Hz, 3H, β'-CH₃), 0.91 (s, 9H, γ-H Tbg). ¹³C NMR (100 MHz, CDCl₃): δ 173.6, 173.4, 169.6 (CO), 155.9 (NHCOO), 142.3, 138.6, 136.9, 136.4, 135.7, 130.8, 129.8, 128.8, 128.7, 128.6, 128.5, 128.4, 128.3, 127.2, 119.9 (Ar), 75.6 (Cα Azf), 67.2 (OCH₂), 57.9 (Cα Tbg), 54.7 (Cγ' Azf), 53.2 (Cα His), 37.6 (Cβ Azf), 35.9 (Cβ Tbg), 30.2 (Cβ' Azf), 29.9 (Cβ His), 26.5 (Cγ Tbg), 15.7 (β'-CH₃). MS (ES)⁺: 831.3 [M+H]⁺.

### Boc-L-Tbg-(αS,β'R)-Azl-L-Nle-OMe (59)

Syrup. Yield: 73% (Method E, from **34**)**.** Eluents: MeOH:DCM (1:20). HPLC: t_{R}= 4.39 min (gradient from 70 to 95% of A, in 10 min). ¹H NMR (400 MHz, CDCl₃): δ 8.69 (d, J = 7.7 Hz, 1H, α-NH Nle), 5.14 (d, J = 10.0 Hz, 1H, α-NH Tbg), 4.46 (m, 2H, α-Nle, γ'-H Azl), 3.88 (d, J = 9.9 Hz, α-H Tbg), 3.69 (s, 3H, OMe), 3.45 (dd, J = 8.4, 4.4 Hz, γ'-H Azl), 2.58 (m 1H, β'-H Azl), 2.23 (m, 1H, β-H Azl), 1.90-1.70 (m, 3H, β-H Azl, γ-H Azl, β-H Nle), 1.66 (m, 1H, β-H Nle), 1.29 (s, 9H, C(CH₃)₃), 1.29 (m (4-H, γ,δ-H Nle), 1.27 (d, J = 7.0 Hz, 3H, β'-CH₃), 1.00 (s, 9H, γ-H Tbg), 0.91 (d, J = 6.3 Hz, 3H, δ-H Azl), 0.90 (d, J = 6.3 Hz, 3H, δ-H Azl), 0.85 (m, 3H, ε-H Nle). ¹³C NMR (100 MHz, CDCl₃): δ 173.5, 172.6, 172.0 (CO), 156.0 (NHCOO), 79.9 (C, Boc), 78.2 (Cα Azf), 56.9 (Cα Tbg), 54.7 (Cγ' Azl), 52.4 (Cα Nle), 52.2 (OMe), 44.2 (Cβ Azl), 34,6 (Cβ Tbg), 33.8 (Cβ' Azl), 32.0 (Cβ Nle), 28.4 (C(CH₃)₃), 27.7 (Cγ Nle), 26.4 (Cγ Tbg), 24.9 (Cδ Azl), 24.6 (Cγ Azl), 23.2 (Cδ Azl), 22.5 (Cδ Nle), 17.6 (β'-CH₃), 13.9 (Cε Nle). MS (ES)⁺: 512.0 [M+H]⁺.

### Boc-L-Tbg-(αS,β'R)-Azl-L-Nle-NH₂ (60)

Syrup. Yield: 88% (method F, from **59**)**.** The crude was lyophylized in ACN/H₂O, 1:2. HPLC: t_{R}= 8.49 min (gradient from 30 to 95% of A, in 10 min). ¹H NMR (400 MHz, CDCl₃): δ 7.86 (d, J = 8.7 Hz, 1H, α-NH Nle), 6.65 (br s, 1H, CONH₂), 5.46 (br s, 1H, CONH₂), 5.12 (d, J = 9.3 Hz, 1H, α-NH Tbg), 4.52 (t, J = 8.4 Hz, 1H, Hy' Azl), 4.45 (m 1H, α-Nle), 3.90 (d, J = 9.2 Hz, α-H Tbg), 3.52 (dd, J = 8.6, 4.5 Hz, γ'-H Azl), 2.54 (m 1H, β'-H Azl), 2.16 (dd, J = 14.5, 4.6 Hz, 1H, β-H Azl), 1.96 (dd, J = 14.4, 7.5 Hz, 1H, β-H Azl) 1.92 (m (1H, (β-H Nle), 1.82 (m, 1H, γ-H Azl), 1.62 (m, 1H, β-H Nle), 1.43 (s, 9H, CH₃ Boc), 1.30 (m (4-H, γ,δ-H Nle), 1.20 (d, J = 7.1 Hz, 3H, β'-CH₃), 1.03 (s, 9H, γ-H Tbg), 0.95 (d, J = 6.6 Hz, 3H, δ-H Azl), 0.92 (d, J = 6.5 Hz, 3H, δ-H Azl), 0.89 (m, 3H, ε-H Nle). ¹³C NMR (100 MHz, CDCl₃): δ 174.4, 173.6, 171.3 (CO), 156.0 (NHCOO), 80.2 (C, Boc), 78.4 (Cα Azf), 57.9 (Cα Tbg), 55.1 (Cγ' Azl), 53.1 (Cα Nle), 43.6 (Cβ Azl), 34,5 (Cβ Tbg), 33.0 (Cβ' Azl), 31.5 (Cβ Nle), 28.4 (C(CH₃)₃), 28.1 (Cγ Nle), 26.5 (Cγ Tbg), 25.1 (Cδ Azl), 24.6 (Cγ Azl), 23.6 (Cδ Azl), 22.5 (Cδ Nle), 17.1 (β'-CH₃), 14.0 (Cε Nle). MS (ES)⁺: 497.0 [M+H]⁺.

### Boc-L-Val-(αS,β'R)-Aza(Cy)-Ala(3S-2-oxopyrrolidin-3-yl)-NH₂ (61)

Syrup. Yield: 51% (method E, from **(40)**). Eluents: MeOH:DCM (1:10). HPLC: t_{R}= 6.52 min (gradient from 30 to 95% of A, in 10 min). ¹H NMR (400 MHz, CDCl₃): δ 8.33 (d, *J* = 7.2 Hz, 1H, α-NH Ala), 7.44 (br s, 1H, α-CONH₂), 6.08 (br s, 1H, α-CONH₂), 5.76 (br s, 1H, 1-H), 5.19 (d, *J* = 9.0 Hz, 1H, α-NH Val), 4.53 (m, 1H, α-H Ala), 4.42 (t, *J* = 8.7 Hz, 1H, γ'-H Aza), 4.02 (dd, *J* = 9.0, 6.6 Hz, 1H, α-H, Val), 3.74 (dd, *J* = 8.7, 5.1 Hz, 1H, γ'-H Aza), 3.37 (m, 2H, 5-H), 2.66 (m 1H, β'-H Aza), 2.47-2.37 (m, 2H, 3-H, 4-H), 2.18-2.05 (m, 2H, β-H Ala, β-H Val), 204 (m, 2H, β-H Aza), 1.98-1.85 (m, 2H, 4-H, β-H Ala), 1.73-1.62 (m, 5H, 2, 3, 4, 5, 6-H Cy), 1.59 (m, 1H, 1-H Cy), 1.42 (s, 9H, C(CH₃)), 1.31-1.07 (m, 5H, 2, 3, 4, 5, 6-H Cy), 1.14 (d, J= 7.1 Hz, 3H, β'-CH₃), 1.10-1.06 (m, 2H, 2, 6-H Cy), 0.99 (d, *J* = 6.7 Hz, 3H, γ-H Val), 0.93 (d, *J* = 6.8 Hz, 3H, γ-H Val). ¹³C NMR (100 MHz, CDCl₃): δ 180.2, 174.6, 173.1, 171.0 (CO), 156.0 (NHCOO), 80.0 (C(CH₃)), 77.0 (Cα Aza), 56.2 (Cα Val), 54.4 (Cγ' Aza), 53.1 (Cα Ala), 41.3 (Cβ Aza), 41.0 (C5), 39.2 (C3), 35.8, 34.5 (C2-, C6- Cy), 33.6 (C1 Cy), 32.9 (Cβ Ala), 32.2 (CP'Aza), 30.0 (Cβ Val), 28.9 (C4), 28.5 (C(CH₃)), 26.5, 26.4, 26.2 (C3-, C4-, C5- Cy), 19.7, 17.7 (Cγ Val), 15.9 (β'-CH₃). MS (ES)⁺: 564.3 [M+H]⁺.

### Boc-L-Tbg-(αS,β'R)-Aza(Cy)-Ala(3S-2-oxopyrrolidin-3-yl)-NH₂ (62)

Syrup. Yield: 70% (method E, from **(41)**). Eluents: MeOH:DCM (1:30). HPLC: t_{R}= 6.85 min (gradient from 30 to 95% of A, in 10 min). ¹H NMR (400 MHz, CDCl₃): δ 8.04 (d, *J* = 7.7 Hz, 1H, α-NH Ala), 7.39 (br s, 1H, α-CONH₂), 5.98 (br s, 1H, α-CONH₂), 5.57 (br s, 1H, 1-H), 5.11 (d, *J* = 9.3 Hz, 1H, α-NH Tbg), 4.63 (q, *J* = 7.8 Hz, 1H, α-H Ala), 4.44 (t, *J* = 8.7 Hz, 1H, γ'-H Aza), 3.93 (d, *J* = 9.2 Hz, 1H, α-H, Tbg), 3.65 (dd, *J* = 8.7, 5.0 Hz, 1H, γ'-H Aza), 3.36 (m, 2H, 5-H), 2.64 (m 1H, β'-H Aza), 2.48-2.35 (m, 2H, 3-H, 4-H), 2.10-1.84 (m, 5H, β-H Ala, β-H Aza, 4-H), 1.72-1.52 (m, 5H, 2, 3, 4, 5, 6-H Cy), 1.48 (m, 1H, 1-H Cy), 1.44 (s, 9H, (C(CH₃)), 1.26-1.19 (m, 3H, 3, 4, 5-H Cy), 1.17 (d, *J* = 7.1 Hz, 3H, β'-CH₃), 1.03 (s, 9H, γ-H Tbg), 1.02-0.91 (m, 2H, 2, 6-H Cy). ¹³C NMR (100 MHz, CDCl₃): δ 179.8, 174.0, 172.9, 171.0 (CO), 155.9 (NHCOO), 80.2 (C(CH₃)), 77.0 (Cα Aza), 57.8 (Cα Tbg), 55.0 (Cy' Aza), 52.1 (Cα Ala), 41.6 (Cβ Aza), 40.7 (C5), 38.5 (C3), 35.6 (Cβ Tbg), 34.8, 34.5 (C2-, C6- Cy), 33.7 (C1 Cy), 33.6 (Cβ Ala), 32.4 (Cβ'Aza), 28.5 (C4), 28.4 (C(CH₃)), 26.55 (Cy Tbg), 26.5, 26.4, 26.2 (C3-, C4-, C5- Cy), 16.3 (β'-CH₃). MS (ES)⁺: 578.4 [M+H]⁺.

### Boc-D-β-Val-(αS,β'R)-Azf-L-Ala(3S-2-oxopyrrolidin-3-yl)-NH₂ (63)

Syrup. Yield: 54% (method E, from **42**) Eluents: MeOH:DCM (1:15). HPLC: t_{R}= 7.82 min (gradient from 15 to 95% of A, in 10 min). ¹H NMR (400 MHz, CDCl₃): δ 8.93 (br s, 1H, α-NH Ala), 7.65 (br s, 1H, α-CONH₂ Ala), 7.32-7.18 (m, 5H, Ph), 6.03 (br s, 1H, α-CONH₂ Ala), 5.38 (br s, 2H, 1-H, α-NH β-Val), 4.44 (m, 1H, α-H Ala), 3.90 (m, 1H, α-H β-Val), 3.56 (d, *J* = 14.2 Hz, 1H, β-H Azf), 3.41-3.36 (m, 3H, γ'-H Azf, 5-H), 3.04 (t, *J* = 8.2 Hz, 1H, γ'-H Azf), 2.56 (m, 1H, β'-H Azf), 2.49 (m, 2H, β-H Azf, β-H Ala), 2.19 (m, 2H, β-H, β-Val), 2.07-1.90 (m, 2H, γ-H, 3-H, β-H Ala), 1.43 (s, 9H, C(CH₃)₃), 1.11 (d, *J* = 7.1 Hz, 3H, β'-CH₃), 0.91 (d, *J* = 7.1 Hz, 3H, δ-H β-Val), 0.90 (d, *J* = 7.1 Hz, 3H, δ-H β-Val). ¹³C NMR (100 MHz, CDCl₃): δ 181.1, 174.8, 172.3, 170.2 (CO), 156.2 (NHCOO), 136.5, 131.0, 128.4, 127.0 (Ar), 79.4 (C(CH₃)₃), 76.0 (Cα Azf), 54.3 (Cα Ala), 53.4 (Cy' Azf), 52.4 (Cα β-Val), 41.1 (C5), 39.6 (C3), 37.6 (Cβ Azf), 33.2 (Cβ Ala), 32.2 (Cα β-Val), 32.0 (Cβ β-Val), 29.5 (Cβ' Azf ), 29.4 (C4), 28.6 (C(CH₃)₃), 18.8, 18.5 (CH₃ β-Val), 15.3 (β'-CH₃). MS (ES)⁺: 572.3 [M+H]⁺.

### General Procedure for the Synthesis of modified N- and C-terminal Substituted Tripeptide Derivatives.

- *First step from Z-derivatives:* A solution of the corresponding Z-derivative (1.0 eq) in MeOH (10 mL/mmol) was successively added 12 M HCl (1.0 eq) and Pd/C (20% w/w). The suspension was hydrogenated at rt and atmospheric pressure for 4-6 h. After filtration of the catalyst, the solvent was evaporated and the product dissolved in H₂O/acetonitrile and lyophilized to be used in the methods below.
- *First step from Boc derivatives:* Boc-protected tripeptide derivative **50** (202 mg, 0.34 mmol,) was treated with a 4M Dioxane/HCl solution (0.39 mL) and stirred at rt for 3 h. Then the solution was evaporated to dryness by coevaporating several times with CH₂Cl₂ and lyophilized in H₂O/acetonitrile. Yield 161mg (90 %).

***Method** G.* The resulting N-terminal amino hydrochloride (1.0 eq) was redissolved in dry THF (5 mL/mmol), and successively treated with PyBrop (1.2 eq), trifuoracetic acid (1.2 eq) and TEA (2.2 eq). After being stirred for 24 h at rt, the solvent was evaporated under vacuum, the residue was extracted with CH₂Cl₂, and the extract was washed successively with a solution of citric acid (10%), NaHCO₃ (10%), and brine. The organic phase was dried over Na₂SO₄ and evaporated to dryness. The residue was purified by chromatography on silica gel as specified in each case.

***Method H.*** To a 0º C solution of N-terminal amino hydrochloride (1eq) in MeOH (5 mL/mmol) was added TEA (3.9 eq), followed by ethyl trifluoroacetate (1.6 eq) and stirred for 16h. Then, the solvent was evaporated under vacuum, the residue was extracted with CH₂Cl₂, and the extract was washed with H₂O and brine. The organic phase was dried over Na₂SO₄ and evaporated to dryness. The residue was purified by chromatography on silica gel as specified in each case.

***Method I.*** The resulting hydrochloride derivative (1.0 eq) was redissolved in dry CH₂Cl₂ (5 mL/mmol) was successively treated with TEA (1.0 eq), DMAP (0.1 eq), and di-tert-butyldicarbonate (1.1 eq), and stirring overnight. Then the solvent was evaporated to dryness, and the residue was purified on a silica gel as specified.

***Method J.*** The resulting amino hydrochloride derivative (1.0 eq) was dissolved in anhydrous THF (5 mL/mmol) and TEA (1.0 eq) was added and the mixture was stirred at rt for 5 min. Then, propylene oxide (15 eq) and cyclopropanecarbonyl chloride, or methylchloroformate (1.5 eq), or 2,2-difluroethylchloroformate (1.2 eq) were added at 0 ºC dropwise, and the reaction was stirred at this temperature for 2h. After that time, saturated NaHCOs solution was added and extracted with DCM (x2). The organic phase was washed with brine, dried over Na₂SO₄ and filtered. The solvent was evaporated under reduced pressure and the residue was purified on a silica gel column, as specified.

***Method K.*** To a solution (4-methylphenoxy)acetic acid in THF (5 mL/mmol), HATU (1.2 eq) was added at 0 ºC and the mixture was stirred for 10 min at this temperature. Then, the hydrochloride derivative (1.0 eq) and DIPEA (2.4 eq) were added. The reaction mixture was stirred at rt overnight. The solvent was evaporated under reduced pressure. The residue was redissolved in EtOAc, washed with 10% citric acid solution, a 10% NaHCOs solution and brine. The organic layer was dried over Na₂SO₄, filtered and evaporated, and the residue was purified on a silica gel column, as specified.

***Method L.*** Compound **60** (210 mg, 0.42 mmol) was treated with 4M HCl/dioxane (0.48 mL) and stirred at rt for 3h. The acid was evaporated and the crude sample lyophilized. The obtained compound was dissolved in THF (1 mL) and tretated with TEA (0,063 mL, 0,45 mmol) and *tert*-butylisocyanate (0,059 mL, 0.050 mmol), and the mixture stirred for 1h. The solvent was evaporated, and the residue was dissolven in DCM and successively washed with H₂O and brine. The organic layer was dried over Na₂SO₄, filtered and evaporated. The resulting residue was purified on a silica gel column, using the indicated eluent mixture.

### Trifluoroacetyl-L-Val-(αS,βR)-Azl-L-Ala(3S-2-oxopyrrolidin-3-yl)-NH₂ (64)

Syrup. Yield: 36% (method G, from **43**). Eluents: MeOH:DCM (1:25). HPLC: t_{R}= 5.95 min (gradient from 10% to 95% of A, in 10 min). ¹H NMR (400 MHz, CDCl₃): δ 8.38 (d, *J =* 6.7 Hz, 1H, α-NH Ala), 7.58 (d, *J =* 9.0 Hz, 1H, α-NH Val ), 7.40 (br s, 1H, α-CONH₂), 6.05 (br s, 1H,α-CONH₂), 5.86 (br s, 1H, 1-H), 4.54 (m, 1H, α-H Ala), 4.45 (t, *J =* 8.7 Hz, 1H, γ'-H Azl), 4.38 (dd, *J =* 9.0, 6.8 Hz, 1H, α-H Val), 3.83 (dd, *J =* 8.7, 5.3 Hz, 1H, γ'-H Azl), 3.44 (m, 2H, 5-H), 2.73 (m, 1H, β-H Val), 2.43 (m, 2H, 3-H, 4-H), 2.29 (m, 1H, β'-H Azl), 2.16-1.98 (m, 3H, β-H Ala, β-H Azl), 1.96-1.87 (m, 2H, 4-H, β-H Ala), 1.80 (m, 1H, γ-H Azl), 1.16 (d, *J =* 7.2 Hz, 3H, β'-CH₃), 1.03 (d, *J =* 6.8 Hz, 3H, γ-H Val), 0.98 (d, *J =* 6.8 Hz, 3H, γ-H Val), 0.95 (d, *J =* 6.8 Hz, 3H, δ-H Azl), 0.85 (d, *J =* 6.8 Hz, 3H, δ-H Azl). ¹³C NMR (100 MHz, MeOD): δ 181.6, 176.3, 172.8, 172.3 (CO), 159.0 (q, *J =* 37.1 Hz, COCF₃), 116.0 (q, *J =* 287 Hz, CF₃), 78.6 (Cα Azl), 56.9 (Cα Val), 55.5 (Cγ' Azl), 53.7 (Cα Ala), 44.0 (Cβ Azl), 41.5 (C5), 40.3 (C3), 35.1 (Cβ Ala), 33.7 (Cβ Val), 30.3 (Cβ' Azl), 29.3 (C4), 25.4 (Cδ Azl), 25.3 (Cγ Azl), 24.1 (Cδ Azl), 19.6, 18.8 (Cγ Val), 16.2 (β'-CH₃). MS (ES)⁺: 520.2 [M+H]⁺.

### Trifluoroacetyl-L-Val-(αR,βS)-Azl-L-Ala(3S-2-oxopyrrolidin-3-yl)-NH₂ (65)

Syrup. Yield: 35% (method H, from **44**). Eluents: MeOH:DCM (1:25). HPLC: t_{R}= 5.96 min (gradient from 10 to 95% of A, in 10 min). ¹H NMR (400 MHz, CDCl₃): δ 8.22 (d, *J =* 6.4 Hz, 1H, α-NH Ala), 8.07 (d, *J =* 7.0 Hz, 1H, α-NHVal), 6.96 (brs, 1H, α-CONH₂), 5.94 (brs, 1H, α-CONH₂), 5.88 (brs, 1H, 1-H), 4.41 (m, 1H,α-H Ala), 4.16 (m, 1H, α-H Val), 4.14 (t, *J =* 8.6 Hz, 1H, γ'-H Azl), 3.76 (dd, *J =* 8.7, 5.3 Hz, 1H, γ'H Azl), 3.26 (m, 2H, 5-H), 2.63 (m, 1H, β'-H Azl), 2.44 (m, 1H, 3-H), 2.28 (m, 1H, β-H Ala), 2.14-1.90 (m, 3H, β-H Ala, β-H Azl, β-H Val, 4-H), 1.86-1.72 (m, 2H, 4-H, γ-H Azl), 1.11 (d, *J =* 7.1 Hz, 3H, β'-CH₃), 0.95 (d, *J =* 6.8 Hz, 3H, γ-H Val), 0.91 (d, *J =* 6.6 Hz, 6H, γ-H Val), 0.90 (d, *J =* 6.6 Hz, 3H, δ-H Azl), 0.85 (d, *J =* 6.8 Hz, 3H, δ-H Azl). ¹³C NMR (100 MHz, CDCl₃): δ 180.2, 174.3, 170.7, 170.6 (CO), 158.9 (q, *J =* 37.2 Hz, COCF₃), 117.4 (q, *J =* 287 Hz, CF₃), 78.2 (Cα Azl), 55.5 (Cα Val), 54.1 (Cγ' Azl), 52.4 (Cα Ala), 44.5 (Cβ Azl), 40.6 (C5), 37.8 (C3), 34.2 (Cβ Val), 33.0 (Cβ Ala), 30.4 (Cβ' Azl), 28.3 (C4), 25.0 (Cγ Azl), 25.0, 24,7, 23.7 (Cδ Azl), 19.6, 17.9 (Cγ Val ), 15.7 (β'-CH₃). MS (ES)⁺: 520.3 [M+H]⁺.

### Trifluoroacetyl-L-Val-(αS,β'R)-Azf-L-Ala(3S-2-oxopyrrolidin-3-yl)-NH₂ (66)

Syrup. Yield: 36% (method H, from **45**). Eluents: MeOH:DCM (1:15). HPLC (t_{R}, min): 7.57 (gradient from 10 to 95% of A, in 10 min). ¹H NMR (400 MHz, CDCl₃): δ 8.69 (d, *J* = 6.2 Hz, 1H, α-NH Ala), 7.57 (d, *J* = 9.2 Hz, 1H, α-NH Val), 7.43 (br s, 1H, α-CONH₂), 7.28-7.04 (m, 5H, Ar), 6.02 (br s, 1H, α-CONH₂), 5.85 (br s, 1H, 1-H), 4.52 (m, 1H, α-H Ala), 4.32 (dd, *J* = 9.3, 5.3 Hz, 1H, α-H Val), 3.54 (d, *J* = 14.3 Hz, 1H, β-H Azf), 3.49 (m, 1H, γ'-H Azf), 3.38 (m, 2H, 5-H), 3.28 (m, 1H, γ'-H Azf), 3.23 (d, *J* = 14.3 Hz, 1H, β-H Azf), 2.65 (m, 1H, 3-H), 2.48-2.40 (m, 2H, β'-H Azf, 4-H), 2.33 (m, 1H, β-H Val), 2.22 (m 1H, β-H Ala), 1.94-1.86 (m, 2H, β-H Ala, 4-H), 1.14 (d, *J* = 7.2 Hz, 3H, β'-CH₃), 0.98 (d, *J =* 6.8 Hz, 3H, γ-H Val), 0.92 (d, *J =* 6.8 Hz, 3H, γ-H Val). ¹³C (100 MHz, CDCl₃): 180.3, 174.5, 171.5, 170.0 (CO), 157.8 (q, *J =* 37.0 Hz, COCF₃), 135.5, 130.7, 128.7, 127.5 (C Ar), 117.5 (q, *J =* 287 Hz, CF₃), 77.4 (Cα Azf), 54.4 (Cα Val), 54.3 (Cα Ala), 53.7 (Cγ' Azf), 40.9 (C5), 39.7 (C3), 39.7 (Cβ Azf), 32.7 (Cβ Ala), 30.3 (Cβ' Azf), 29.9 (Cβ Val), 29.6 (C4), 19.9, 17.0 (Cγ Val), 15.1 (β'-CH₃). MS (ES)⁺: 554.2 [M+H]⁺.

### Trifluoroacetyl-L-Tbg-(αS,β'R)-Azf-L-Ala(3S-2-oxopyrrolidin-3-yl)-NH₂ (67)

Syrup. Yield: 10% (method H, from **51**). Eluents: MeOH:DCM (1:15). HPLC: t_{R}= 7.32 min (gradient from 15 to 95% of A, in 10 min). ¹H NMR (400 MHz, MeOD): δ 7.26-7.17 (m, 5H, Ph), 4.54 (m, 1H, α-H Tbg ), 4.30 (s, *J =* 9.7 Hz, 1H, α-H Ala), 3.62 (d, *J* = 13.9 Hz, 1H, β-H Azf), 3.49 (dd, *J* = 8.3, 4.9 Hz, 1H, γ'-H Azf), 3.31 (m, 2H, 5-H), 3.22 (t, *J* = 8.4 Hz, 1H, γ'-H Azf), 3.12 (d, *J* = 13.9 Hz, 1H, β-H Azf), 2.64 (m, 1H, 3'-H), 2.52 (m, 1H, β'-H Azf), 2.45 (m, 1H, 4-H), 2.13 (m, 1H, β-H Ala). 1.90-1.79 (m, 2H, β-H Ala, 4-H), 1.17 (d, *J* = 7.1 Hz, 3H, β'-CH₃), 1.07 (s, 9H, γ-H Tbg). ¹³C NMR (100 MHz, MeOD): δ 181.6, 176.2, 172.4, 171.9 (CO), 159.3 (q, *J* = 37.0 Hz, COCF₃), 136.7, 131.8, 129.4, 128.2 (Ar), 116.2 (q, *J =* 285 Hz, CF₃), 78.1 (Cα Azf), 57.2 (Cα Tbg), 55.6 (Cγ' Azf), 53.5 (Cα Ala), 41.5 (C5), 40.2 (C3), 39.0 (Cβ Azf), 36.8 (Cβ Tbg), 35.5 (Cβ Ala), 31.2 (Cβ' Azf), 29.3 (C4), 26.8 (Cγ Tbg), 16.0 (β'-CH₃). MS (ES)⁺: 568.2 [M+H]⁺.

### Boc-L-Val-(αS,β'R)-Azf-L-Ala(3S-2-oxopyrrolidin-3-yl)-NH₂ (68)

Yield: 50% (method I, from **45**). Eluents: MeOH:DCM (1:70). HPLC: t_{R}= 8.02 min (gradient from 10 to 95% of A, in 10 min). ¹H NMR (400 MHz, CDCl₃): δ 8.62 (d, *J =* 6.6 Hz, 1H, α-NH Ala), 7.53 (br s, 1H, α-CONH₂), 7.31-7.14 (m, 5H, Ph), 5.64 (br s, 1H, α-CONH₂), 5.47 (br s, 1H,1-H), 5.19 (d, *J =* 9.4 Hz, 1H, α-NH Val), 4.52 (m, 1H, α-H Ala), 4.01 (dd, *J =* 9.3, 5.0 Hz, 1H, α-H, Val), 3.59 (d, *J* = 14.2 Hz, 1H, β-H Azf), 3.45 (dd, *J =* 8.4, 4.6 Hz, 1H, γ'-H Azf), 3.36 (m, 2H, 5-H), 3.24 (m, 2H, β-H Azf, γ'-H Azf), 2.60 (m, 1H, 3-H), 2.47-2.37 (m, 2H, 4-H, β'-H Azf), 2.24-2.15 (m, 2H, β-H Ala, β-H Val), 1.98-1.84 (m, 2H, β-H Ala, 4-H), 1.49 (s, 9H, C(CH₃)), 1.13 (d, *J =* 7.2 Hz, 3H, β'-CH₃), 0.97 (d, *J =* 6.8 Hz, 3H, γ-H Val), 0.88 (d, *J =* 6.6 Hz, 3H, γ-HVal). ¹³C NMR (100 MHz, CDCl₃): δ 180.1, 174.5, 173.6, 170.4 (CO), 155.8 (NHCOO), 135.9, 131.0, 128.6, 127.3 (Ar), 79.9 (C(CH₃)), 77.0 (Cα Azf), 55.7 (Cα Val), 54.2 (Cα Ala), 53.7 (Cγ' Azf), 40.7 (C5), 39.5 (C3), 37.6 (Cβ Azf), 32.7 (Cβ Ala), 30.2 (Cβ Val, Cβ' Azf), 29.5 (C4), 28.5 (C(CH₃)), 19.9, 17.1 (Cγ Val), 15.2 (β'-CH₃). MS (ES)⁺: 558.3 [M+H]⁺.

### Cyclopropylcarbonyl-L-Val-(αS,β'R)-Azf-L-Ala(3S-2-oxopyrrolidin-3-yl)-NH₂. (69)

Syrup. Yield: 99% (method J, from **45**). Eluents: MeOH:DCM: (1:9). HPLC (t_{R}, min): 6.64 (gradient from 10% to 95% of A, in 10 min). ¹H NMR (400 MHz, CDCl₃): δ 8.55 (d, *J* = 6.0 Hz, 1H, α-NH Ala), 8.01 (d, *J* = 8.5 Hz, 1H, α-NH Val), 7.53 (s, 2H, α-CONH₂ Ala), 7.23-7.21 (m, 3H, Ph), 7.08-7.06 (m, 2H, Ph), 6.42 (s, 1H, 1-H), 4.52-4.48 (m, 2H, α-H Ala, α-H Val), 3.53-3.50 (m, 3H, γ'-H Azf, β-H Azf), 3.38-3.36 (m, 2H, 5-H), 3.23 (d, *J* = 14.3 Hz, 1H, β-H Azf), 2.56-2.39 (m, 3H, β'-H Azf, 3-H, 4-H), 2.27-2.21 (m, 2H, β-H Ala, β-H Val), 1.95-1.86 (m, 2H, β-H Ala, 4-H), 1.84-1.78 (m, 1H, CH cPr), 1.10 (d, *J* = 6.7 Hz, 3H, β'-CH₃), 1.00-0.89 (m, 10H, γ-H Val, CH₂cPr). ¹³C (100 MHz, CDCl₃): 180.4, 175.6, 174.3, 174.1, 170.5 (CO), 135.6, 130.9, 128.7, 127.1 (Ar), 77.4 (Cα Azf), 54.6 (Cα Val), 54.1 (Cα Ala), 53.7 (Cγ' Azf), 41.0 (C5), 40.1 (C3), 37.8 (Cβ Azf), 32.5 (Cβ Ala), 30.0, 29.9 (Cβ Val, Cβ' Azf), 29.5 (C4), 19.8, 17.2 (Cγ Val), 15.10 (β'-CH₃), 14.2 (CHcPr), 7,8, 7.6 (CH₂cPr). MS (ES)⁺: 526.2 [M+H]⁺.

### p-Tolyloxyacetyl-L-Tbg-(αS,β'R)-Azf-L-Ala(3S-2-oxopyrrolidin-3-yl)-NH₂ (70)

Syrup. Yield: 69% (method K, from 51). Eluents: MeOH:DCM (1:9). HPLC (t_{R}, min): 8.32 (gradient from 10 to 95% of A, in 10 min). ¹H NMR (400 MHz, CDCl₃): δ 8.14 (d, *J* = 7.0 Hz, 1H, α-NH Ala), 7.53 (s, 1H, α-CONH₂), 7.21-7.13 (m, 8H, Ph, C₆H₄, α-NH Tbg), 6.90 (d, *J* = 8.7 Hz, 2H, C₆H₄), 5.84 (s, 1H, 1-H), 5.55 (s, 1H, α-CONH₂), 4.66 (dt, *J* = 9.5, 6.7 Hz, 1H, α-H Ala), 4.55 (d, *J* = 14.9 Hz, 2H, OCH₂), 4.49 (d, *J* = 15.0 Hz, 1H, OCH₂), 4.31 (d, *J* = 10.0 Hz, α-H Tbg), 3.63 (d, *J* = 14.2 Hz, 1H, β-H Azf), 3.48 (d, *J* = 8.4, 4.7 Hz, 1H, γ'-H Azf), 3.43-3.33 (m, 3H, 5-H, γ'-H Azf), 3.20 (d, *J* = 14.2 Hz, 1H, β-H Azf), 2.63 (m, 1H, β'-H Azf), 2.52-2.39 (m, 2H, 3-H, 4-H), 2.31 (s, 3H, CH₃Ph), 2.13-2.01 (m, 2H, β-H Ala), 1.93-1.88 (m, 1H, 4-H), 1.16 (d, *J* = 7.1 Hz. 3H, β'-CH₃), 1.02 (s, 9H, γ-H Tbg). ¹³C (100 MHz, CDCl₃): δ 179.9, 173.9, 172.0, 170.5, 167.8 (CO), 155.1, 135.6, 131.9, 131.0, 130.4, 128.4, 127.3, 114.7 (Ar), 77.4 (Cα Azf), 67.4 (OCH₂), 54.9 (Cα Tbg), 54.5 (Cγ' Azf), 53.0 (Cα Ala), 40.7 (C5), 38.8 (C3), 38.1 (Cβ Azf), 35.6 (Cβ Tbg), 35.5 (Cβ Ala), 29.9 (Cβ' Azf), 28.9 (C4), 26.5 (Cγ Tbg), 20.6 (CH₃Ph), 15.6 (β'-CH₃). MS (ES)⁺: 620.4 [M+H]⁺.

### Methoxycarbonyl-L-Tbg-(αS,β'R)-Azf-L-Ala(3S-2-oxopyrrolidin-3-yl)-NH₂ (71)

Syrup. Yield: 80% (method J, from **51**). Eluents: MeOH:DCM (1:9). HPLC (t_{R}, min): 6.77 (gradient from 10 to 95% of A, in 10 min). ¹H NRM (400 MHz, CDCl₃): δ 7.96 (d, *J* = 7.3 Hz, 1H, α-NH Ala), 7.50 (s, 1H, α-CONH₂ Ala), 7.23-7.21 (m, 3H, Ph), 7.16-7.14 (m, 2H, Ph), 5.79 (s, 1H, 1-H), 5.73 (s, 1H, α-CONH₂ Ala), 5.61 (d, *J* = 9.8 Hz, α-NH Tbg), 4.67 (m, 1H, α-H Ala), 3.90 (d, J= 9.8, 1H, α-H Tbg), 3.74 (s, 3H, OCH₃), 3.63 (d, *J* = 14.2 Hz, 1H, β-H Azf), 3.46-3.34 (m, 4H, γ'-H Azf, 5-H), 3.21 (d, *J* = 14.2 Hz, 1H, β-H Azf), 2.62 (m, 1H, β'-H Azf), 2.50-2.39 (m, 2H, 3-H, 4-H), 2.15-1.99 (m, 2H, β-H Ala), 1.99-1.87 (m, 1H, 4-H), 1.16 (d, *J=* 7.1 Hz, 3H, β'-CH₃), 1.03 (s, 9H, γ-H Tbg). ¹³C (100 MHz, CDCl₃): δ 179.8, 174.0, 173.1, 170.4 (CO), 156.1 (COO), 135.6, 131.0, 128.4, 127.2 (Ar), 77.4 (Cα Azf), 57.8 (Cα Tbg), 54.5 (Cγ' Azf), 52.9 (Cα Ala), 52.6 (OCH₃), 40.6 (C5), 38.8 (C3), 38.1 (Cβ Azf), 35.3 (Cβ Tbg), 33.4 (Cβ Ala), 29.9 (Cβ' Azf), 28.8 (C4), 26.4 (Cγ Tbg), 15.6 (β'-CH₃). MS (ES)⁺: 530.3 [M+H]⁺.

### Methyloxycarbonyl-L-Tbg-(αS,β'R)-Azf-LAla(3S-2-oxopyrrolidin-3-yl)-OMe (72)

Syrup. Yield: 66% (method J, from **56**). Eluents: MeOH:DCM (1:25). HPLC: t_{R}= 8.46 min (gradient from 10 to 95% of A, in 10 min). ¹H NMR (400 MHz, CDCl₃): δ 8.29 (d, *J =* 7.7 Hz, 1H, α-NH Ala), 7.27-7.15 (m, 5H, Ph), 5.78 (br s, 1H, 1-H), 5.55 (d, *J* = 9.9 Hz, 1H, α-NH Tbg), 4.66 (m, 1H, α-H Ala), 3.83 (d, *J =* 9.9 Hz, 1H, α-H Tbg), 3.77 (s, 3H, OCH₃), 3.75 (s, 3H, CO₂CH₃), 3.70 (d, *J =* 13.8 Hz, 1H, β-H Azf), 3.32 (m, 2H, 5-H), 3.20 (m, 2H, γ'-H Azf), 3.02 (d, *J* = 13.8 Hz, 1H, β-H Azf), 2.59 (m, 1H, 3-H), 2.47 (m, 1H, 4-H), 2.39 (m, 1H, β'-H Azf), 2.23 (m, 1H, β-H Ala), 1.94-1.83 (m, 2H, β-H Ala,4-H), 1.22 (d, *J =* 7.0 Hz, 3H, β'-CH₃), 1.01 (s, 9H, γ-H Tbg). ¹³C NMR (100 MHz, CDCl₃): δ 178.9, 173.5, 172.15, 172,14 (CO), 156.6 (NHCOO), 135.1, 130.9, 128.5, 127.4 (Ar), 77.6 (C(CH₃)), 77.4 (Cα Azf), 57.4 (Cα Tbg), 54.4 (Cγ' Azf), 52.7, 52.5 (OCH₃), 51.1 (Cα Ala), 40.2 (C5), 39.0 (Cβ Azf), 38.2 (C3), 35.6 (Cβ Tbg), 34.7(CβAzf), 30.4 (Cβ' Azf), 28.3 (C4), 26.3 (Cγ Tbg), 17.1 (β'-CH₃). MS (ES)⁺: 545.2 [M+H]⁺.

### 2,2-Difluoroethyloxycarbonyl-L-Tbg-(αS,β'R)-Azf-L-Ala(3S-2-oxopyrrolidin-3-yl)-OMe (73)

Syrup. Yield: 74% (method J, from **56**). Eluents: MeOH:DCM (1:20). HPLC: t_{R}= 7.82 min (gradient from 15 to 95% of A, in 10 min). ¹H NMR (400 MHz, CDCl₃): δ 8.26 (d, *J* = 7.6 Hz, 1H, α-NH Ala), 7.26-7.15 (m, 5H, Ph), 5.99 (tt, *J* = 55.3, 4.1 Hz, 1H, CHF₂), 5.82 (br s, 1H, 1-H), 5.73 (d, *J* = 9.8 Hz, 1H, α- NH Tbg), 4.66 (m, 1H, α-H Ala), 4.31 (m, 2H, OCH₂), 3.81 (d, *J* = 9.8 Hz, 1H, α-H Tbg), 3.69 (d, *J* = 13.9 Hz, 1H, β-H Azf), 3.34 (m, 2H, 5-H), 3.20 (d, *J* = 6.2 Hz, 2H, γ'-H Azf), 3.04 (d, *J* = 13.9 Hz, 1H, β-H Azf), 2.62 (m,1H, β'-H Azf), 2.53-2.37 (m, 3H, 4-H, 3-H), 2.22 (m, 1H, β-H Ala), 1.92-1.83 (m, 3H, 4-H, β-H Ala), 1.22 (d, *J* = 7.0 Hz, 3H, β'-CH₃), 1.02 (s, 9H, γ-H Tbg).¹³C NMR (100 MHz, CDCl₃): δ 179.0, 172.8, 172.1, 172,0 (CO), 154.9 (NHCOO), 135.1, 130.9, 128.5, 127.4 (Ar), 113.10 (t, *J* = 241.3 Hz, CHF₂), 77.7 (Cα Azf), 63.5 (t, *J* = 29.4 Hz, OCH₂), 57.7 (Cα Tbg), 54.4 (Cγ' Azf), 52.7 (OMe), 51.1 (Cα Ala), 40.3 (C5), 39.0 (Cβ Azf), 38.3 (C3), 35.9 (Cβ Tbg), 34.7 (Cβ Ala), 30.5 (Cβ' Azf), 28.2 (C4), 26.3 (Cγ Tbg), 17.0 (β'-CH₃). MS (ES)⁺: 595.3 [M+H]⁺.

### 2,2-Difluoroethyloxycarbonyl-L-Tbg-(αS,β'R)-Azf-L-Ala(3S-2-oxopyrrolidin-3-yl)-NH₂ (74)

Syrup. Yield: 74% (method J, from **51**). Eluents: MeOH:DCM (1:15). HPLC: t_{R}= 6.96 min (gradient from 15 to 95% of A, in 10 min). ¹H NMR (400 MHz, CDCl₃): δ 8.05 (d, *J* = 7.2 Hz, 1H, α-NH Ala), 7.61 (br s, 1H, α-CONH₂ Ala), 7.26-7.15 (m, 5H, Ph), 5.98 (tt, *J* = 54.9, 3.7 Hz, 1H, OCH₂), 5.91 (br s, 1H, α-CONH₂ Ala), 5.73 (br s, 1H, 1-H), 5.58 (d, J= 9.3 Hz, 1H, α- NH Tbg), 4.68 (m, 1H, α-H Ala), 4.33 (m, 2H, OCH₂), 3.87 (d, *J* = 9.3 Hz, 1H, α-H Tbg), 3.62 (d, *J* = 14.2 Hz, 1H, β-H Azf), 3.47-3.34 (m, 4H, 5-H, γ'-H Azf), 3.22 (d, *J* = 14.2 Hz, 1H, β-H Azf), 2.65 (m, 1H, 3-H), 2.54-2.39 (m, 2H, 4-H, β'-H Azf), 2.07 (m, 1H, β-H Ala), 1.89 (m, 1H, 4-H), 1.16 (d, *J* = 7.1 Hz, 3H, β'-CH₃), 1.03 (s, 9H, γ-H Tbg).¹³C NMR (100 MHz, CDCl₃): δ 179.9, 174.1, 172.3, 170.4 (CO), 155.2 (NHCOO), 135.6, 131.0, 128.5, 127.3 (Ar), 113.1 (t, *J* = 241.3 Hz, CHF₂), 78.1 (Cα Azf), 63.5 ((t, *J* = 28.7 Hz, OCH₂), 58.7 (Cα Tbg), 54.6 (Cγ' Azf), 52.7 (Cα Ala), 40.8 (C5), 38.7 (Cβ Azf), 38.3 (C3), 35.4 (Cβ Tbg), 33.4 (Cβ Ala), 30.0 (Cβ' Azf), 28.7 (C4), 26.5 (Cγ Tbg), 15.6 (β'-CH₃). MS (ES)⁺: 580.3 [M+H]⁺.

### ^{t}BuNHCO-L-Tbg-(αS,β'R)-Azl-L-Nle-NH₂ (75)

Syrup. Yield: 71% (method L, from **59**). Eluents: EtOAc:hexane (2:3). HPLC: t_{R}= 7.41 min (gradient from 30 to 95% of A, in 10 min). ¹H NMR (400 MHz, CDCl₃): δ 7.65 (br s, 1H, CONH₂), 7.33 (br s, 1H, CONH₂), 6.80 (d, J = 10.1 Hz, 1H, α-NH Nle), 6.32 (br s, 2H, NHCONH), 4.69 (t, J = 8.5 Hz, 1H, Hy' Azl), 4.45 (ddd, J = 10.0, 8.6, 3.9 Hz, 1H, α-Nle), 4.13 (s, 1H, α-H Tbg), 3.54 (dd, J = 8.4, 4.0 Hz, γ'-H Azl), 2.64 (m 1H, β'-H Azl), 2.16 (dd, J = 14.7, 4.9 Hz, 1H, β-H Azl), 2.01 (dd, J = 14.7, 7.0 Hz, 1H, β-H Azl), 1.93-1.80 (m, 2H, β-H Nle, γ-H Azl), 1.63 (ddd, J = 11.8, 8.5, 6.1 Hz, 1H, β-H Nle), 1.30 (m, 4H, γ,δ-H Nle),1.27 (s, 9H, CH₃ N*^{t}*Bu), 1.23 (d, J = 7.1 Hz, 3H, β'-CH₃), 1.00 (s, 9H, γ-H Tbg), 0.93 (d, J = 6.6 Hz, 3H, δ-H Azl), 0.90 (d, J = 6.6 Hz, 3H, δ-H Azl), 0.88 (t, J = 7.1 Hz, 3H, ε-H Nle). ¹³C NMR (100 MHz, CDCl₃): δ 176.7, 176.1, 169.9 (CO), 157.8 (NHCONH), 79.1 (Cα Azf), 56.8 (Cα Tbg), 55.1 (Cγ' Azl), 52.8 (Cα Nle), 50.1 (NHC(CH₃)₃) 43.7 (Cβ Azl), 34,2 (Cβ Tbg), 32.9 (Cβ' Azl), 31.8 (Cβ Nle), 29.5 (NHC(CH₃)₃), 27.8 (Cγ Nle), 26.8 (Cγ Tbg), 25.2 (Cδ Azl), 24.6 (Cγ Azl), 24.0 (Cδ Azl), 22.3 (Cδ Nle), 16.7 (β'-CH₃), 14.0 (Cε Nle). MS (ES)⁺: 496.0 [M+H]⁺.

### 2,2-Difluoroethyloxycarbonyl-L-Tbg-(αS,β'R)-Azl-L-Ala(3S-2-oxopyrrolidin-3-yl)-NH₂ (76)

Syrup. Yield: 88% (method H, from **(49)**). Eluents: MeOH:DCM (1:15). HPLC: t_{R}= 6.74 min (gradient from 15 to 95% of A, in 10 min). ¹H NMR (400 MHz, CDCl₃): δ 7.96 (d, *J* = 7.5 Hz, 1H, α-NH Ala), 7.40 (br s, 1H, α-CONH₂), 6.13 (br s, 1H, α-CONH₂), 5.88 (br s, 1H, 1-H), 5.86 (tt, *J* = 54.9, 3.8 Hz, 1H, CHF₂), 5.78 (d, *J* = 9.3 Hz, 1H, α- NH Tbg), 4.63 (q, *J* = 7.8 Hz, 1H, α-H Ala), 4.46 (t, *J* = 8.6 Hz, 1H, γ'-H Azl), 4.28 (m, 2H, OCH₂), 3.98 (d, *J* = 9.3 Hz, 1H, α-H Tbg), 3.70 (dd, *J* = 8.7, 5.2 Hz, 1H, γ'-H Azl), 3.36 (m, 2H, 5-H), 2.67 (m, 1H, β'-H Azl), 2.48-2.34 (m, 2H, 4-H, 3-H), 2.11 (dd, *J* = 14.8, 4.8 Hz, 1H, β-H Azl), 2.05-1.92 (m, 3H, β-H Azl, 2-H), 1.93-1.78 (m, 2H, 4-H, γ-H Azl), 1.17 (d, *J* = 7.1 Hz, 3H, β'-CH₃), 1.05 (s, 9H, γ-H Tbg), 0.97 (d, *J* = 6.6 Hz, 3H, δ-H Azl), 0.88 (d, *J* = 6.7 Hz, 3H, δ-H Azl). ¹³C NMR (100 MHz, CDCl₃): δ 179.9, 174.0, 172.1, 170.7 (CO), 155.7 (NHCOO), 113.0 (t, *J* = 241.3 Hz, CHF₂), 77.8 (Cα Aza), 63.3 (t, *J* = 28.6 Hz, OCH₂), 58.7 (Cα Tbg), 55.0 (Cγ' Aza), 52.3 (Cα Ala), 42.5 (Cβ Azl), 40.6 (C5), 38.5 (C3), 34.7 (Cβ Tbg), 33.5 (Cβ'Azl), 32.0 (Cβ Ala), 28.5 (C4), 26.5 (Cγ Tbg), 25.3 (Cγ Val), 24.3 (Cβ Val), 23.6 (Cγ Val), 16.1 (β'-CH₃). MS (ES)⁺: 546.2 [M+H]⁺.

### Trifluoroacetyl-L-Tbg-(αS,β'R)-Aza(Cy)-L-Ala(3S-2-oxopyrrolidin-3-yl)-NH₂ (77)

Syrup. Yield: 16% (method G, from **(62)**). Eluents: MeOH:DCM (1:8). HPLC: t_{R}= 7.32 min (gradient from 30 to 95% of A, in 10 min). ¹H NMR (400 MHz, MeOD): δ 4.51 (dd, *J* = 10.5, 4.6 Hz, 1H, α-H Ala), 4.40-4.35 (m, 2H, α-H Tbg, γ'-H Aza), 3.74 (dd, *J* = 8.6, 5.2 Hz, 1H, γ'-H Aza), 3.31 (m, 2H, 5-H), 2.68 (m, 1H, β'-H Aza), 2.51-2.35 (m, 2H, 3-H, 4-H), 2.10 (m, 1H, β-H Ala), 2.00 (m, 2H, β-H Aza), 1.90 (m, 1H, 4-H), 1.77 (m, 1H, β-H Ala), 1.72-1.55 (m, 5H, 2, 3, 4, 5, 6-H Cy), 1.53 (m, 1H, 1-H Cy), 1.34-1.19 (m 3H, m, 3H, 3, 4, 5-H Cy), 1.18 (d, *J* = 7.0 Hz, 3H, β'-CH₃), 1.09 (s, 9H, γ-H Tbg), 1.07-1.03 (m, 2H, 2,6-H Cy). ¹³C NMR (100 MHz, MeOD): δ 181.6, 176.1, 172.9, 171.7 (CO), 159.2 (q, *J =* 37.0 Hz, COCF₃), 116.2 (q, *J =* 285 Hz, CF₃), 78.7 (Cα Aza), 57.7 (Cα Tbg), 56.1 (Cγ' Aza), 53.0 (Cα Ala), 42.5 (Cβ Aza), 41.5 (C5), 40.1 (C3), 36.7 (Cβ Tbg), 36.0, 35.6 (C2-, C6- Cy), 35.5 (Cβ Ala), 34.9 (C1 Cy), 33.6 (CP'Aza), 29.1 (C4), 27.5, 27.3, 27.2 (C3-, C4-, C5- Cy), 26.8 (Cγ Tbg), 16.4 (β'-CH₃). MS (ES)⁺: 574.3 [M+H]⁺.

### 2,2-Difluoroethyloxycarbonyl-L-Tbg-(αS,β'R)-Aza(Cy)-L-Ala(3S-2-oxopyrrolidin-3-yl)-NH₂ (78)

Syrup. Yield: 69% (method H, from 62). Eluents: MeOH:DCM (1:15). HPLC: t_{R}= 5.77 min (gradient from 30 to 95% of A, in 10 min). ¹H NMR (400 MHz, CDCl₃): δ 7.88 (d, *J* = 7.7 Hz, 1H, α-NH Ala), 7.37 (br s, 1H, α-CONH₂), 6.01 (br s, 1H, α-CONH₂), 5.92 (tt, *J* = 54.9, 3.8 Hz, 1H, CHF₂), 5.57 (br s, 1H, 1-H), 5.73 (d, *J* = 9.1 Hz, 1H, α- NH Tbg), 4.63 (m, 1H, α-H Ala), 4.44 (t, *J* = 8.6 Hz, 1H, γ'-H Aza), 4.31 (td, *J* = 14.0, 3.9 Hz, 2H, OCH₂), 3.98 (d, *J* = 9.1 Hz, 1H, α-H Tbg), 3.71 (dd, *J* = 8.7, 5.3 Hz, 1H, γ'-H Aza), 3.35 (m, 2H, 5-H), 2.66 (m, 1H, β'-H Aza), 2.46-2.34(m, 2H, 4-H, 3-H), 2.07-2.00 (m, 4H, β-H Ala, β-H Aza), 1.93-1.76 (m, 1H, 4-H), 1.74-1.53 (m, 5H, 2, 3, 4, 5, 6-H Cy), 1.45 (m, 1H, 1-H Cy), 1.31-1.19 (m 3H, m, 3H, 3, 4, 5-H Cy), 1.16 (d, *J* = 7.2 Hz, 3H, β'-CH₃), 1.06 (s, 9H, γ-H Tbg), 1.02-0.80 (m, 2H, 2,6-H Cy). ¹³C NMR (100 MHz, CDCl₃): δ 179.9, 174.1, 172.0, 170.8 (CO), 155.6 (NHCOO), 113.1 (t, *J* = 241.2 Hz, CHF₂), 77.8 (Cα Aza), 63.4 (t, *J* = 28.6 Hz, OCH₂), 58.9 (Cα Tbg), 55.2 (Cγ' Aza), 52.4 (Cα Ala), 41.1 (Cβ Aza), 40.6 (C5), 38.7 (C3), 35.7 (Cβ Tbg), 34.8, 34.6 (C2-, C6- Cy), 33.6 (C1 Cy), 33.4 (Cβ Ala), 32.0 (CP'Aza), 28.6 (C4), 26.6 (Cγ Tbg), 26.55, 26.5, 26.2 (C3-, C4-, C5- Cy), 16.0 (β'-CH₃).MS (ES)⁺: 586.3 [M+H]⁺.

### 2,2-Difluoroethyloxycarbonyl-L-Val-(αS,β'R)-Azf-Ala(3S-2-oxopyrrolidin-3-yl)-NH₂ (79)

Syrup. Yield: 75% (method J, from **45**). Eluents: MeOH:DCM (1:10). HPLC: t_{R}= 6.43 min (gradient from 15 to 95% of A, in 10 min). ¹H NMR (400 MHz, CDCl₃): δ 8.60 (d, *J* = 6.2 Hz, 1H, α-NH Ala), 7.60 (br s, 1H, α-CONH₂ Ala), 7.26-7.13 (m, 5H, Ph), 6.99 (br s, 1H, α-CONH₂ Ala), 6.89 (d, *J* = 9.6 Hz, 1H, α-NH Val), 6.01 (tt, 1H, *J* = 55.1, 3.8 Hz, CHF₂), 6.00 (br s, 1H, 1-H), 4.44 (m, 1H, α-H Ala), 4.34 (m, 2H, OCH₂), 4.01 (dd, *J* = 9.6, 6.8 Hz, 1H, α-H Val), 3.56 (m, 1H, γ'-H Azf), 3.45-3.31 (m, 4H, β-H Azf, 5-H, γ'-H Azf), 3.19 (d, *J* = 14.3 Hz, 1H, β-H Azf), 2.56 (m, 1H, 3-H), 2.44-2.36 (m, 2H, 4-H, β'-H Azf), 2.23-2.14 (m, 1H, β-H Ala, β-H Val), 1.92-1.79 (m, 2H, β-H Ala, β-H Val), 1.09 (d, *J* = 7.2 Hz, 3H, β'-CH₃), 0.95 (d, *J* = 6.8 Hz, 3H, γ-H Val), 0.86 (d, *J* = 6.7 Hz, 3H, γ-H Val). ¹³C NMR (100 MHz, CDCl₃): δ 180.7, 175.6, 173.0, 170.4 (CO), 155.6 (NHCOO), 135.6, 130.9, 128.5, 126.9 (Ar), 113.3 (t, *J* = 241.4 Hz, CHF₂), 76.3 (Cα Azf), 63.3 (t, *J* = 28.8 Hz, OCH₂), 56.3 (Cα Val), 54.8 (Cα Ala), 54.1 (Cγ' Azf), 41.0 (C5), 40.3 (C3), 37.7 (Cβ Azf), 32.2 (Cβ Ala), 29.8 (Cβ Val), 29.7 (Cβ' Azf), 29.6 (C4), 19.6, 17.3 (Cγ Val), 14.9 (β'-CH₃).MS (ES)⁺: 566.3 [M+H]⁺.

### General Procedures for the Synthesis of C-terminal α-Hydroxymethyl Derivatives.

To a stirred solution of methoxy carbonyl derivatives (1 eq) in dry THF (10 mL/mmol) was added LiBH₄ (5 eq) at 0 °C under an Argon atmosphere. The reaction mixture was stirred at 0 °C for 1 h. The reaction was quenched by the drop wise addition of 1.0 M HCl (aq) (1.2 mL) with cooling in an ice bath. The solution was diluted with ethyl acetate and H₂O. The organic layer was washed with brine and dried over Na₂SO₄. Then the solvent was evaporated to dryness, and the residue was purified on a silica gel as specified in each case.

### Boc-L-Tbg-(αS,β'R)-Azf-NH-25-3-(3'S-2'-oxopyrrolidin-3'-yl)-propan-1-lo (80)

Syrup. Yield: 50% (from 56). Eluents: MeOH:DCM (1:15). HPLC: t_{R}= 8.56 min (gradient from 10 to 95% of A, in 10 min). ¹H NMR (400 MHz, CDCl₃): δ 7.41 (d, *J* = 8.5 Hz, 1H, 2-NH), 7.31-7.16 (m, 5H, Ph), 5.97 (br s, 1H, OH), 5.91 (br s, 1H, 1'-H), 5.29 (d, *J* = 9.5 Hz, 1H, α-NH Tbg), 4.12 (m, 1H, 2-H), 3.82 (d, *J* = 9.5 Hz, 1H, α-H Tbg), 3.71 (dd, *J* = 11.6, 5.6 Hz,, 1H, 1-H), 3.61 (d, *J* = 14.1 Hz, 1H, β-H Azf), 3.56 (dd, *J* = 11.6, 5.6 Hz,, 1H, 1-H1), 3.36-3.21 (m, 4H, 5'-H, y'-H Azf), 3.17 (d, *J* = 14.1 Hz, 1H, β-H Azf), 2.55(m, 1H,β'-H Azf), 2.48-2.37 (m, 2H, 3'-H, 4'-H), 2.05 (m, 1H, β-H Ala), 1.85 (m, 1H, 4'-H), 1.57 (m, 1H, 3-H Ala), 1.49 (s, 9H, C(CH₃)), 1.19 (d, *J* = 7.1 Hz, 3H, β'-CH₃), 0.98 (s, 9H, γ-H Tbg). ¹³C NMR (100 MHz, CDCl₃): δ 180.2, 173.4, 171.2 (CO), 155.4 (NHCOO), 135.6, 130.9, 128.6, 127.3 (Ar), 80.0 (C(CH₃)), 78.2 (Ca Azf), 65.10 (C1), 57.6 (Ca Tbg), 54.6 (Cy' Azf), 50.4 (C2), 40.4 (C5'), 39.0 (Cβ Azf), 38.3 (C3'), 35.7 (Cβ Tbg), 32.4 (C3 Ala), 30.4 (Cβ' Azf), 28.6 (C(CH₃)), 28.5 (C4'), 26.5 (Cy Tbg), 16.5 (β'-CH₃). MS (ES)⁺: 559.4 [M+H]⁺.

### Methyloxycarbonyl-L-Tbg-(αS,β'R)-Azf-NH-2S-3-[(3'S-2'-oxopyrrolidin-3'-yl)-propan-1-ol (81)

Syrup. Yield: 60% (from **72**). Eluents: MeOH:DCM (1:15). HPLC: t_{R}= 6.77 min (gradient from 10 to 95% of A, in 10 min). ¹H NMR (400 MHz, CDCl₃): δ 7.36 (d, *J* = 8.5 Hz, 1H, 2-NH), 7.26-7.14 (m, 5H, Ph), 6.0 (br s, 1H,1-H), 5.53 (d, *J* = 9.8 Hz, 1H, α-NH Tbg), 4.13 (m, 1H, 2-H), 3.84 (d, *J* = 9.8 Hz, 1H, α-H Tbg), 3.77 (s, 3H, OCH₃), 3.61 (m, 2H, 1-H), 3.61 (d, *J=* 14.0 Hz, 1H, β-H Azf), 3.32 (m, 2H, 5'-H), 3.24 (m, 2H, γ'-H Azf), 3.15 (d, *J =* 14.0 Hz, 1H, β-H Azf), 2.59 (m, 1H, β'-H Azf), 2.50-2.36 (m, 2H, 3'-H, 4'-H), 2.06 (m, 1H, β-H Ala), 1.85 (m, 1H, 4'-H), 1.20 (d, *J* = 7.1 Hz, 3H, β'-CH₃). 0.99 (s, 9H, γ-H Tbg). ¹³C NMR (100 MHz, CDCl₃): δ 180.2, 173.4, 171.1 (CO), 156.7 (NHCOO), 135.4, 130.9, 128.5, 127.3 (Ar), 77.9 (Ca Azf), 65.1 (C1), 57.6 (Ca Tbg), 54.5 (Cγ' Azf), 52.6 (OCH₃), 50.3 (C2), 40.4 (C5'), 38.9 (Cβ Azf), 38.3 (C3), 35.4 (Cβ Tbg), 32.4 (C3'), 30.2 (CP' Azf), 28.4 (C4'), 26.4 (Cγ Tbg), 16.5 (β'-CH₃). MS (ES)⁺: 517.2 [M+H]⁺.

### 2,2-Difluoroethyloxycarbonyl-L-Tbg-(αS,β'R)-Azf-NH-2S-3-[(3'S-2'-oxopyrrolidin-3'-yl)-propan-1-ol (82)

Syrup. Yield: 69 % (from **73**). Eluents: MeOH:DCM (1:20). HPLC: t_{R}= 6.88 min (gradient from 15 to 95% of A, in 10 min). ¹H NMR (400 MHz, CDCl₃): δ 7.38 (d, *J* = 8.5 Hz, 1H, 2-NH), 7.27-7.15 (m, 5H, Ph), 5.98 (tt, *J* = 55.1, 3.9 Hz, 1H, OCH₂) 5.92 (brs, 1H, 1'-H), 5.71 (m, 2H, OCH₂), 4.13 (m, 1H, 2-H), 3.82 (d, *J=* 9.6 Hz, 1H, α-NH Tbg), 3.74 (dd, *J* = 11.7, 3.8 Hz, 1H, 1-H), 3.61 (d, *J* = 14.1 Hz, 1H, β-H Azf), 3.56 (dd, *J* = 11.7, 3.8 Hz, 1H, 1-H), 3.34 (m, 2H, 5'-H), 3.26 (m, 2H, y'-H Azf), 3.16 (d, *J* = 14.1 Hz, 1H, β-H Azf), 2.61 (m, 1H, β'-H Azf), 3.34 (m, 2H, 5'-H), 2.48-2.33 (m, 2H, 3-H, 4'-H), 2.06 (m, 1H, 3-H), 1.92 (m, 1H, 3-H), 1.89 (m, 1H, 4'-H), 1.21 (d, *J* = 7.1 Hz, 3H, β'-CH₃), 1.00 (s, 9H, γ-H Tbg).¹³C NMR (100 MHz, CDCl₃): δ 180.2, 172.7, 171.0, 155.0 (CO), 135.4, 130.8, 128.4, 127.4 (Ar), 113.1 (t, *J* = 241.3 Hz, CHF₂), 78.0 (Ca Azf), 65.1 (C1), 63.5 (t, *J* = 29.1 Hz, OCH₂), 58.1 (Ca Tbg), 54.6 (Cγ' Azf), 50.3 (C2), 40.4 (C5'), 39.0 (Cβ Azf), 38.3 (C3'), 35.6 (Cβ Tbg), 32.4 (C3), 30.3 (CP' Azf), 28.5 (C4'), 26.4 (Cy Tbg), 16.5 (β'-CH₃). MS (ES)⁺: 567.2 [M+H]⁺.

### General procedures for the synthesis of the invention's compounds

### General Procedure for the Synthesis of C-terminal α-Cyano Peptidomimetics.

***Method M**.* POCl₃ (1.1 eq) was added dropwise, under Ar atmosphere, to the reaction mixture of the corresponding tripeptide amide derivative (1 eq) in dry dichloromethane (4 mL/mmol) and TEA (3.3 eq). The reaction mixture was stirred at 0 ºC for 30 min and then at rt for an additional 1 h. Then, the mixture was cooled in an ice bath and quenched by adding saturated aqueous NaHCO₃. After the gas evolution ceased, the organic layer was separated and washed with saturated aqueous NaHCO₃, and brine, dried over Na₂SO₄ and finally, purified on centrifugal circular chromatography using the solvent system specified in each case.

***Method N**.* A solution of the corresponding tripeptide amide derivative (1 eq) in dry dichloromethane (5mL/mmol) was added methyl *N-*(triethylammoniosulfonyl)carbamate, inner salt (Burgess reagent, 2.5 eq). The reaction mixture was stirred at rt for 2h. Then, the reaction was quenched with saturated aqueous NaHCOs, and brine. The organic phase was separated, dried over Na₂SO₄ and evaporated to dryness. The residue was purified using silica gel and the solvent system specified in each case.

***Método* O.** A solution of the Z-derivative **46** (70 mg, 0.097 mmol) in MeOH (20 mL) was added Pd/C (14 mg, 20% w/w). The suspension was hydrogenated at rt and atmospheric pressure for 3 h. After filtration of the catalyst, the solvent was evaporated. The residue was dissolved in DCM and, then, converted in the corresponding CN derivative following Method L.

### General Procedures for the Synthesis of C-terminal α-Aldehyde Peptidomimetics.

***Method P**.* To a solution of the corresponding C-terminal hydroxymethyl derivative (1.0 eq) in anhydrous DCM (5 mL/mmol), 1M DIBAL solution in toluene was added, at -78 ºC, and the reaction mixture was stirred for 3h at this temperature. Then, MeOH was added at -78 ºC to quench the reaction. 1M HCl solution was added and stirred for 30 min at rt. The mixture was extracted with DCM, and the organic layer was washed with brine and dried over Na₂SO₄. The solvent was evaporated under reduced pressure, and the residue was purified on centrifugal circular chromatography, silica gel, using the solvent system specified in each case.

***Method Q.*** To a solution of the corresponding hydroxymethyl tripeptide derivative (1 eq) in dry CH₂Cl₂ (7 mL/mmol) was added NaHCOs (4 eq) and 1,1,1-Tris(acetyloxy)-1,1-dihydro-1,2-benziodoxol-3-(1*H*)-one (Dess-Martin reagent, 3 eq). The resulting mixture was stirred at rt for 3 h. Then the reaction was quenched with a saturated NaHCOs solution and a 10 % Na₂S₂O₃ solution. The layers were separated. The organic layer was then washed with brine, dried over anhydrous Na₂SO₄ and concentrated under vacuum.

***Method R.*** The corresponding hydroxymethyl tripeptide derivative (1 eq) was dissolved in dry CH₂Cl₂ (9 mL/mmol), and treated with Dess-Martin Periodinane (1.5 eq) at rt. The reaction mixture was then stirred at rt, exposed to the atmosphere, for 2.5 h. The reaction mixture was then quenched by addition of 1.2 mL of 10% Na₂S₂O_{3(aq)} solutionfollowed by stirring for 15 min. The layers were then separated and the organic phase was washed with 10% Na₂S₂O_{3(aq)} solution, saturated NaHCO₃ solution, H₂O and brine. The DCM layer was then dried over Na₂SO₄ and concentrated. The resulting residue was purified on centrifugal circular chromatography over silica gel using the solvent system specified in each case.

### EXAMPLE 1

### Z-L-Val-(αR,β'S)-Azl-NH-[(1S)-cyano-2-[(3S)-2'-oxopyrrolidin-3'-yl]ethane

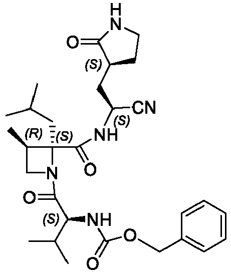

Syrup. Yield: 49% (method M, from intermediate 43). Eluents: MeOH:DCM (1:25). HPLC: t_{R}= 7.89 min (gradient from 10 to 95% of A, in 10 min). [αD]=-46.29 (c 0.10, CHCl₃). ¹H NMR (400 MHz, CDCl₃): δ 9.08 (d, *J* = 7.7 Hz, 1H, 1-NH), 7.34 (m, 5H, Ph), 5.72 (br s, 1H, 1'-H), 5.29 (d, *J* = 9.5 Hz, α-NH Val), 5.10 (s, 2H, OCH₂), 4.99 (q, *J* = 7.9 Hz, 1H, 1-H), 4.46 (t, *J* = 8.4 Hz, 1H, γ'-Azl), 3.91 (dd, *J* = 7.8 Hz, 1H, α-Val), 3.47 (m, 1H, y'-Azl), 3.34 (m, 2H, 5'-H), 2.61 (m, 1H, P'-Azl), 2.51-2.41 (m, 2H, 3'-H, 4'-H), 2.25 (m, 2H, 2-H), 1.96-1.87 (m, 3H, γ-H Azl, β-H Val, β-H Azl), 1.82- 1.74 (m, 2H, 4'-H, β-H Azl), 1.30 (d, *J* = 7.1 Hz, 3H, β'-CH₃), 0.96 (d, *J* = 6.7 Hz, 3H, γ-H Val), 0.95 (d, *J* = 6.8 Hz, 3H, γ-H Val), 0.94 (d, *J* = 6.8 Hz, 6H, δ-H Azl). ¹³C NMR (100 MHz, CDCl₃): δ 178.1, 173.7, 171.6 (CO), 156.5 (CO, NHZ), 136.3, 128.6, 128.4, 128.1 (Ar), 118.3 (CN), 77.9 (Ca Azl), 67.3 (OCH₂), 56.4 (Ca Val), 54.3 (Cy' Azl), 44.9 (Cβ Azl), 40.3 (C5'), 39.3 (C1), 37.8 (C3'), 34.7 (CP' Azl), 34.5 (C2), 31.0 (Cβ Val), 28.4 (C4), 24.8 (Cy Azl), 24.7 (Cδ Azl), 23.2 (Cδ Azl), 19.3, 18.2 (Cy Val), 17.5 (β'-CH₃). MS (ES)⁺: 540.3 [M+H]⁺. Exact Mass calculated for C₂₉H₄₁N₅O₅. 539.3108; found: 539.3096.

### EXAMPLE 2

### Z-L-Val-(αR,β'S)-Azl-NH-[(1S)-1-cyano-2-[(3S)-2'-oxopyrrolidin-3'-yl]ethane

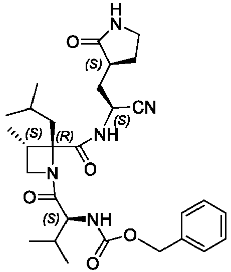

Syrup. Yield: 50% (method N, from intermediate 44). Eluents: MeOH:DCM (1:25). HPLC: t_{R}= 8.22 min (gradient from 10 to 95% of A, in 10 min). [αD] = +33.53 (c 0.10, CHCl₃). ¹H NMR (400 MHz, CDCl₃): δ 8.51 (d, *J* = 7.4Hz, 1H, 1-NH), 7.49-7.29 (m, 5H, Ph), 5.65 (br s, 1H, 1'-H), 5.42 (d, *J* = 6.4 Hz, α-NH Val), 5.23, 5.19 (d, *J* = 12.4 Hz, 1H, OCH₂), 4.99 (m, 1H, 1-H), 4.22 (t, *J* = 8.5 Hz, 1H, γ'-H Azl), 3.88-3.80 (m, 2H, α-H Val, γ'-H Azl), 3.24 (m, 2H, 5'-H), 2.72 (m, 1H, β'-H Azl), 2.54-2.38 (m, 2H, 3'-H, 2-H), 2.36-2.26 (m, 2H, 4'-H), 2.11-1.93 (m, 3H, β-H Val, β-H Azl), 1.92-1.82 (m, 2H, 2-H, γ-H Azl), 1.76 (m, 1H, 4'-H), 1.21 (d, *J* = 7.1 Hz, 3H, β'-CH₃), 1.02 (d, *J* = 6.7 Hz, 3H, γ-H Val), 0.97 (m, 6H, γ-H Val, δ-H Azl), 0.95 (d, *J* = 6.8 Hz, 3H, δ-H Azl). ¹³C NMR (100 MHz, CDCl₃): δ 178.6, 171.6, 170.4 (CO), 157.0 (NHCOO), 136.2, 128.6, 128.5 (Ar), 118.8 (CN), 77.3 (Ca Azl), 67.7 (OCH₂), 56.4 (Ca Val), 54.2 (Cy' Azl), 44.1 (Cβ Azl), 40.3 (C5'), 38.9 (C1), 37.3 (C3'), 34.2 (Cβ' Azl), 33.7 (C2), 30.4 (Cβ Val), 28.1 (C4'), 25.1 (Cδ Azl ), 25.0 (Cγ-Azl), 23.7 (Cδ Azl), 19.5, 18.2 (Cy Val), 15.5 (β'-CH₃). MS (ES)⁺: 540.3 [M+H]⁺. Exact Mass calculated for C₂₉H₄₁N₅O₅: 539.3108; found: 539.3097.

### EXAMPLE 3

### Trifluoroacetyl-L-Val-(αS,β'R)-Azl-NH-[(1S)-1-cyano-2-[(3'S)-2'-oxopyrrolidin-3'-yl]ethane

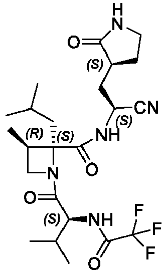

Syrup. Yield: 71% (method M, from intermediate 56). Eluents: Acetone:DCM (1:1). HPLC: t_{R}= 7.32 min (gradient from 10 to 95% of A, in 10 min). [αD]= -95.87 (c 0.12, CHCl₃). ¹H NMR (400 MHz, CDCl₃): δ 8.96 (d, *J* = 6.7 Hz, 1H, 1-NH), 7.50 (d, *J* = 8.2 Hz, 1H, α-NH Val), 5.73 (br s, 1H, 1'-H), 4.74 (m, 1H, 1-H), 4.38 (dd, *J* = 8.2, 5.5 Hz, 1H, α-H Val), 4.25 (t, *J* = 8.6 Hz, 1H, γ'-H Azl), 3.82 (dd, *J* = 8.6, 6.1 Hz, 1H, γ'-H Azl), 3.54 (m, 2H, 5'-H), 2.79 (m, 1H, β'-H Azl), 2.62 (m,1, 3'-H), 2.50 (m, 1H, 4'-H), 2.42 (m, 1H, 2-H), 2.30-1.80 (m, 6H, 4'-H, 2-H, γ-H Azl, β-H Val, β-H Azl), 1.23 (d, *J* = 7.1 Hz, 3H, β'-CH₃), 1.03, 0.99 (d, *J* = 6.8 Hz, 3H, γ-H Val), 0.96, 0.94 (d, *J* = 6.8 Hz, 3H, δ-H Azl). ¹³C NMR (100 MHz, CDCl₃): δ 179.3, 170.5, 169.6 (CO), 157.3 (q, *J* = 37.0 Hz, COCF₃), 118.3 (CN), 114.7 (q, *J* = 286.9 Hz, CF₃), 78.6 (Ca Azl), 55.1 (Ca Val), 54.2 (Cy' Azl), 43.2 (Cβ Azl), 40.7 (C5'), 40.2 (C1), 37.9 (C3'), 34.0 (CP' Azl), 33.2 (C2), 30.7 (Cβ Val), 28.7 (C4'), 25.1 (Cδ Azl), 24.8 (Cy Azl), 23.8 (Cδ Azl), 19.9, 17.3 (Cγ Val), 15.4 (β'-CH₃). MS (ES)⁺: 502.2 [M+H]+. Exact Mass calculated for C₂₃H₃₄ F₃N₅O₄: 501.2563; found: 501.2558.

### EXAMPLE 4

### Trifluoroacetyl-L-Val-(αR,β'S)-Azl-NH-[(1S)-1-cyano-2-[(3'S)-2'-oxopyrrolidin-3'-yl]ethane

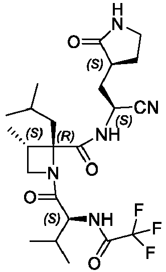

Syrup. Yield: 73% (method N, from intermediate 57). Acetone:DCM (1:1). HPLC: t_{R}= 7.32 min (gradient from 10 to 95% of A, in 10 min). [αD] = +40.28 (c 0.46, CHCl₃)^{1.} H NMR (400 MHz, CDCl₃): δ 8.82 (d, *J* = 7.4 Hz, 1H, 1-NH), 6.86 (d, *J* = 8.8 Hz, 1H, α-NH Val), 5.53 (br s, 1H, 1'-H), 4.97 (q, *J* = 7.9 Hz, 1H, 1-H), 4.40 (t, *J* = 8.4 Hz, 1H, γ'-H Azl), 4.26 (dd, *J* = 8.8, 7.3 Hz, 1H, α-H Val), 3.55 (dd, *J* = 8.4, 4.9 Hz, 1H, γ'-H Azl), 3.37 (m, 2H, 5'-H), 2.68 (m, 1H, β'-H Azl), 2.68 (m, 1H , β'-H), 2.55-2.38 (m, 3H, 2-H, 3'-H, 4'-H), 2.28 (m, 1H, 2-H), 2.15-1.99 (m, 4H, β-H Azl, γ-H Azl, β-H Val), 1.94-1.80 (m, 2H, β-H Azl, 2-H, 4'-H), 1.32 (d, *J* = 7.1 Hz, 3H, β'-CH₃), 1.02, 0.98 (d, *J* = 6.8 Hz, 6H, γ-H Val), 0.97 and 0.95 (d, *J* = 6.8 Hz, 6H, δ-H Azl). ¹³C NMR (100 MHz, CDCl₃): δ 178.1, 171.4, 171.1 (CO), 157.4 (q, *J* = 37.0 Hz, COCF₃), 118.2 (CN), 114.6 (q, *J* = 286.9 Hz, CF₃), 78.0 (Cα Azl), 54.8 (Cα Val), 54.3 (Cy' Azl), 44.5 (Cβ Azl), 40.3 (C5'), 39.5 (C1), 37.9 (C3'), 34.5 (CP' Azl ), 34.4 (C2), 31.3 (Cβ Val), 28.6 (C4'), 24.8 (Cδ Azl), 24.7 (Cγ-Azl), 23.1 (Cδ Azl),19.3, 17.9 (Cy Val), 17.1 (β'-CH₃). MS (ES)⁺: 502.2 [M+H]⁺. Exact Mass calculated for C₂₃H₃₄ F₃N₅O₄: 501.2563; found: 501.2552.

### EXAMPLE 5

### Z-L-Val-(αS,β'R)-Azf-NH[(1S)-1-cyano-2-[(3S)-2-oxopyrrolidin-3-yl]ethane.

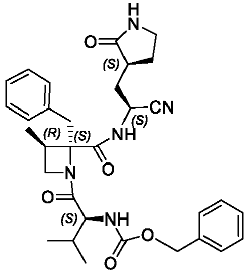

Syrup. Yield: 56% (method N, from intermediate **45**). Eluents: MeOH:DCM (1:9). HPLC (t_{R}, min): 7.93 (gradient from 10 to 95% of A, in 10 min). [αD] = -25.45 (c 0.10, CHCl₃). ¹H NMR (400 MHz, CDCl₃): δ 8.83 (d, *J* = 7.4 Hz, 1H, 1-NH), 7.43-7.32 (m, 5H, Ph), 7.22-7.11 (m, 5H, Ph), 5.84 (s, 1H, 1'-H), 5.39 (d, *J=* 9.3 Hz, α-NH Val), 5.18 (d, *J* = 12.3 Hz, 1H, OCH₂), 5.13 (d, *J* = 12.3 Hz, 1H, OCH₂), 4.52 (q, *J* = 7.8 Hz, 1H, 1-H), 3.86 (dd, *J* = 9.3, 7.2 Hz, 1H, α-H Val), 3.56 (d, *J* = 13.9 Hz, 1H, β-H Azf), 3.38-3.34 (m, 3H, 5'-H, γ'-H Azf), 3.20 (dd, *J* = 8.2, 4.2 Hz, 1H, γ'-H Azf), 3.06 (d, *J* = 13.9 Hz, 1H, β-H Azf), 2.62 (m, 1H, β'-H Azf), 2.54-2.45 (m, 2H, 3'-H, 4'-H), 2.29 (ddd, *J* = 13.9, 8.0, 5.8 Hz, 1H, 2-H), 2.00-1.86 (m, 3H, 2-H, 4'-H, β-H Val), 1.21 (d, *J=* 7.2 Hz, 3H, β'-CH₃), 0.95-0.92 (m, 6H, γ-H Val). ¹³C (100 MHz, CDCl₃): δ 178.4, 174.0, 171.7 (CO), 156.1 (NHCOO), 136.4, 134.6, 130.6, 128.8, 128.6, 128.4, 128.3, 127.6 (Ar), 118.2 (CN), 77.8 (Cα Azf), 67.2 (OCH₂), 56.2 (Cα Val), 53.9 (Cy' Azf), 40.5 (C1), 39.9 (C5'), 39.6 (Cβ Azf), 38.0 (C3'), 34.3 (C2), 31.5 (Cβ Val), 31.3 (Cβ' Azf), 28.5 (C4'), 19.3, 17.9 (Cy Val), 16.8 (β'-CH₃). MS (ES)⁺: 574.2 [M+H]⁺. Exact mass calculated for C₃₂H₃₉N₅O₅: 573.2943; found: 573.2951.

### EXAMPLE 6

### Boc-L-Val-(αS,β'R)-Azf-NH-[(1S)-1-cyano-2-[(3'S)-2'-oxopyrrolidin-3'-yl]ethane

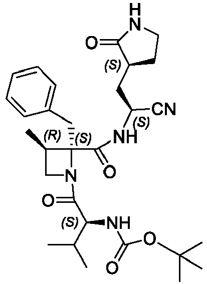

Syrup. Yield: 50% (method N, from intermediate 60). Eluents: MeOH:DCM (1:25). HPLC: t_{R}= 9.13 min (gradient from 10 to 95% of A, in 10 min). [αD]= -64.18 (c 0.10, CHCl₃). ¹H NMR (400 MHz, CDCl₃): δ 8.89 (d, *J* = 7.5 Hz, 1H, 1-NH), 7.32-7.16 (m, 5H, Ph), 5.79 (br s, 1H, 1'-H), 5.12 (d, *J* = 9.3 Hz, 1H, α-NH Val), 5.00 (q, *J* = 7.9 Hz, 1H, 1-H), 3.84 (dd, *J* = 9.4, 6.7 Hz, 1H, α-H Val), 3.58 (d, *J* = 13.9 Hz, 1H, β-H Azf), 3.41-3.31 (m, 3H, γ'-H Azf, 5'-H), 3.19 (dd, *J* = 8.2, 4.1 Hz, 1H, γ'-H Azf), 3.08 (d, *J* = 13.9 Hz, 1H, β-H Azf), 2.60 (m, 1H, β'-H Azf), 2.48 (m, 2H, 4'-H,3'-H ), 2.29 (m, 1H, 2-H), 1.98-1.83 (m, 3H, 2-H, 4'-H, β-H Val), 1.50 (s, 9H, C(CH₃)), 1.22 (d, *J* = 7.1 Hz, 3H, β'-CH₃), 0.93, 0.92 (d, *J* = 7.0 Hz, 6H, γ-H Val). ¹³C NMR (100 MHz, CDCl₃): δ 178.2, 174.4, 171.8 (CO), 155.7 (NHCOO), 134.8, 130.7, 128.7, 127.6 (Ar), 118.2 (CN), 80.0 (C(CH₃)), 77.9 (Ca Azf), 55.7 (Ca Val), 53.9 (Cy' Azf), 40.4 (C5'), 40.1 (Cβ Azf), 39.6 (C3'), 37.9 (C1), 34.4 (C2), 31.6 (CP' Azf ), 31.2 (Cβ Val), 28.5 (C(CH₃) ), 28.4 (C4'), 19.4, 17.8 (Cy Val), 16.9 (β'-CH₃). MS (ES)⁺: 540.3 [M+H]⁺. ]⁺. Exact Mass calculated for C₂₉H₄₁N₅O₅: 539.3108; found: 539.3099.

### EXAMPLE 7

### Trifluoroacetyl-L-Val-(αS,β'R)-Azf-NH-[(1S)-1-cyano-2-[(3'S)-2'-oxopyrrolidin-3'-yl]ethane

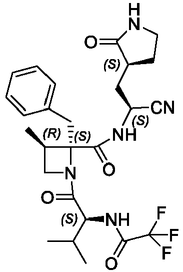

Syrup. Yield: 50% (method N, from intermediate **58**). Eluents: MeOH:DCM (1:20). HPLC: t_{R}= 8.49 min (gradient from 10 to 95% of A, in 10 min). [αD]=-55.12 (c 0.14, CHCl₃). ¹H NMR (400 MHz, CDCl₃): δ 8.75 (d, *J* = 7.0 Hz, 1H, 1-NH), 7.27-7.08 (m, 5H, Ph), 7.00 (d, *J* = 9.1 Hz, 1H, α-NH Val), 5.66 (br s, 1H, 1'-H), 5.00 (dt, *J* = 8.8, 7.2 Hz, 1H, 1-H), 4.15 (dd, *J* = 9.1, 6.9 Hz, 1H, α-H Val), 3.58 (d, *J* = 13.9 Hz, 1H, β-H Azf), 3.41-3.22 (m, 4H, γ'-H Azf, 5'-H), 3.08 (d, *J* = 13.9 Hz, 1H, β-H Azf), 2.68 (m, 1H, 3'-H), 2.54-2.41 (m, 2H, H4', β'-H Azf), 2.30 (m, 1H, 2-H), 2.11-1.85 (m, 3H, 2-H, 4'-H, β-H Val), 1.23 (d, *J* = 7.1 Hz, 3H, β'-CH₃), 1.00,0.95 (d, *J* = 6.8 Hz, 6H, γ-H Val). ¹³C NMR (100 MHz, CDCl₃): δ 178.4, 171.8, 171.1 (CO), 157.5 (q, *J* = 37.0 Hz, COCF₃), 134.6, 130.5, 128.7, 127.8 (Ar), 118.2 (CN), 114.6 (q, *J* = 286.9 Hz, CF₃), 77.8 (Ca Azf), 54.5 (Ca Val), 53.9 (Cy' Azf), 40.5 (C5'), 40.0 (C1), 39.5 (Cβ Azf), 38.1 ( C3'), 34.1 (C2), 31.5 (CP' Azf), 31.4 (Cβ Val), 28.7 (C4'), 19.3, 17.7 (Cy Val), 16.4 (β'-CH₃). MS (ES)⁺: 536.1 [M+H]⁺. Exact Mass calculated for C₂₆H₃₂F₃N₅O₄: 535.2406; found: 535.2395.

### EXAMPLE 8

### Cyclopropylcarbonyl-L-Val-(αS,β'R)-Azf-NH[(1S)-1-cyano-2-[(3'S)-2'-oxopyrrolidin-3'-yl]ethane

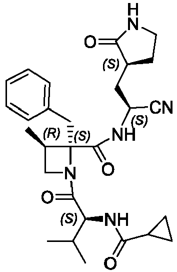

Syrup. Yield: 15% (method N, from intermediate **61**). Eluents: MeOH:DCM (1:9). HPLC (t_{R}, min): 6.32 (gradient from 10 to 95% of A, in 10 min). [αD] = -147.06 (c 0.10, CHCl₃). ¹H NMR (400 MHz, CDCl₃): δ 8.90 (d, *J* = 7.6 Hz, 1H, 1-NH), 7.29-7.25 (m, 3H, Ph), 7.15-7.13 (m, 2H, Ph), 6.34 (d, *J* = 8.9 Hz, 1H, α-NH Val), 5.99 (s, 1H, 1'-H), 5.00 (q, *J* = 7.9 Hz, 1H, 1-H), 4.19 (dd, *J* = 9.0, 7.7 Hz, 1H, α-H Val), 3.56 (d, *J* = 13.9 Hz, 1H, β-H Azf), 3.52 (t, *J* = 8.3 Hz, 1H, γ'-H Azf), 3.40-3.31 (m, 2H, 5'-H), 3.24 (dd, *J* = 8.3, 4.2 Hz, 1H, y'-H Azf), 3.09 (d, *J* = 13.9 Hz, 1H, β-H Azf), 2.59 (dtd, *J* = 8.3, 7.1, 4.2 Hz, 1H, β'-H Azf), 2.51-2.43 (m, 2H, 3'-H, 4'-H), 2.29 (ddd, *J* = 14.1, 8.2, 5.8 Hz, 1H, 2-H), 1.99-1.86 (m, 3H, β-H Val, 2-H, 4'-H), 1.49 (tt, *J* = 7.9, 4.6 Hz, 1H, CH cPr), 1.21 (d, *J* = 7.1 Hz, 1H, β'-CH₃), 1.04-0.99 (m, 2H, CH₂ cPr), 0.94, 0.93 (d, *J* = 6.7 Hz, 6H, γ-H Val), 0.84 (m, 2H, CH₂ Cpr). ¹³C (100 MHz, CDCl₃): 178.3, 174.1, 173.3, 171.7 (CO), 134.7, 130.6, 128.7, 127.6 (Ar), 118.2 (CN), 78.1 (Cα Azf), 54.2 (Cα Val), 54.0 (Cγ' Azf), 40.5 (C5'), 40.3 (Cβ Azf), 39.5 (C1), 37.9 (C3'), 34.4 (C2), 31.8 (CP' Azf), 31.4 (Cβ Val), 28.4 (C4'), 19.3, 18.2 (Cγ Val), 16.8 (β'-CH₃), 14.8(CH cPr), 7,8, 7.7 (CH2 cPr). MS (ES)⁺: 508.2 [M+H]⁺. Mass calculated for C₂₈H₃₇N₅O₄. 507.2838; found: 507.2846.

### EXAMPLE 9

### Z-L-Val-(αS,β'R)-Azy-NH[(1S)-1-cyano-2-[(3'S)-2'-oxopyrrolidin-3'-yl]ethane

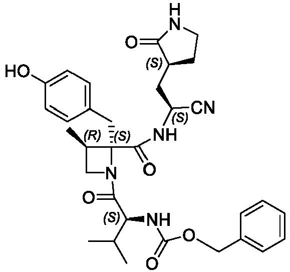

Syrup. Yield: 40% (method N, from intermediate **46**). Eluents: MeOH:DCM (1:9). HPLC (t_{R}, min): 7.93 (gradient from 10 to 95% of A, in 10 min). [αD] = -12.31 (c 0.10, CHCl₃). ¹H NMR (400 MHz, CDCl₃): δ 8.80 (d, *J* = 6.5 Hz, 1H, 1-NH), 7.44-7.35 (m, 5H, Ph), 6.96 (d, *J* = 7.3 Hz, 2H, C₆H₄), 6.54 (d, *J=* 7.2 Hz, 2H, C₆H₄), 5.87 (s, 1H, 1'-H), 5.59 (d, *J* = 9.3 Hz, α-NH Val), 5.20 (d, *J* = 12.3 Hz, 1H, OCH₂), 5.12 (d, *J* = 12.2 Hz, 1H, OCH₂), 4.95 (q, *J* = 8.2 Hz, 1H, 1-H), 3.85 (m, 1H, α-H Val), 3.46 (d, *J* = 14.0 Hz, 1H, β-H Azy), 3.40-3.34 (m, 3H, 5'-H, γ'-H Azy), 3.20 (dd, *J* = 8.4, 4.1 Hz, 1H, γ'-H Azy), 2.96 (d, *J* = 13.9 Hz, 1H, β-H Azy), 2.59 (m, 1H, β'-H Azy), 2.51-2.42 (m, 2H, 3'-H, 4'-H), 2.29 (m, 1H, 2-H), 1.99-1.88 (m, 3H, 2-H, 4'-H, β-H Val), 1.21 (d, *J* = 7.0 Hz, 3H, β'-CH₃), 0.93 (m, 6H, γ-H Val).¹³C (100 MHz, CDCl₃): δ 178.4, 173.9, 171.7 (CO), 156.2 (NHCOO), 155.3, 136.7, 131.7, 128.8, 128.4, 128.1, 126.2 (Ar), 118.2 (CN), 115.5 (Ar), 78.0 (Ca Azy), 67.2 (OCH₂), 56.1 (Cα Val), 53.9 (Cy' Azy), 40.4 (C5'), 39.6 (C1), 39.1 (Cβ Azy), 37.9 (C3'), 34.2 (C2), 31.4, 31.3 (Cβ Val, Cβ' Azy), 28.4 (C4'), 19.2, 17.8 (Cy Val), 16.8 (β'-CH₃). MS (ES)⁺: 590.2 [M+H]⁺. Mass calculated for C₃₂H₃₉N₅O₆: 589.29; found: 589.2309.

### EXAMPLE 10

### Z-L-Val-(αS,β'R)-Azy(^{t}Bu)-NH[(1S)-1-cyano-2-[(3'S)-2'-oxopyrrolidin-3'-yl]ethane

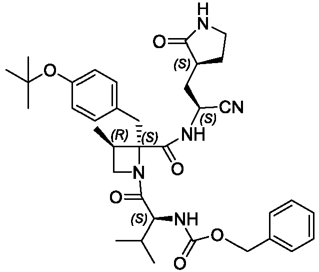

Syrup. Yield: 60% (method N, from intermediate 47). Eluents: MeOH:DCM (1:20). HPLC (t_{R}, min): 9.89 (gradient from 10 to 95% of A, in 10 min). [αD] = +140.80 (c 0.11, CHCl₃). ¹H NMR (400 MHz, CDCl₃): δ 8.83 (d, *J* = 7.5 Hz, 1H, 1-NH), 7.42-7.30 (m, 5H, Ph), 7.03 (d, *J* = 8.4 Hz, 2H, C₆H₄), 6.85 (d, *J* = 8.4 Hz, 2H, C₆H₄), 5.90 (s, 1H, 1'-H), 5.41 (d, *J* = 9.4 Hz, α-NH Val), 5.16 (m, 2H, OCH₂), 4.98 (q, *J* = 7.9 Hz, 1H, 1-H), 3.87 (dd, *J* = 9.4, 7.1, 1H, α-H Val), 3.53 (d, *J* = 14.0 Hz, 1H, β-H Azy), 3.38-3.33 (m, 3H, 5'-H, γ'-H Azy), 3.21 (dd, *J* = 8.2, 4.2 Hz, 1H, γ'-H Azy), 3.01 (d, *J* = 14.1 Hz, 1H, β-H Azf), 2.65-2.56 (m, 1H, β'-H Azf), 2.53-2.43 (m, 2H, 3'-H, 4'-H), 2.28 (ddd, *J* = 14.0, 8.1, 5.9 Hz, 1H, 2-H), 1.99-1.85 (m, 3H, 2-H, 4'-H, β-H Val), 1.31 (OC(CH₃)₃), 1.21 (d, *J* = 7.0 Hz, 3H, β'-CH₃), 0.93 (t, *J* = 7.3 Hz, 6H, γ-H Val). ¹³C (100 MHz, CDCl₃): δ 178.4, 173.9, 171.8 (CO), 156.1 (COO), 154.9, 136.5, 131.1, 129.4, 128.8, 128.4, 128.0, 124.2 (Ar), 118.2 (CN), 78.6 (OC(CH₃)₃), 77.9 (Ca Azf), 67.2 (OCH₂), 56.2 (Ca Val), 54.0 (Cy' Azf), 40.4 (C5'), 39.6 (C1), 39.2 (Cβ Azf), 37.9 (C3'), 34.3 (C2), 31.4, 31.3 (Cβ Val, Cβ' Azf), 29.0 (OC(CH₃)₃), 28.4 (C4'), 19.2, 17.9 (Cγ Val), 16.8 (β'-CH₃). MS (ES)⁺: 646.43 [M+H]⁺. Exact mass calculated for C₃₆H₄₇N₅O₆: 645,3526; found:645.3534.

### EXAMPLE 11

### Z-L-Val-(αS,β'R)-Azw-NH-[(1S)-1-cyano-2-[(3'S)-2'-oxopyrrolidin-3'-yl]ethane

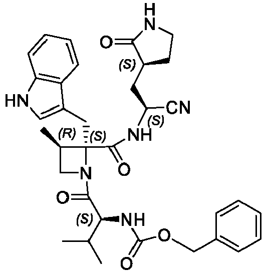

Syrup. Yield: 70% (method N, from intermediate 48). Eluents: MeOH:DCM (1:20). HPLC: t_{R}= 6.91 min (gradient from 30% to 95% of A, in 10 min). [αD] = +229.14 (c 0.11, CHCl₃). ¹H NMR (400 MHz, CDCl₃): δ 8.70 (d, *J* = 7.5 Hz, 1H, 1-NH), 7.59-6.64 (m, 11H, Ar, NH-In), 5.68 (br s, 1H, 1-H), 5.47 (d, *J* = 9.5 Hz, α-NH Val), 5.30 (s, 2H, OCH₂), 3.87 (dd, *J* = 9.6, 6.6 Hz, 1H, α-H Val), 3.61 (d, *J* = 14.9 Hz, 1H, β-H Azw), 3.35 (m, 2H, 5'-H), 3.29 8(d, *J* = 14.9 Hz, 1H, β-H Azw), 3.25 (m, 1H, γ'-H Azw), 3.12 (m, 1H, γ'-H Azw), 2.62 (m, 1H, 3'-H), 2.50-2.44 (m, 2H, P'-H Azl, 4'-H), 2.30 (m, 1H, 2-H), 2.02-1.84 (m, 3H, 2-H, β-H Val, 4'-H), 1.17 (d, *J* = 7.1 Hz, 3H, β'-CH₃), 0.95 (d, *J* = 6.8 Hz, 3H, γ-H Val), 0.95 (d, *J* = 6.8 Hz, 3H, γ-H Val). ¹³C NMR (100 MHz, CDCl₃): δ 178.2, 174.2, 172.1 (CO), 156.2 (COO), 137.2, 135.8, 129.0, 128.7, 128.6, 128.4, 124.7, 122.1, 119.8, 118.5 (Ar), 118.3 (CN), 111.5, 108.3 (Ar), 79.1 (Ca Azw), 67.1 (OCH₂), 56.1 (Ca Val), 54.3 (Cy' Azw), 40.4 (C5'), 39.6 (C3'), 37.9 (C1), 34.4 (C2), 31.9 (CP' Azw), 31.3 (Cβ Val), 29.7 (C4'), 28.5 (Cβ Azw), 19.3, 17.7 (Cγ Val), 16.9 (β'-CH₃). MS (ES)⁺: 613.4 [M+H]⁺. Exact Mass calculated for C₃₄H₄₆N₆O₅: 612.3060; found: 612.3052.

### EXAMPLE 12

### Boc-L-Tbg-(αS,β'R)-Azl-NH-[(1S)-1-cyano-2-[(3S)-2-oxopyrrolidin-3-yl]ethane

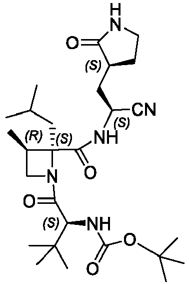

Syrup. Yield: 74% (method N, from intermediate 49). Eluents: MeOH:DCM (1:20). HPLC: t_{R}= 8.78 min (gradient from 15 to 95% of A, in 10 min). [αD] = -262.05 (c 0.13, CHCl₃). ¹H NMR (400 MHz, CDCl₃): δ 9.16 (d, *J* = 7.7 Hz, 1H, 1-NH), 5.82 (s, 1H, 1'-H), 5.07 (d, *J* = 9.8 Hz, 1H, NHBoc), 4.99 (q, *J* = 7.9 Hz, 1H, 1-H), 4.52 (t, *J* = 8.3 Hz, 1H, γ'-Azl), 3.86 (d, *J* = 9.7 Hz, 1H, α-Tbg), 3.47 (dd, *J* = 8.4, 4.3 Hz, 1H, γ'-Azl), 3.36 (m, 2H, 5'-H), 2.59 (m 1H, 3'-H), 2.52-2.44 (m, 2H, P'-Azl, 4'-H), 2.30-2.20 (m, 2H, β-H Azl, 2-H), 1.94-1.76 (m, 4H, β-H Azl, 2-H, 4'-H, γ-H Azl,), 1.43 (s, 9H, C(CH₃)₃), 1.29 (d, *J* = 7.1 Hz, 3H, β'-CH₃), 1.00 (s, 9H, γ-H Tbg), 0.94 (m, 6H, δ-H Azl, δ-H Azl). ¹³C NMR (100 MHz, CDCl₃): δ 178.1, 174.1, 171.9 (CO), 156.1 (NCOO), 118.3 (CN), 80.2 (C(CH₃)₃), 77.9 (Cα Azl), 57.1 (Cα Tbg), 54.8 (Cγ' Azl), 44.6 (Cβ Azl), 40.3 (C5'), 39.2 (C1), 37.8 ( C3'), 34.5 (Cβ Tbg), 34.5 (C2), 34.2 (Cβ' Azl), 28.4 (C4'), 28.4 (C(CH₃)₃), 24.8 (Cy Azl), 26.5 (Cy Tbg), 24.7, 23.2 (Cδ Azl), 17.5 (β'-CH₃). MS (ES)⁺: 520.3 [M+H]⁺. Exact mass calculated for C₂₇H₄₅N₅O₅. 519.334.21; found: 519.3435.

### EXAMPLE 13

### Z-L-Tbg-(αS,β'R)-Azf-NH[(1S)-1-cyano-2-[(3'S)-2-oxopyrrolidin-3-yl]ethane.

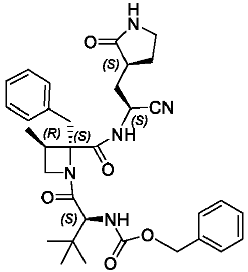

Syrup. Yield: 60% (method N, from intermediate 50). Eluents: MeOH:DCM (1:9). HPLC (t_{R}, min): 9.84 (gradient from 10 to 95% of A, in 10 min). [αD] = -41.67 (c 0.10, CHCl₃). ¹H NMR (400 MHz, CDCl₃): δ 8.82 (d, *J* = 7.5 Hz, 1H, 1-NH), 7.45-7.32 (m, 5H, Ph), 7.15-7.19 (m, 1H, Ph), 7.12-7.07 (m, 4H, Ph), 6.07 (s, 1H, 1'-H), 5.54 (d, *J* = 9.8 Hz, α-NH Tbg), 5.19 (d, *J* = 12.2 Hz, 1H, OCH₂), 5.14 (d, *J=* 12.2 Hz, 1H, OCH₂), 4.99 (q, *J* = 7.9 Hz, 1H, 1-H), 3.85 (d, *J=* 9.8, 1H, α-H Tbg), 3.58 (d, *J=* 13.8 Hz, 1H, β-H Azf), 3.39-3.31 (m, 3H, γ'-H Azf, 5'-H), 3.22 (dd, *J* = 8.1, 4.1 Hz, 1H, γ'-H Azf), 3.03 (d, *J* = 13.8 Hz, 1H, β-H Azf), 2.63-2.54 (m, 1H, β'-H Azf), 2.52-2.43 (m, 2H, 3'-H, 4'-H), 2.29 (ddd, *J* = 14.1, 8.1, 6.0 Hz, 1H, 2-H), 1.98-1.86 (m, 2H, 4'-H, 2-H), 1.21 (d, *J =* 7.1 Hz, 3H, β'-CH₃), 0.98 (s, 9H, γ-H Tbg). ¹³C (100 MHz, CDCl₃): δ 178.4, 173.4, 171.8 (CO), 156.0 (NHCOO), 136.4, 134.6, 130.6, 128.8, 128.54, 128.51, 128.4, 127.5 (Ar), 118.2 (CN), 77.4 (Cα Azf), 67.3 (OCH₂), 57.5 (Cα Tbg), 54.6 (Cy' Azf), 40.4 (C5'), 39.7, 39.6 (Cβ Azf, C1), 37.9 (C3'), 35.5 (Cβ Tbg), 34.4 (C2), 31.1 (Cβ' Azf), 28.4 (C4'), 26.4 (Cγ Tbg), 16.9 (β'-CH₃). MS (ES)⁺: 588.3 [M+H]⁺. Mass calculated for C₃₃H₄₁N₅O₅: 587.3105; found: 587.3108.

### EXAMPLE 14

### Boc-L-Tbg-(αS,β'R)Azf-NH-[(1S)-1-cyano-2-[(3'S)-2'-oxopyrrolidin-3'-yl]ethane

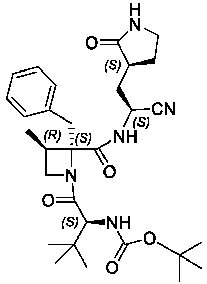

Syrup. Yield: 87% (method N, from intermediate 51). Eluents: MeOH:DCM (1:20). HPLC: t_{R}= 8.29 min (gradient from 10% to 95% of A, in 10 min). [αD] = -33.28 (c 0.12, CHCl₃). ¹H NMR (400 MHz, CDCl₃): δ 8.91 (d, *J* = 7.6 Hz, 1H, 1-NH), 7.32-7.15 (m, 5H, Ph), 5.57 (br s, 1H, 1'-H), 5.25 (d, *J* = 9.7 Hz, 1H, α-NH Tbg), 5.00 (q, *J* = 7.9 Hz, 1H, 1-H), 3.82 (d, *J* = 9.8 Hz, 1H, α-H Tbg), 3.60 (d, *J* = 13.8 Hz, 1H, β-H Azf), 3.45-3.34 (m, 3H, 5'-H, γ'-H Azf), 3.23 (dd, *J* = 8.2, 4.0 Hz, 1H, γ'-H Azf), 3.08 (d, *J* = 13.8 Hz, 1H, β -H Azf), 2.56 (m, 1H, 3'-H), 2.49 (m, 2H, β'-H Azf, 4'-H), 2.30 (m, 1H, 2-H), 1.92 (m, 2H, 4'-H, 2-H), 1.51 (s, 9H, C(CH₃)), 1.21 (d, *J* = 7.1 Hz, 3H, β'-CH₃), 0.98 (s, 9H, γ-H Tbg). ¹³C NMR (100 MHz, CDCl₃): δ 178.0 173.8, 171.9 (CO), 155.4 (NHCOO), 134.8, 130.7, 128.6, 127.5 (Ar), 118.3 (CN), 80.0 (C(CH₃), 77.8 (Cα Azf), 57.1 (Cα Tbg), 54.6 (Cy' Azf), 40.3 (C5'), 40.0 (Cβ Azf), 39.9 (C1), 37.8 (C3'), 35.6 (Cβ Tbg), 34.5 (C2), 31.3 (Cβ' Azf), 28.6 (C(CH₃)), 28.5 (C4'), 26.4 (Cγ Tbg), 17.0 (β'-CH₃). MS (ES)⁺: 554.3 [M+H]⁺. Exact Mass calculated for C₃₀H₄₃N₅O₅. 553.3264; found: 553.3258.

### EXAMPLE 15

### Trifluoroacetyl-L-Tbg-(αS,β'R)-Azf-NH-[(1S)-1-cyano-2-[(3S)-2-oxopyrrolidin-3-yl]ethane

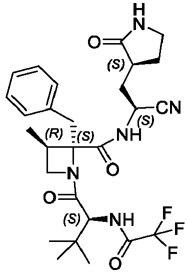

Syrup. Yield: 11% (method N, from intermediate **59**). Eluents: MeOH:DCM (1:20). HPLC: t_{R}= 8.43 min (gradient from 15% to 95% of A, in 10 min). [αD] = -138.95 (c 0.10, CHCl₃). ¹H NMR (500 MHz, CDCl₃): δ 8.73 (d, *J* = 7.0 Hz, 1H, 1-NH), 7.29-7.08 (m, 5H, Ph), 6.99 (d, *J* = 9.6 Hz, 1H, α-NH Tbg), 5.55 (br s, 1H, 1'-H), 4.96 (m, 1H, 1-H), 4.14 (d, *J* = 9.6 Hz, 1H, α-H Tbg), 3.60 (d, *J* = 13.9 Hz, 1H, β-H Azf), 3.39 (m, 2H, 5'-H), 3.27 (m, 2H, γ'-H Azf), 3.04 (d, *J* = 13.9 Hz, 1H, β-H Azf), 2.66 (m, 1H, β'-H Azf), 2.54-2.47 (m, 2H, 3'-H, 4'-H), 2.31 (m, 1H, 2-H), 2.00 (m, 1H, 2-H), 1.93 (m, 3H, 4'-H), 1.23 (d, *J* = 7.1 Hz, 3H, β-CH₃), 1.03 (s, 9H, γ-H Tbg). ¹³C NMR (125MHz, CDCl₃): δ 178.3, 171.2, 171.3 (CO), 156.5 (q, *J* = 37.0 Hz, COCF₃)_{,} 134.5, 130.5, 128.7, 127.9 (Ar), 118.2 (CN), 114.6 (q, *J* = 286.9 Hz, CF₃), 77.8 (Cα Azf), 55.9 (Cα Tbg), 54.7 (Cy' Azf), 40.5 (C5'), 40.0 (C1) 39.2 (Cβ Azf), 38.0 (C3'), 36.3 (Cβ Tbg), 34.2 (C2), 31.1 (Cβ' Azf), 28.7 (C4'), 26.4 (Cγ Tbg), 16.5 (β'-CH₃). MS (ES)⁺: 550.2 [M+H]⁺. Exact Mass calculated for C₂₇H₃₄F₃N₅O₄: 549.2563; found: 549.2571.

### EXAMPLE 16

### Methyloxycarbonyl-L-Tbg-(αS,β'R)-Azf-NH[(1S)-1-cyano-2-[(3'S)-2'-oxopyrrolidin-3'-yl]ethane.

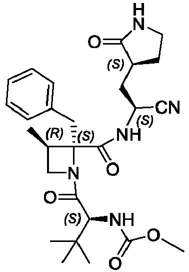

Syrup. Yield: 58% (method M, from intermediate 63). Eluents: MeOH:DCM (1:9). HPLC (t_{R}, min): 7.44 (gradient from 15 to 95% of A, in 10 min). [αD] = -60.68 (c 0.10, CHCl₃). ¹H NRM (400 MHz, CDCl₃): δ 8.83 (d, *J* = 7.6 Hz, 1H, 1-NH), 7.29-7.21 (m, 3H, Ph), 7.16-7.14 (m, 2H, Ph), 5.91 (s, 1H, 1'-H), 5.45 (d, *J* = 9.8 Hz, α-NH Tbg), 5.00 (m, 1H, 1-H), 3.83 (d, *J* = 9.9, 1H, α-H Tbg), 3.76 (s, 3H, OCH₃), 3.59 (d, *J* = 13.9 Hz, 1H, β-H Azf), 3.40-3.31 (m, 3H, γ'-H Azf, 5'-H), 3.24 (dd, *J* = 8.2, 4.2 Hz, 1H, γ'-H Azf), 3.05 (d, *J* = 13.8 Hz, 1H, β-H Azf), 2.65-2.53 (m, 1H, β'-H Azf), 2.55-2.44 (m, 2H, 3'-H, 4'-H), 2.29 (ddd, *J* = 14.0, 8.0, 6.0 Hz, 1H, 2-H), 1.99-1.86 (m, 2H, 4'-H, 2-H), 1.22 (d, *J=* 7.1 Hz, 3H, β'-CH₃), 0.98 (s, 9H, γ-H Tbg). ¹³C (100 MHz, CDCl₃): δ 178.3, 173.6, 171.8 (CO), 156.7 (COO), 134.6, 130.7, 128.6, 127.6 (Ar), 118.3 (CN), 77.4 (Cα Azf), 57.4 (Cα Tbg), 54.6 (Cy' Azf), 52.6 (OCH₃), 40.4 (C5'), 39.7 (Cβ Azf), 39.6 (C1), 37.9 (C3'), 35.3 (Cβ Tbg), 34.4 (C2), 31.1 (CP' Azf), 28.5 (C4'), 26.3 (Cy Tbg), 16.9 (β'-CH₃). MS (ES)⁺: 512.3 [M+H]⁺. Mass calculated for C₂₇H₃₇N₅O₅: 511.2785; found: 511.2795.

### EXAMPLE 17

### p-Tolyloxyacetyl-L-Tbg-(αS,β'R)-Azf-NH[(1S)-1-cyano-2-[(3S)-2-oxopyrrolidin-3-yl]ethane

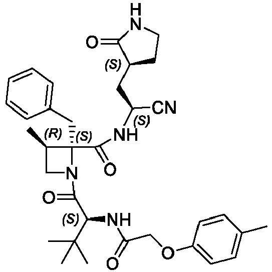

Syrup. Yield: 70% (method M, from intermediate 62). Eluents: MeOH:DCM (1:9). HPLC (t_{R}, min): 9.29 (gradient from 10 to 95% of A, in 10 min). [αD] = -54.84 (c 0.10, CHCl₃). ¹H NMR (400 MHz, CDCl₃): δ 8.84 (d, *J* = 7.3 Hz, 1H, 1-NH), 7.23-7.12 (m, 8H, Ph, C₆H₄, α-NH Tbg), 6.91 (d, *J* = 8.6 Hz, 2H, C₆H₄), 5.85 (s, 1H, 1'-H), 5.00 (q, *J* = 7.6Hz, 1H, 1-H), 4.59 (d, *J* = 15.0 Hz, 2H, OCH₂), 4.53 (d, *J* = 15.0 Hz, 2H, OCH₂), 4.23 (d, *J* = 9.8, 1H, α-H Tbg), 3.59 (d, *J* = 13.8 Hz, 1H, β-H Azf), 3.42-3.33 (m, 3H, γ'-H Azf, 5'-H), 3.28 (dd, *J* = 8.2, 4.2 Hz, 1H, γ'H -Azf), 3.06 (d, *J* = 13.9 Hz, 1H, β-H Azf), 2.61 (m, 1H, β'-H Azf), 2.53-2.46 (m, 2H, 3'-H, 4'-H), 2.35-2.23 (m, 4H, 2-H, CH₃Ph), 2.01-1.88 (m, 2H, 2-H, 4'-H), 1.23 (d, *J=* 7.1 Hz, 3H, β'-CH₃), 0.97 (s, 9H, γ-H Tbg). ¹³C (100 MHz, CDCl₃): δ 178.3, 172.8, 171.7, 167.8 (CO), 155.2, 134.6, 131.9, 130.7, 130.4, 128.5, 127.6 (Ar), 118.3 (CN), 114.8 (Ar), 77.4 (Cα Azf), 67.6 (OCH₂), 54.8 (Cα Tbg), 54.6 (Cy' Azf), 40.4 (C5'), 39.7 (Cβ Azf), 39.6 (C1), 37.9 (C3'), 35.8 (Cβ Tbg), 34.4 (C2), 31.2 (Cβ' Azf), 28.5 (C4'), 26.4 (Cγ Tbg), 20.7 (CH₃Ph), 16.9 (β'-CH₃). MS (ES)⁺: 602.4 [M+H]⁺. Exact mass calculated for C₃₄H₄₃N₅O₅: 601.3265; found: 601.3264.

### EXAMPLE 18

### 2,2-Difluoroethyloxycarbonyl-L-Tbg-(aS,β'R)-Azf-NH-[(1S)-1-cyano-2-[(3S)-2-oxopyrrolidin-3-yl]ethane

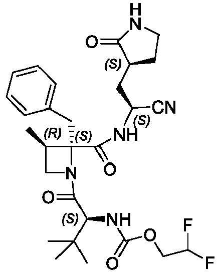

Syrup. Yield: 59% (method N, from intermediate **60**). Eluents: MeOH:DCM (1:15). HPLC: t_{R}= 8.02 min (gradient from 15% to 95% of A, in 10 min). [αD] = -43.62 (c 0.11, CHCl₃). ¹H NMR (400 MHz, CDCl₃): δ 8.78 (d, *J=* 7.5 Hz, 1H, 1-NH), 7.29-7.13 (m, 5H, Ph), 6.00 (tt, *J* = 55.1, 4.0 Hz, 1H, CHF₂), 5.79 (br s, 1H, 1'-H), 5.60 (d, *J* = 9.8 Hz, α-NH Tbg), 4.99 (q, *J=* 7.8 Hz, 1H, 1-H), 4.32 (m, 2H, OCH₂), 3.81 (d, *J=* 9.9 Hz, 1H, α-H Tbg), 3.60 (d, *J* = 13.9 Hz, 1H, β-H Azf), 3.41-3.26 (m, 4H, 5'-H, γ'-H Azf), 3.06 (d, *J* = 13.9 Hz, 1H, β-H Azf), 2.63 (m, 1H, 3'-H), 2.54-2.44 (m, 2H, β'-H Azf, 4'-H), 2.30 (m, 1H, 2-H), 2.00-1.88 (m, 2H, 2-H, 4'-H), 1.23 (d, *J* = 7.1 Hz, 3H, β-CH₃), 1.00 (s, 9H, γ-H Tbg).¹³C NMR (100MHz, CDCl₃): δ 178.3, 172.9, 171.6 (CO), 155.0 (NHCOO), 134.7, 130.7, 128.5, 127.6 (Ar), 118.2 (CN), 113.1 (t, *J* = 241.4 Hz, CHF₂), 77.8 (Cα Azf), 63.50 (t, *J* = 29.3 Hz, OCH₂), 57.7 (Cα Tbg), 54.6 (Cy' Azf), 40.5 (C5'), 39.72 (C1), 39.7 (Cβ Azf), 38.0 ( C3'), 35.6 (Cβ Tbg), 34.4 (C2), 31.1 (CP' Azf), 28.5 (C4'), 26.3 (Cγ Tbg), 16.8 (β'-CH₃). MS (ES)⁺: 562.3 [M+H]+. Exact Mass calculated for C₂₈H₃₇F₂N₅O₅: 561.2763; found: 561.2768.

### EXAMPLE 19

### N-(Methoxycarbonyl)sulfamoyl)-L-Lys(Boc)-(αS,β'R)-Azf-NH-[(1S)-1-cyano-2-[(3S)-2-oxopyrrolidin-3-yl]ethane

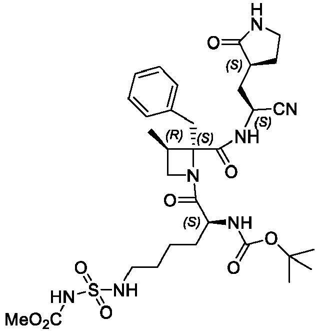

Syrup. Yield: 36% (method N, from intermediate **52**). Eluents: MeOH:DCM (1:15). HPLC: t_{R}= 7.86 min (gradient from 15 to 95% of A, in 10 min). [αD] = -27.85 (c 1.05, CHCl₃). ¹H NMR (400 MHz, CDCl₃): δ 8.58 (d, *J=* 7.8 Hz, 1H, 1-NH), 7.31-7.15 (m, 5H, Ar), 6.52 (br s, 5H, Ar), 6.36 (t, *J* = 6.1 Hz, 1H, NHSO₂NH), 5.34 (d, *J* = 8.8 Hz, 1H, NHBoc), 4.92 (m, 1H, 1-H), 4.04 (m, 1H, α-H Lys), 3.76 (s, 3H, OMe), 3.59 (d, *J* = 13.9 Hz, 1H, β-H Azf), 3.35 (m, 2H, γ'-H Azf, 5'-H), 3.25 (dd, *J* = 8.3, 4.5 Hz, 1H, γ'-H Azf), 3.11 (d, *J* = 14.0 Hz, 1H, β-H Azf), 3.05 (m, 1H, 5'-H), 2.61 (m, 1H, β'-H Azf), 2.50-2.39 (m, 3H, 3'-H, 4'-H, 2-H), 1.96-1.83 (m, 2H, 4'-H, 2-H), 1.75-1.49 (m, 4H, β-H Lys, δ-H Lys), 1.47 (s, 9H, , (C(CH₃)₃),1.38 (m, 2H, γ-H Lys), 1.18 (d, *J* = 7.1 Hz, 3H, β'-CH₃),¹³C NMR (100 MHz, CDCl₃): δ 179.6, 174.0, 171.5 (CO), 155.3 (NHCOO), 152.6 (CO₂Me), 134.9, 130.8, 128.6, 127.5 (Ar), 118.4 (CN), 80.1 (C(CH₃)₃), 77.7 (Cα Azf), 53.6 (Cy' Azf), 53,5 (OMe), 50.3 (Cα Lys), 43.0 (C5'), 40.8 (Cε Lys), 39.3 (Cβ Azf), 39.2 (C1), 38.5 ( C3'), 34.3 (C2), 31.6 (Cβ Lys), 31.3 (CP' Azf), 28.6 (C4'), 28.5 (C(CH₃)₃), 28.2 (Cδ Lys), 21.9 (Cγ Lys), 16.4 (β'-CH₃). MS (ES)⁺: 706.4 [M+Na]⁺. Exact mass calculated for C₃₂H₄₇N₇O₉S: 705,3156; found: 705.3151.

### EXAMPLE 20

### Z-L-Lys(Boc)-(αS,β'R)-Azf-NH-[(1S)-1-cyano-2-[(3S)-2-oxopyrrolidin-3-yl]ethane

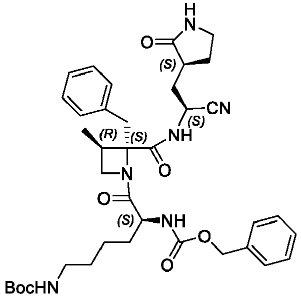

Syrup. Yield: 75% (method N, from intermediate **53**). Eluents: MeOH:DCM (1:20). HPLC: t_{R}= 9.16 min (gradient from 15 to 95% of A, in 10 min). [αD] = +265.82 (c 0.10, CHCl₃). ¹H NMR (400 MHz, CDCl₃): δ 8.97 (br s, 1H, 1-NH), 7.40-7.13 (m, 10H, Ar), 6.53 (brs, 1H,1'-H), 5.52 (d, *J =* 8.6 Hz, 1H, α-NH Lys), 5.16 (d, *J* = 12.3 Hz, 1H, OCH₂), 5.18 (d, *J* = 12.3 Hz, 1H, OCH₂), 5.10 (d, *J* = 12.3 Hz, 1H, OCH₂), 4.82 (m, 1H, 1-H), 4.04 (m, 1H, α-H Lys), 3.63 (d, *J* = 14.1 Hz, 1H, β-H Azf), 3.36 (m, 2H, 5'-H), 3.30 (t, *J* = 8.2 Hz, 1H, γ'-H Azf), 3.22 (m, 1H, γ'-H Azf), 3.15 (d, *J* = 14.1 Hz, 1H, β-H Azf), 3.10 (m, 2H, ε-H Lys), 2.62 (m, 1H, 3'-H), 2.48-2.38 (m, 2H, β'-H Azf, 4'-H), 2.33 (m, 1H, 2-H), 2.33 (m, 1H, 2-H), 1.95-1.70 (m, 2H, β-H Lys, 4'-H), 1.60-1.44 (m, 3H, β-H Lys, δ-H Lys), 1.42 (m, 2H, γ-H Lys), 1.41 (s, 9H, (C(CH₃)₃), 1.12 (d, *J* = 7.1 Hz, 3H, β'-CH₃). ¹³C NMR (100 MHz, CDCl₃): δ 178.8, 173.3, 171.3 (CO), 156.6, 156.0 (NHCOO), 136.5, 135.3, 130.9, 128.7, 128.5, 128.4, 128.2, 127.1 (Ar), 118.2 (CN), 79.5 (C(CH₃)₃), 77.3 (Cα Azf), 67.1 (OCH₂), 53.7 (Cy' Azf), 51.3 (Cα Lys), 40.6 (C5'), 40.4 (Cε Lys), 40.2 (C1), 38.8 ( C3'), 38.6 (Cβ Azf), 33.7 (C2), 31.5 (CP' Azf), 30.9 (Cβ Lys), 29.2 (Cδ Lys), 29.0 (C4'), 28.6 (C(CH₃)₃), 22.3 (Cγ Lys), 16.1 (β'-CH₃). MS (ES)⁺: 725.4 [M+Na]⁺. Exact mass calculated for C₃₈H₅₆N₆O₅: 702,3741; found: 702.3752.

### EXAMPLE 21

### Z-L-Lys-(αS,β'R)-Azf-NH-[(1S)-1-cyano-2-[(3S)-2-oxopyrrolidin-3-yl]ethane

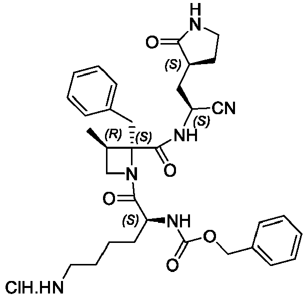

Compound of example **20** (61 mg, 0.087 mmol) was dissolved in trifluoroethanol (1 mL). The stirred solution is cooled in an ice bath and TMSCI (16 µL) is added dropwise. The mixture was same temperature until the reaction completion (4h). Then the reaction was evaporated to dryness (yield crude: 75% (41 mg) and was purified by semipreparative RPHPLC-MS (Waters 2545) coupled to a mass spectrometer 3100 detector, using a SUNFIRETM column C18 (5µ, 10x150 mm) and a flux of 8 mL/min with a gradient of CH₃CN (0.1% HCO₂H) [Solvent A]:H₂O (0.1% HCO₂H) [Solvent B] as mobile phase using a gradient from 30 to 50% of A, in 10 min. HPLC: t_{R}= 5.98 min (gradient from 10 to 95% of A, in 10 min). [αD] = -172.04 (c 0.10, H₂O). ¹H NMR (400 MHz,D₂O): δ 8.32 (br s, 1H, 1-NH), 7.33-7.09 (m, 10H, Ar), 6.97 (br s, 1H,1'-H), 5.06 (d, *J* = 12.8 Hz, 1H, OCH₂), 4.95 (d, *J* = 12.8 Hz, 1H, OCH₂), 4.85 (m, 1H, α-NH Lys), 4.80 (m, 1H, 1-H), 3.85 (dd, *J* = 9.1, 5.1 Hz, α-H Lys), 3.36 (d, *J* = 14.0 Hz, 1H, β-H Azf), 3.31-3.15 (m, 4H, γ'-H Azf, 5'-H), 3.05 (d, *J* = 14.0 Hz, 1H, β-H Azf), 2.79 (m, 2H, ε-H Lys), 2.69 (m, 1 H, β'-H Azf), 2.52 (m, 1H, 3'-H), 2.29-2.20 (m, 2H, 4'-H, 2-H), 1.94 (m, 1H, 2-H), 1.78 (m, 1H, 4'-H), 1.64-1.43 (m, 4H, β-H Lys, δ-H Lys), 1.28 (m, 2H, γ-H Lys), 0.92 (d, *J=* 7.1 Hz, 3H, β'-CH₃), ¹³C NMR (100 MHz, D₂O): δ 180.9, 172.9, 171.0 (CO), 156.7, (NHCOO), 136.4, 134,7, 130.9, 130.8, 128.8, 128.6, 128.4, 127.3 (Ar), 118.3 (CN), 75.6 (Cα Azf), 67.0 (OCH₂), 54.2 (Cy' Azf), 51.7 (Cα Lys), 40.6 (C5'), 39.4 (Cε Lys), 39.3 (C1), 38.0 (Cβ Azf), 38.0 ( C3'), 32.3 (C2), 30.9 (CP' Azf), 29.4 (Cβ Lys), 29.3 (Cδ Lys), 26.5 (C4'), 21.8 (Cγ Lys), 14.0 (β'-CH₃). MS (ES)⁺: 603.4 [M+H]⁺. Exact Mass calculated for C₃₃H₄₂N₆O₅: 6602,3216; found: 602.3217.

### EXAMPLE 22

### Z-L-Tbg-(αS,β'R)-Azk(Boc)-NH-[(1S)-1-cyano-2-[(3S)-2-oxopyrrolidin-3-yl]ethane

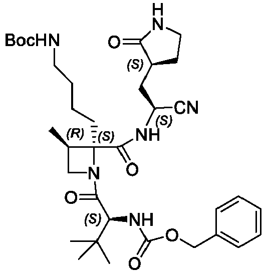

Syrup. Yield: 73% (method N, from intermediate **54**). Eluents: MeOH:DCM (1:20). HPLC: t_{R}= 7.64 min (gradient from 30 to 95% of A, in 10 min). [αD] = -91.80 (c 0.10, CHCl₃). ¹H NMR (400 MHz, CDCl₃): δ 8.77 (d, *J* = 7.9 Hz, 1H, 1-NH), 7.37-7.22 (m, 5H, Ph), 6.05 (brs, 1H, ε-NH Azk), 5.42 (d, *J* = 8.6 Hz, 1H, α-NH Tbg), 5.10 (m, 2H, OCH₂), 4.88 (m, 1H, 1-H), 4.38 (t, *J* = 8.3 Hz, 1H, γ'-Azk), 3.94 (d, *J* = 8.6 Hz, 1H, α-H Tbg), 3.55 (m, 1H, γ'-Azk), 3.37 (m, 2H, ε-H Azk), 2.57 (m, 1H, 3'-H), 2.51-2.40 (m, 2H, P'-Azk, 4'-H), 2.27 (m, 1H, 2-H), 2.05 (m, 2H, δ-H Azk), 2.00-1.79 (m, 2H, 4'-H, 2-H), 1.46-1.39 (m, 12H, C(CH₃)₃, β-H Azk, γ-H Azk), 1.28 (m, 1H, γ-H Azk), 1.23 (d, *J* = 7.1 Hz, 3H, β'-CH₃), 1.01 (s, 9H, γ-H Tbg), ¹³C NMR (100 MHz, CDCl₃): δ 178.6, 173.0, 171.6 (CO), 156.5, 156.3 (NHCOO), 136.3, 128.7, 128.4, 128.2 (Ar), 118.4 (CN), 79.4 (C(CH₃)₃), 77.4 (Cα Azk), 67.2 (OCH₂), 57.7 (Cα Tbg), 54.9 (Cγ' Azk), 40.6 (C5'), 40.3 (Cε Azk), 39.7 (C1), 38.1 (C3'), 34.8 (Cδ Azk), 33.8 (C2), 32.6 (CP' Azk), 29.7 (Cβ Azk), 28.6 (C4', C(CH₃)₃), 26.5 (Cγ Tbg), 20.7 (Cγ Azk), 16.7 (β'-CH₃). MS (ES)⁺: 691.3 [M+Na]⁺. Exact Mass calculated for C₃₅H₅₂N₆O₇: 668.3897; found: 668.3901.

### EXAMPLE 23

### Z-L-Tbg-(αS,β'R')-Azk-NH-[(1S)-1-cyano-2-[(3S)-2-oxopyrrolidin-3-yl]ethane

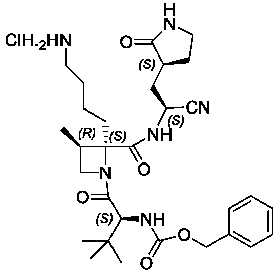

Compound of example **22** (55 mg, 0.082 momol) was dissolved in trifluoroethanol (1 mL). The stirred solution is cooled in an ice bath and TMSCI (15 µL) is added dropwise. The mixture was same temperature until the reaction completion (2h). Then the reaction was evaporated to dryness (yield crude: 61% (30 mg) and was purified by semipreparative RPHPLC-MS (Waters 2545) coupled to a mass spectrometer 3100 detector, using a SUNFIRETM column C18 (5µ, 10x150 mm) and a flux of 8 mL/min with a gradient of CH₃CN (0.1% HCO₂H) [Solvent A]:H₂O (0.1% HCO₂H) [Solvent B] as mobile phase using a gradient from 20 to 45% of A, in 10 min). HPLC: t_{R}= 5.75 min (gradient from 100 to 95% of A, in 10 min). [αD] = -152.85 (c 0.10, H₂O). ¹H NMR (500 MHz, D₂O): δ 8.45 (brs, 1H, 1-NH), 7.46-7.38 (m, 5H, Ph), 6.05 (brs, 1H, ε-NH Azk), 5.58 (d, *J* = 8.6 Hz, 1H, α-NH Tbg), 5.16 (d, *J* = 12.9 Hz, 1H, OCH₂), 5.11 (d, *J* = 12.9 Hz, 1H, OCH₂), 4.88 (m, 1H, 1-H), ), 3.82 (m, 1H, α-H Tbg), 3.42-3.30 (m, 3H, γ'-Azk, 4'-H), 3.13 (dd, *J* = 13.7, 6.4 Hz, 1H, γ'-Azk,), 2.77 (m, 2H, ε-H Azk), 2.84 (m, 1H, P'-Azk), 2.77 (m, 1H, 3'-H), 2.35 (m, 2H, 4'-H), 2.21 (m, 1H, 2-H), 2.04 (m 2H, δ-H Azk), 1.90 (m, 1H, 2-H), 1.66-1.57 (m, 2H, β-H Azk), 1.06-0.96 (m, 14H, β'-CH₃, γ-H Tbg, γ-H Azk). ¹³C NMR (125 MHz, D₂O): δ 180.8, 179.3, 171.1 (CO), 157.8 (NHCOO), 136.7, 128.8, 128.3, 127.3 (Ar), 118.7 (CN), 76.9 (Cα Azk), 67.0 (OCH₂), 64.1 (Cα Tbg), 54.7 (Cy' Azk), 43.6 (C5'), 40.65 (Cε Azk), 40.6 (C1) 39.1 (C3'), 38.2 (Cβ' Azk), 38.0 (Cδ Azk), 37.8 (Cβ Tbg), 33.3 (C2), 32.5 (Cβ' Azk), 26.5 (Cγ Tbg), 25.9 (C4'), 25.85 (Cβ Azk), 25.8 (Cγ Azk), 16.4 (β'-CH₃). MS (ES)⁺: 569.4 [M+H]⁺. Exact mass calculated for C₃₆H₄₄N₆O₅: 568.3373; found: 568.3361.

### EXAMPLE 24

### Z-L-Tbg-(αS,β'R)-Azf-NH-[(1S)-1-cyano-2-[(3S)-1H-imidazol-4-yl]ethane

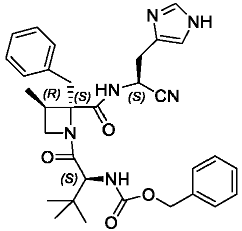

A solution of trityl histidine derivative of intermediate **57** (0.10 mmol, 88 mg) in DCM (1.4 mL), at 0º C, was successively treated with TIPS (0.20 mmol, 43 µL) and TFA (2.0 mmol, 0.16 mL). After the mixture reaction was stirred at rt for 4 h, then the solvent was evaporated under vacuum and lyophilized. The solution of deprotected trityl derivative (0.10 mmol, 74 mg) in dry DCM (0.5 mL), under Ar atmosphere and at 0 ºC, was successively treated with TEA (0.43 mmol, 63 µL) and POCl₃ was added dropwise (0.11 mmol, 11µL). The reaction mixture was stirred at 0 ºC for 30 min and then at rt for an additional 1h. Then, the mixture was cooled in an ice bath and quenched by adding saturated aqueous NaHCOs. After the gas evolution subsided, the organic layer was separated and washed with saturated aqueous NaHCOs, H₂O and brine, dried over Na₂SO₄ and finally, purified on a silica and using as eluents MeOH:DCM (1:20). 13 mg of cyano derivative **23** was obtained (Yield: 22%). HPLC: t_{R}= 6.87 min (gradient from10 to 95% of A, in 10 min). [αD] = -10.93 (c 0.10, CHCl₃). ¹H NMR (400 MHz, CDCl₃): δ 8.76 (d, *J=* 7.8 Hz, 1H, α-1-NH), 7.67 (s, 1H, 2-H Im), 7.44-7.06 (m, 10H, Ar), 7.04 (s, 1H, 5-H Im), 5.56 (d, *J=* 9.7 Hz, 1H, α-NH Tbg), 5.19 (d, *J* = 12.2 Hz, 1H, OCH₂), 5.14 (d, *J* = 12.2 Hz, 1H, OCH₂), 5.10 (m, 1H, 1-H), 3.84 (d, *J* = 9.7 Hz, 1H, α-H Tbg), 3.59 (d, *J* = 13.8 Hz, 1H, β-H Azf), 3.34 (t, *J* = 8.2 Hz, 1H, γ'-H Azf), 3.24 (dd, *J* = 8.2, 4.4 Hz, 1H, γ'-H Azf), 3.17 (d, *J* = 6.6 Hz, 2H, 2-H), 3.09 (d, *J=* 13.8 Hz, 1H, γ'-H Azf), 2.59 (m, 1H, β'-H Azf), 1.16 (d, *J=* 7.1 Hz, 3H, β'-CH₃), 0.98 (s, 9H, γ-H Tbg).¹³C NMR (100 MHz, CDCl₃): δ 173.1, 171.3 (CO), 156.0 (NHCO), 136.4, 135.2, 134.8, 133.4, 130.7, 128.8, 128.5, 128.4, 127.5, 118.1, 115.9 (Ar), 77.3 (Cα Azf), 67.3 (OCH₂), 57.6 (Cα Tbg), 54.6 (Cy' Azf), 41.3 (C1), 39.2 (Cβ Azf), 35.5 (Cβ Tbg), 30.9 (Cβ' Azf), 30.7 (C2), 26.4 (Cγ Tbg), 16.4 (β'-CH₃). MS (ES)⁺: 571.3 [M+H]⁺. Exact mass calculated for C₃₂H₃₈N₆O₄: 570.2955; found: 570.2955.

### EXAMPLE 25

### ^{t}BuNHCO-L-Tbg-(αS,β'R)-Azl-NH-[(1S)-1-cyano]pentane

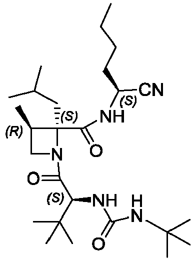

Syrup. Yield: 65% (Method N, from intermediate **67**). Eluents: EtOAc:hexane (2:1). HPLC: t_{R} = 6.72 min (gradient from 50 to 95% of A, in 10 min). [αD] = -77.70 (c 0.10, CHCl₃). ¹H NMR (400 MHz, CDCl₃): δ 9.21 (d, *J* = 8.1 Hz, 1H, 1-NH), 4.72(dt, *J* = 8.1, 6.9 Hz, 1H, 1-H), 4.60 (t, *J* = 8.4 Hz, 1H, Hy' Azl), 4.02 (s, 1H, α-H Tbg), 3.54 (dd, *J* = 8.5, 4.2 Hz, γ'-H Azl), 2.57 (m, β'-H Azl), 2.27 (m, 1H, β-H Azl), 1.78 (m, 4H, β-H Azl, γ-H Azl, 2-H), 1.45 (m, 2H, 3-H), 1.39 (m, 2H, 4-H), 1.32 (s, 9H, CH₃ N*^{t}*Bu), H, 1.29 (d, *J* = 7.1 Hz, 3H, β'-CH₃), 0.99 (s, 9H, γ-H Tbg), 0.95 (d, *J* = 6.1 Hz, 3H, δ-H Azl), 0.90 (m, 6H, δ-H Azl, 5-H). ¹H NMR (400 MHz, (CD₃)₂CO): δ 9.08 (d, *J=* 8.0 Hz, 1H, 1-NH), 5.51 (s, 1H, NH*^{t}*Bu), 5.42 (d, *J* = 9.1, 1H, α-NH Tbg ), 4.63 (dt, *J* = 8.0, 6.89 Hz, 1H, 1-H), 4.48 (t, *J* = 8.4 Hz, 1H, Hy' Azl), 3.84 (d, *J* = 9.1 1H, α-H Tbg), 3.44 (dd, *J* = 8.6, 4.3 Hz, γ'-H Azl), 2.44 (m, β'-H Azl), 2.07 (m, 1H, β-H Azl), 1.71 (m, 4H, β-H Azl, γ-H Azl, 2-H), 1.34 (m, 2H, 3-H), 1.25 (m, 2H, 4-H), 1.17 (s, 9H, γ-H Tbg), 1.10 (d, *J=* 7.1 Hz, 3H, β'-CH₃), 0.88 (s, 9H, γ-H Tbg), 0.80 (m, 9H, δ-H Azl, 5-H).¹³C NMR (100 MHz, CDCl₃): δ 175.0, 171.9 (CO), 157.0 (NHCONH), 118.6 (CN), 78.0 (Cα Azl), 56.3 (Cα Tbg), 54.9 (Cy' Azl), 50.9 (C N*^{t}*Bu), 44.8 (Cβ Azl), 40.0 (C1), 34,4 (Cβ Tbg), 34.3 (CP' Azl), 32.7 (C2), 29.5 (CH₃ N*^{t}*Bu), 27.5 (C3), 26.5 (Cγ Tbg), 24.73 (Cδ Azl), 24.70 (Cγ Azl), 23.2 (Cδ Azl), 22.2 (C4), 17.7 (y'-CH3), 13.9 (C5). MS (ES)⁺: 478.0 [M+H]⁺. Exact Mass calculated for C₂₆H₄₇N₅O₃: 477.3679; found: 477.3684.

### EXAMPLE 26

### Boc-L-β-Val-(αS,β'R)-Azf-NH-[(1S)-1-cyano-2-[(3S)-2-oxopyrrolidin-3-yl]ethane

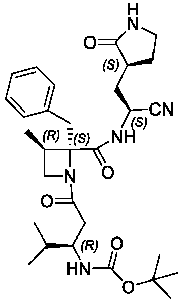

Syrup. Yield: 38% (method N, from intermediate **63**). Eluents: MeOH:DCM (1:20). HPLC: t_{R}= 7.20 min (gradient from 30 to 95% of A, in 10 min). [αD] = -10.74 (c 0.23, CHCl₃). ¹H NMR (400 MHz, CDCl₃): δ 8.85 (d, *J=* 7.0 Hz, 1H, 1-NH), 7.34-7.19 (m, 6H, Ph, α-NH β-Val), 6.00 (br s, 1H, 1'-H), 4.92 (m, 1H, 1-H), 3.70 (m, 1H, α-H β-Val), 3.61 (d, *J* = 13.9 Hz, 1H, β-H Azf), 3.40 (m, 2H, 5'-H), 3.13 (d, *J* = 13.9 Hz, 1H, β-H Azf), 3.11 (m 1H, γ'-H Azf), 3.02 (t, *J* = 8.1 Hz, 1H, γ'-H Azf), 2.58 (m. 2H, 3'-H, β'-H Azf), 2.39 (m, 1H, 2-H), 2.23-1.89 (m, 5H, β-H β-Val, γ-H β-Val, 2-H), 1.46 (s, 9H, (C(CH₃)₃), 1.18 (d, *J* = 7.1 Hz, 3H, β'-CH₃), 0.93 (d, *J* = 7.1 Hz, 3H, δ-H β-Val), 0.91 (d, *J =* 7.1 Hz, 3H, δ-H β-Val). ¹³C NMR (100 MHz, CDCl₃): δ 178.8, 173.4, 171.5 (CO), 156.2 (NHCOO), 135.7, 130.9, 128.5, 127.3 (Ar), 118.1 (CN). 79.6 (C(CH₃)₃), 77.4 (Cα Azf), 53.6 (C1), 53.5 (Cy' Azf), 40.5 (C5'), 39.8 (Cα β-Val), 38.9 (Cβ Azf), 38.1 (C3'), 34.8 (C2), 33.9 (Cβ β-Val), 31.7 (Cγ β-Val), 30.7 (Cβ' Azf), 28.7 (C(CH₃)₃), 28.4 (C4'), 19.4 (Cδ β-Val), 18.6 (Cδ β-Val), 16.4 (β'-CH₃). MS (ES)⁺: 554.2 [M+H]⁺. Exact Mass calculated for C₃₀H₄₃N₅O₅: 553.3264; found: 553.3266.

### EXAMPLE 27

### Z-L-Val-(αS,β'R)-Azl-L-Ala(3S-2-oxopyrrolidin-3-yl)-H

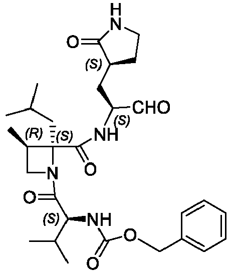

Syrup. Yield: 15% method P, from compound of example **1**). Eluents: MeOH:DCM (1:15). HPLC: t_{R}= 6.98 min (gradient from 10 to 95% of A, in 10 min). [αD] = -60.41 (c 0.10, CHCl₃). ¹H NMR (500 MHz, CDCl₃): δ 9.58 (s, 1H, CHO), 8.77 (d, *J* = 7.1 Hz, 1H, α-NH Ala), 7.34 (s, 5H, Ph), 5.46 (br s, 1H, 1 -H), 5.32 (d, *J* = 8.7 Hz, α-NH Val), 5.10 (s, 2H, OCH₂), 4.48 (m, 1H, α-H Val), 4.45 (t, *J* = 8.3 Hz, 1H, γ'-H Azl), 3.97 (dd, *J* = 9.2, 7.5 Hz, 1H, α-H Ala), 3.51 (m, 1H, γ'-H Azl), 3.34 (m, 2H, 5-H), 2.65 (m, 1H, β'-H Azl), 2.50-2.30 (m, 2H, 3-H, 4-H), 2.26 (dd, *J* = 14.0, 4.1, β-H Azl), 2.04 (m, 2H, β-H Ala), 1.99-1.73 (m, 4H, γ-H Azl, β-H Val, β-H Azl, 4-H), 1.28 (d, *J* = 7.1 Hz, 3H, β'-CH₃), 0.98 (d, *J* = 6.7 Hz, 3H, γ-H Val), 0.96 (d, *J* = 6.7 Hz, 3H, γ-H Val), 0.94 (d, *J* = 6.5 Hz, 3H, δ-H Azl), 0.92 (d, *J* = 6.5 Hz, 3H, δ-H Azl). ¹³C NMR (125 MHz, CDCl₃): δ 198.8 (CHO), 178.7, 173.1, 172.2 (CO), 156.1 (NHCOO), 136.2, 128.5, 128.2, 127.9 (Ar), 77.9 (Cα Azl), 67.0 (OCH₂), 57.1 (Cα Val), 56.1 (Cα Ala), 54.0 (Cy' Azl), 44.3 (Cβ Azl), 40.0 (C5), 37.6 (C3), 33.9 (Cβ' Azl), 30.9 (Cβ Val), 30.6 (Cβ Ala), 28.3 (C4), 24.8 (Cy- Azl), 24.7, 23.1 (Cδ Azl), 19.1, 18.0 (Cγ Val), 17.2 (β'-CH₃). MS (ES)⁺: 543.3 [M+H]⁺. Exact Mass calculated for C₂₉H₄₂N₄O₆: 542.3104; found: 542.3087.

### EXAMPLE 28

### Z-L-Val-(αS,β'R)-Azf-L-Ala(3S-2-oxopyrrolidin-3-yl)-H

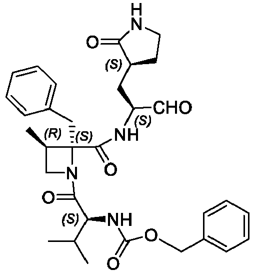

Syrup. Yield: 20% (method P, from intermediate **55**). Eluents: MeOH:DCM (1:9). HPLC: t_{R}= 7.82 min (gradient from 10 to 95% of A, in 10 min). [αD] = -60.98 (c 0.10, CHCl₃). ¹H NRM (400 MHz, CDCl₃): δ 9.60 (s, 1H, α-CHO Ala), 8.54 (d, *J* = 6.6 Hz, 1H, α-NH Ala), 7.42-7.31 (m, 5H, Ph), 7.20-7.10 (m, 5H, Ph), 5.75 (s, 1H, 1-H), 5.49 (d, *J* = 9.4 Hz, α-NH Val), 5.18 (d, *J* = 12.3 Hz, 1H, OCH₂), 5.12 (d, *J* = 12.3 Hz, 1H, OCH₂), 4.52 (m, 1H, α-H Ala), 3.90 (dd, *J* = 9.4, 6.6 Hz, 1H, α-H Val), 3.67 (d, *J=* 13.9 Hz, 1H, β-H Azf), 3.39-3.18 (m, 4H, 5-H, γ'-H Azf), 3.09 (d, *J* = 13.9 Hz, 1H, β-H Azf), 2.62 (m, 1H, β'-H Azf), 2.49-2.40 (m, 2H, 3-H, 4-H), 2.09-1.84 (m, 5H, β-H Val, β-H Ala, 4-H), 1.20 (d, *J* = 7.1 Hz, 3H, β'-CH₃), 0.97 (d, *J* = 6.7 Hz, 3H, γ-H Val), 0.93 (d, *J* = 6.8 Hz, 3H, γ-H Val). ¹³C (100 MHz, CDCl₃): δ 198.9 (CHO), 179.3, 173.6, 172.2 (CO), 156.2 (NHCOO), 136.6, 135.2, 130.8, 128.8, 128.6, 128.4, 128.2, 127.4 (Ar), 77.6 (Cα Azf), 67.1 (OCH₂), 57.7 (Cα Val), 56.2 (Cα Ala), 53.8 (Cy' Azf), 40.6 (C5), 39.2 (C3), 38.2 (Cβ Azf), 31.3 (Cβ Ala), 30.8 (Cβ Val), 30.7 (CP' Azf), 28.6 (C4), 19.4, 17.7 (Cγ Val), 16.6 (β'-CH₃). MS (ES)⁺: 577.4 [M+H]⁺. Mass calculated for C₃₂H₄₆N₄O₆: 576.2931; found: 576.2948.

### EXAMPLE 29

### Boc-L-Tbg-(αS,β'R')-Azf-L-Ala(3S-2-oxopyrrolidin-3-yl)-H

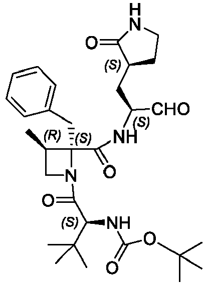

Syrup. Yield: 21% (method Q), 60% (method R, from intermediate **80**). Eluents: MeOH:DCM (1:20). HPLC: t_{R}= 8.54 min (gradient from 10 to 95% of A, in 10 min). [αD] = -85.63 (c 0.10, CHCl₃). ¹H NMR (400 MHz, CDCl₃): δ 9.62 (s, 1H, CHO), 8.54 (d, *J* = 6.9 Hz, 1H, α-NH Ala), 7.30-7.17 (m, 5H, Ph), 5.78 (br s, 1H, 1-H), 5.37 (d, *J* = 9.8 Hz, 1H, α-NH Tbg), 4.56 (m, 1H, α-H Ala), 3.83 (d, *J* = 9.8 Hz, 1H, α-H Tbg), 3.71 (d, *J* = 13.9 Hz, 1H, β-H Azf), 3.38-3.20 (m, 4H, 5-H, γ'-H Azf), 3.08 (d, *J* = 13.8 Hz, 1H, β-H Azf), 2.57 (m, 1H, 3-H), 2.48-2.39 (m, 2H, β'-H Azf, 4-H), 2.07 (m, 1H, β-H Ala), 1.97 (m, 1H, 4-H), 1.92 (m, 1H, β-H Ala), 1.49 (s, 9H, C(CH₃)₃), 1.21 (d, *J* = 7.1 Hz, 3H, β'-CH₃), 1.00 (s, 9H, γ-H Tbg). ¹³C (100 MHz, CDCl₃): δ 198.7 (CHO), 179.0, 173.5, 172.5 (CO), 155.4 (NHCOO), 135.2, 130.8, 128.6, 127.4 (Ar), 77.6 (Cα Azf), 57.5 (Cα Tbg), 57.0 (Cy' Azf), 54.5 (Cα Ala), 40.3 (C5), 39.3 (Cβ Azf), 37.8 (CP' Azf), 35.8 (Cβ Tbg), 30.8 (Cβ Ala), 30.6 (C3), 28.6 (C4), 28.5 (C(CH₃)₃), 26.4 (CγTbg), 16.9 (β'-CH₃). MS (ES)⁺: 557.4 [M+H]⁺. Exact Mass calculated for C₃₆H₄₄N₄O₆: 556.3261; found: 556.3253.

### EXAMPLE 30

### Methyloxycarbonyl-L-Tbg-(αS,β'R)-Azf-L-Ala(3S-2-oxopyrrolidin-3-yl)-CHO

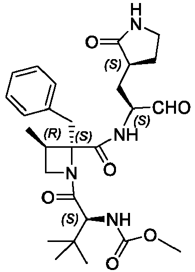

Syrup. Yield: 62% (method Q, from intermediate **81**). Eluents: MeOH:DCM (1:15). HPLC: t_{R}= 6.51 min (gradient from 10 to 95% of A, in 10 min). [αD] = -60.67 (c 0.12, CHCl₃). ¹H NMR (400 MHz, CDCl₃): δ 9.62 (s, 1H, CHO), 8.45 (d, *J* = 6.8 Hz, 1H, α-NH Ala), 7.28-7.12 (m, 5H, Ph), 6.62 (d, *J=* 9.1 Hz, 1H, α-NH Tbg), 5.66 (br s, 1H, 1-H), 4.58 (m, 1H, α-H Ala), 3.84 (d, *J* = 9.9 Hz, 1H, α-H Tbg), 3.75 (s, 3H, OCH₃), 3.71 (d, *J* = 13.8 Hz, 1H, β-H Azf),3.43-3.21 (m, 4H, 5-H, γ'-H Azf), 3.08 (d, *J* = 13.9 Hz, 1H, β-H Azf), 2.61 (m, 1H, 3-H), 2.50-2.34 (m, 2H, β'-H Azf, 4-H), 2.18 (m, 1H, β-H Ala), 2.00 (m, 1H, β-H Ala), 1.88 (m, 1H, 4-H), 1.22 (d, *J* = 7.1 Hz, 3H, β'-CH₃), 1.01 (s, 9H, γ-H Tbg). ¹³C (100 MHz, CDCl₃): δ 198.7 (CHO), 179.0, 173.5, 172.5 (CO), 155.4 (NHCOO), 135.2, 130.8, 128.6, 127.4 (Ar), 77.6 (Cα Azf), 57.5 (Cα Tbg), 57.0 (Cy' Azf), 54.5 (Cα Ala), 52.6 (OMe), 40.3 (C5), 39.3 (Cβ Azf), 37.8 (C3), 35.8 (Cβ Tbg), 30.8 (Cβ Ala), 30.6 (CP' Azf), 28.6 (C4), 28.5 (C(CH₃)₃), 26.4 (Cγ Tbg), 16.9 (β'-CH₃). MS (ES)⁺: 515.3 [M+H]⁺. Exact Mass calculated for C₂₇H₃₈N₄O₆: 514.2791; found: 514.2793.

### EXAMPLE 31

### 2,2-Difluoroethyloxycarbonyl-L-Tbg-(αS,β'R)-Azf-L-Ala(3S-2-oxopyrrolidin-3-yl)-H

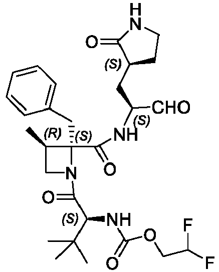

Syrup. Yield: 56 % (method R, from intermediate **82**). Eluents: MeOH:DCM (1:20). HPLC: t_{R}= 6.65 min (gradient from 15 to 95% of A, in 10 min). [αD] = -26.03 (c 0.10, CHCl₃). ¹H NMR (400 MHz, CDCl₃): δ 9.62 (s, 1H, CHO), 8.42 (d, *J* = 6.7 Hz, α-NH Ala), 7.26-7.15 (m, 5H, Ph), 5.99 (tt, *J* = 55.1, 4.0 Hz, CHF₂), 5.75 (brs, 1H, 5-H), 5.70 (d, *J* = 9.8 Hz, 1H, α-NH Tbg), 4.58 (m, 1H, α-H Ala), 4.31 (m, 2H, OCH₂),3.82 (d, *J* = 9.8 Hz, 1H, α-H Tbg), 3.71 (d, *J* = 13.9 Hz, 1H, β-H Azf), 3.35-3.27 (m, 4H, 5-H, γ'-H Azf), 3.09 (d, *J* = 13.9 Hz, 1H, β-H Azf), 2.64 (m, 1H, 3-H), 2.49-2.41 (m, 2H, β'-H Azf, 4-H), 2.00 (m, 2H, β-H Ala), 1.89 (m, 1H, 4-H), 1.22 (d, *J* = 7.1 Hz, 3H, β'-CH₃), 1.01 (s, 9H, γ-H Tbg).¹³C NMR (100 MHz, CDCl₃): δ 198.5, 179.2, 172.6, 172.1 (CO), 154.9 (NHCOO), 135.1, 130.9, 128.5, 127.5 (Ar), 113.10 (t, *J* = 241.3 Hz, CHF₂), 77.4 (Cα Azf), 63.5 (t, *J* = 29.3 Hz, OCH₂), 57.8 (Cα Tbg), 57.6 (Cα Ala), 54.5 (Cy' Azf), 40.4 (C5), 39.2 ( C3), 37.9 (Cβ Azf), 35.8 (Cβ Tbg), 30.8 (Cβ Ala), 30.5 (Cβ' Azf), 28.6 (C4), 26.3 (Cγ Tbg), 16.7 (β'-CH₃). MS (ES)⁺: 565.2 [M+H]⁺. Exact Mass calculated for C₂₈H_{38F2}N₄O₆: 564,2759; found: 564.2749.

### EXAMPLE 32

### Boc-L-Tbg-(αS,β'R)-Azf-NH-[(3S)-(1-benzyloxyrcarbonyl-)-4-[(3S)-2'-oxopyrrolidin-3'-yl-1,2-E-butene

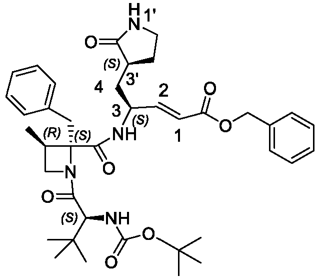

A solution of the compound of example **29** (0.027mmol) in dry THF (0.5 mL) was added, under Ar atmosphere, benzyl(triphenylphosphoranylidene)acetate (33 mg, 0.081 mmol). After 2 h of reaction at rt, the solvent was evaporated and purified on centrifugal circular chromatography using silica gel and the solvent system specified. Syrup. Yield: 60%. Eluents: MeOH:DCM (1:20). HPLC: t_{R}= 9.44 min (gradient from 30 to 95% of A, in 10 min). [αD] = -54.62 (c 0.10, CHCl₃). ¹H NMR (400 MHz, CDCl₃): δ 7.99 (d, *J* = 8.9 Hz, 1H, 3-NH), 7.38-7.15 (m, 10H, Ph), 6.96 (dd, *J* = 15.7, 5.0 Hz, 1H, 2-H), 6.17 (dd, *J* = 15.7, 1.7 Hz, 1H, 1-H), 5.59 (br s, 1H, 1'-H), 5.32 (d, *J* = 9.9 Hz, 1H, α-NH Tbg), 5.20 (s, 2H, OCH₂), 4.78 (m, 1H, 3-H), 3.83 (d, *J* = 9.8 Hz, 1H, α-H Tbg), 3.64 (d, *J* = 13.8 Hz, 1H, β-H Azf), 3.37-3.27 (m, 3H, 5'-H, γ'-H Azf), 3.19 (dd, *J* = 8.3, 4.0 Hz, 1H, γ'-H Azf), 3.08 (d, *J* = 13.9 Hz, 1H, β-H Azf), 2.53 (m, 1H, H3'), 2.46-2.33 (m, 2H, P'-HAzf, H4'), 2.06 (m, 1H, 4-H), 1.84 (m, 1H, 4'-H4), 1.62 (m, 1H, 4-H), 1.50 (s, 9H, C(CH₃)₃), 1.21 (d, *J* = 7.1 Hz, 3H, β'-CH₃), 0.97 (s, 9H,γ-H Tbg). ¹³C NMR (100 MHz, CDCl₃): δ 179.1, 173.8, 171.6, 166.1 (CO), 155.4 (COO), 147.6 (C2), 136.1, 135.2, 130.8, 128.7, 128.6,128.4, 128.3, 127.4 (Ar), 121.3 (C1), 79.9 (C(CH₃)₃), 78.2 (Cα Azf), 66.5 (OCH₂), 56.9 (Cα Tbg), 54.5 (Cy' Azf), 48.3 (C3), 40.4 (C5'), 39.7 (C3'), 38.3 (Cβ Azf), 36.3 (Cβ Tbg), 35.7 (C4), 30.8 (CP' Azf ), 28.6 (C(CH₃)₃), 28.4 (C4'), 26.4 (Cγ Tbg), 17.1 (β'-CH₃). MS (ES)⁺: 689.5 [M+H]⁺. Exact Mass calculated for C₃₉H₅₂N₄O₇: 688.3836; found: 688.3826.

### EXAMPLE 33

### 2,2-Difluoroethyloxycarbonyl-L-Tbg-(αS,β'R)-Azl-NH-[(1S)-1-cyano-2-[(3S)-2-oxopyrrolidin-3-yl]ethane

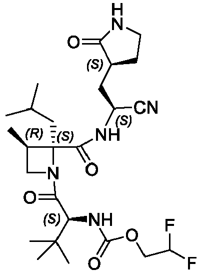

Syrup. Yield: 74% (method L, from **76**). Eluents: MeOH:DCM (1:30). HPLC: t_{R}= 7.90 min (gradient from 15% to 95% of A, in 10 min). [αD] = -138.41 (c 0.23, CHCl₃). ¹H NMR (400 MHz, CDCl₃): δ 9.01 (d, *J* = 7.6 Hz, 1H, 1-NH), 5.92 (tt, *J* = 55.1, 4.0 Hz, 1H, CHF₂), 5.88 (br s, 1H, 1'-H), 5.47 (d, *J* = 9.5 Hz, 1H, α-NH Tbg), 4.99 (q, *J* = 7.8 Hz, 1H, 1-H), 4.43 (t, *J* = 8.4 Hz, 1H, γ'-H Azl), 4.26 (td, *J* = 13.6, 4.1 Hz, 1H, OCH₂), 3.91 (d, *J* = 9.6 Hz, 1H, α-H Tbg), 3.51 (dd, *J* = 8.4, 4.5 Hz, 1H, γ'-H Azl), 3.36 (m, 2H, 5'-H), 2.61 (m, 1H, β'-H Azl), 2.53-2.43 (m, 2H, 3'-H, 4'-H), 2.28-2.21 (m, 2H, β-H Azl, 2-H), 1.91 (m, 1H, 2-H), 1.83-1.74 (m, 2H, β-H Azl, γ-H Azl), 1.30 (d, *J* = 7.0 Hz, 3H, β'-CH₃), 1.01 (s, 9H, γ-H Tbg), 0.95 (d, *J* = 6.3 Hz, 3H, δ-H Azl), 0.94 (d, *J* = 6.3 Hz, 3H, δ-H Azl). ¹³C NMR (100MHz, CDCl₃): δ 178.1, 172.9, 171.6 (CO), 155.4 (NHCOO), 118.3 (CN), 113.0 (t, *J* = 241.4 Hz, CHF₂), 77.8 (Cα Azl), 63.4 (t, *J* = 29.5 Hz, OCH₂), 57.7 (Cα Tbg), 54.9 (Cy' Azl), 44.4 (Cβ Azl), 40.4 (C5'), 39.3 (C1), 37.8 (C3'), 34.9 (Cβ Tbg), 34.5 (C2), 34.2 (CP'Aza), 28.4 (C4'), 26.4 (Cγ Tbg), 26.75 (Cγ Azl), 26.75 (Cδ Azl), 23.1 (Cδ Azl), 17.4 (β'-CH₃). MS (ES)⁺: 528.2[M+H]⁺. Exact Mass calculated for C₂₅H₃₉F₂N₅O₅: 527.2919; found: 527.2918.

### EXAMPLE 34

### Boc-L-Tbg-(aS,β'R)-Azl-NH-[(1S)-1-cyano]pentane

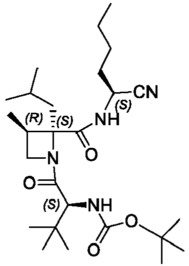

Syrup. Yield: 81% (method L, from **60**). Eluents: MeOH:DCM (1:50). HPLC: t_{R}= 6.61 min (gradient from 60 to 95% of A, in 10 min). [αD] = -144.63 (c 0.94, CHCl₃). ¹H NMR (400 MHz, CDCl₃): δ 9.18 (d, *J* = 8.0 Hz, 1H, 1-NH), 5.07 (d, *J* = 9.8 Hz, 1H, α-NH Tbg), 4.69 (q, *J* = 7.2 Hz, 1H, 1-H), 4.52 (t, *J* = 8.3 Hz, 1H, γ'-H Azl), 3.86 (d, *J* = 9.8 Hz, 1H, α-H Tbg), 3.44 (dd, *J* = 8.3, 4.2 Hz, 1H, γ'-H Azl), 2.57 (m, 1H, β'-H Azl), 2.26 (q, *J* = 8.0 Hz, 1H, β-H Azl), 1.84-1.71 (m, 4H, β-H Azl, 2-H, γ-H Azl), 1.49-1.44 (m, 5H, 2-H, 3-H, 4-H), 1.43 (s, 9H, C(CH₃)), 1.35 (m, 1H, 4-H), 1.30 (d, *J* = 7.1 Hz, 3H, β'-CH₃), 0.99 (s, 9H, γ-H Tbg), 0.95 (d, *J* = 6.1 Hz, 3H, δ-H Azl,) 0.93-0.88 (m, 9H, δ-H Azl, 5-H). ¹³C NMR (100 MHz, CDCl₃): δ 174.1, 171.9 (CO), 156.0 (NHCOO), 118.5 (CN), 80.2 C(CH₃)), 78.0 (Cα Azl), 57.0 (Cα Tbg), 54.8 (Cy' Azl), 44.8 (Cβ Azl), 40.1 (C1), 34.4 (Cβ Tbg), 34.3 (CP' Azl), 32.6 (C2), 28.4 (C(CH₃)), 27.5 (C3), 26.4 (Cγ Tbg), 24.73 (Cδ Azl), 24.70 (Cγ Azl), 23.2 (Cδ Azl), 22.1 (C4), 17.6 (β'-CH₃), 13.9 (C5). MS (ES)⁺: 479.3 [M+H]⁺. Exact Mass calculated for C₂₆H₄₆N₄O₄:478.3519; found: 478.3496

### EXAMPLE 35

### Boc-L-Tbg-(αS,β'R)-Aza(Cy)-NH-[(1S)-1-cyano-2-[(3'S)-2'-oxopyrrolidin-3'-yl]ethane

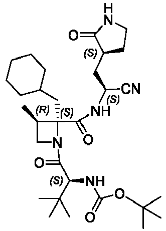

Syrup. Yield: 94% (method L, from **62**). Eluents: MeOH:DCM (1:20). HPLC: t_{R}= 8.62 min (gradient from 30 to 95% of A, in 10 min). [αD] = -111.67 (c 1.25, CHCl₃). ¹H NMR (400 MHz, CDCl₃): δ 9.18 (d, *J* = 7.9 Hz, 1H, 1-NH), 5.70 (br s, 1H, 1'-H), 5.07 (d, *J* = 9.7 Hz, 1H, α-NH Tbg), 4.99 (q, *J* = 8.0 Hz, 1H, 1-H), 4.49 (t, *J* = 8.3 Hz, 1H, γ'-H Aza) 3.86 (d, *J* = 9.7 Hz, 1H, α-H Tbg), 3.46 (dd, *J* = 8.4, 4.3 Hz, 1H, γ'-H Aza), 3.35 (m, 2H, 5'-H), 2.55 (m, 1H, β'-H Aza), 2.49-2.42 (m, 2H, 3'-H, 4'-H), 2.27 (m, 1H, β-H Ala), 2.20 (dd, *J* = 14.2, 4.4 Hz, 1H, β'-H Aza), 1.94-1.74 (m, 3H, β'-H Aza, β-H Ala, 4'-H), 1.74-1.52 (m, 5H, 2, 3, 4, 5, 6-H Cy), 1.47 (m, 1H, 1-H Cy), 1.44 (s, 9H, C(CH₃)), 1.29 (d, *J* = 7.1 Hz, 3H, β'-CH₃), 1.25-1.07 (m, 3H, 3, 4, 5-H Cy), 0.99 (s, 9H, γ-H Tbg), 0.96-0.80 (m, 2H 2, 6-H Cy). ¹³C NMR (100 MHz, CDCl₃): δ 178.1, 174.0, 171.8 (CO), 156.0 (NHCOO), 118.3 (CN), 80.2 (C(CH₃)), 77.7 (Cα Aza), 57.2 (Cα Tbg), 54.8 (Cγ' Aza), 43.4 (Cβ Aza), 40.3 (C5'), 39.2 (C1), 37.8 (C3'), 34.9 (C2-, C6- Cy), 34.6 (C2), 34.5 (Cβ'Aza), 34.4 (C2-, C6- Cy), 34.1 (Cβ Tbg), 33.8 (C1 Cy), 28.45 (C4'), 28.4 (C(CH₃)), 26.5 (Cγ Tbg), 26.4, 26.35, 26.3 (C3-, C4-, C5- Cy), 17.5 (β'-CH₃). MS (ES)⁺: 560.3 [M+H]⁺. Exact Mass calculated for C₃₀H₄₉N₅O₅. 559.3734; found: 559.3715.

### EXAMPLE 36

### Boc-L-Val-(αS,β'R)-Aza(Cy)-NH-[(1S)-1-cyano-2-[(3'S)-2'-oxopyrrolidin-3'-yl]ethane

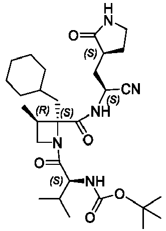

Syrup. Yield: 68% (method L, from **61**). Eluents: MeOH:DCM (1:20). HPLC: t_{R}= 8.17 min (gradient from 30 to 95% of A, in 10 min). [αD] = -69.30 (c 0.21, CHCl₃). ¹H NMR (400 MHz, CDCl₃): δ 9.15 (d, *J* = 7.9 Hz, 1H, 1-NH), 5.76 (br s, 1H, 1'-H), 5.02-4.95 (m, 2H, α-NH Val, 1-H), 4.48 (t, *J*= 8.3 Hz, 1H, γ'-H Aza), 3.83 (t, *J* = 8.5 Hz, 1H, α-H Val), 3.44 (dd, *J* = 8.4, 4.4 Hz, 1H, γ'-H Aza), 3.35 (m, 2H, 5'-H), 2.58 (m, 1H, β'-H Aza), 2.52-2.40 (m, 2H, 3'-H, 4'-H), 2.23 (m, 1H, β'-H Aza), 2.19 (dd, *J* = 14.2, 4.3 Hz, 1H, β-H Ala), 1.93-1.76 (m, 3H, β'-H Aza, β-H Ala, β-H Val, 4'-H), 1.70-1.54 (m, 5H, 2, 3, 4, 5, 6-H Cy), 1.49 (m, 1H, 1-H Cy). 1.47 (s, 9H, C(CH₃)), 1.28 (d, *J* = 7.2 Hz, 3H, β'-CH₃), 1.25-1.07 (m, 3H, 3, 4, 5-H Cy), 0.96 (d, *J* = 7.0 Hz, 3H, γ-H Val), 0.94 (d, *J=* 7.1 Hz, 3H, γ-H Val), 0.92-0.76 (m, 2H 2, 6-H Cy). ¹³C NMR (100 MHz, CDCl₃): δ 178.1, 174.3, 171.7 (CO), 155.9 (NHCOO), 118.3 (CN), 80.1 (C(CH₃)), 77.8 (Cα Aza), 56.0 (Cα Val), 54.2 (Cy' Aza), 43.9 (Cβ Aza), 40.3 (C5'), 39.2 (C1), 37.8 (C3'), 34.9 (C2), 34.7 (C2-, C6- Cy), 34.6 (CP'Aza), 34.0 (C2-, C6- Cy), 33.9 (C1 Cy), 30.8 (Cβ Val), 28.45 (C4'), 28.4 (C(CH₃)), 26.4, 26.3, 26.2 (C3-, C4-, C5- Cy), 19.3, 18.3 (Cγ Val), 16.3 (β'-CH₃). MS (ES)⁺: 546.3 [M+H]⁺. Exact Mass calculated for C₂₉H₄₇N₅O₅: 545.3577; found: 545.3574.

### EXAMPLE 37

### 2,2-Difluoroethyloxycarbonyl-L-Tbg-(αS,β'R)-Aza(Cy)-NH-[(1S)-1-cyano-2-[(3S)-2-oxopyrrolidin-3-yl]ethane

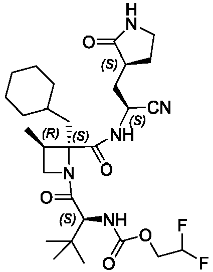

Syrup. Yield: 68% (method L, from **78**). Eluents: MeOH:DCM (1:20). HPLC: t_{R}= 7.37 min (gradient from 30% to 95% of A, in 10 min). [αD] =-105.35 (c 0.22, CHCl₃). ¹H NMR (400 MHz, CDCl₃): δ 8.96 (d, *J* = 7.7 Hz, 1H, 1-NH), 5.97 (tt, *J* = 55.1, 4.0 Hz, 1H, CHF₂), 5.75 (br s, 1H, 1'-H ), 5.45 (d, *J=* 9.6 Hz, 1H, α-NH Tbg), 4.99 (q, *J=* 7.9 Hz, 1H, 1-H), 4.42 (t, *J* = 8.3 Hz, 1H, γ'-H Aza), 4.26 (qd, *J* = 13.8, 4.1 Hz, 2H, OCH₂), 3.92 (d, *J* = 9.6 Hz, 1H, α-H Tbg), 3.50 (dd, *J* = 8.4, 4.5 Hz, 1H, γ'-H Aza), 3.35 (m, 2H, 5'-H), 2.59 (m, 1H, β'-H Aza), 2.52-2.42 (m, 2H, 3'-H, 4'-H), 2.26 (ddd, *J* = 13.5, 7.8, 5.5 Hz, 1H, 2-H), 2.14 (dd, *J* = 14.3, 4.4 Hz, 1H, β-H Aza), 1.95-1.81 (m 2H, 2-H, β-H Aza), 1.77-1.54 (m, 5H, 2, 3, 4, 5, 6-H Cy), 1.47 (m 1H, 1-H Cy), 1.29 (d, *J* = 7.1 Hz, 3H, β-CH₃), 1.25-1.03 (m, 3H, 3, 4, 5-H Cy), 1.01 (s, 9H, γ-H Tbg), 0.99-0.90 (m, 2H, 2, 6-H Cy). ¹³C NMR (100MHz, CDCl₃): δ 178.1, 172.8, 171.7 (CO), 155.3 (NHCOO), 118.3 (CN), 113.0 (t, *J* = 241.4 Hz, CHF₂), 77.7 (Cα Aza), 63.4 (t, *J* = 29.5 Hz, OCH₂), 57.8 (Cα Tbg), 54.9 (Cy' Aza), 43.2 (Cβ Aza), 40.4 (C5'), 39.3 (C1), 39.70 (Cβ Aza), 37.8 ( C3'), 35.05, 35.0 (C2-, C6- Cy), 34.15 (C2), 34.1 (CP'Aza), 34.1 (Cβ Tbg), 33.8 (C1 Cy), 28.4 (C4'), 26.45 (Cγ Tbg), 26.4, 26.3, 26.2 (C3-, C4-, C5- Cy), 17.4 (β'-CH₃). MS (ES)⁺: 568.3[M+H]⁺. Exact Mass calculated for C₂₈H₄₃F₂N₅O₅: 567.3232; found: 567.3210.

### EXAMPLE 38

### Trifluoroacetyl-L-Tbg-(αS,β'R)-Aza(Cy)-NH-[(1S)-1-cyano-2-[(3S)-2-oxopyrrolidin-3-yl]ethane

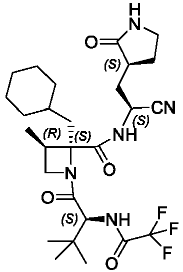

Syrup. Yield: 35% (method L, from **77**). Eluents: MeOH:DCM (1:20). HPLC: t_{R}= 7.98 min (gradient from 30% to 95% of A, in 10 min). [αD] =-91.42 (c 0.20, CHCl₃). ¹H NMR (500 MHz, CDCl₃): δ 8.77 (d, *J* = 7.5 Hz, 1H, 1-NH), 6.84 (d, *J* = 9.4 Hz, 1H, α-NH Tbg), 5.63 (br s, 1H, 1'-H), 4.96 (q, *J* = 7.9 Hz, 1H, 1-H), 4.40 (t, *J* = 8.4 Hz, 1H, γ'-H Aza), 4.27 (d, *J* = 9.4 Hz, 1H, α-H Tbg), 3.57 (dd, *J* = 8.4, 4.8 Hz, 1H, γ'-H Aza), 3.37 (m, 2H, 5'-H), 2.65 (m, 1H, β'-H Aza), 2.52-2.43 (m, 2H, 3'-H, 4'-H), 2.27 (m, 1H, 2-H), 2.06 (dd, *J* = 14.4, 4.4 Hz, 1H, β-H Aza), 1.97-1.84 (m, 3H, 2-H, β-H Aza, 4'-H), 1.74-1.49 (m, 5H, 2, 3, 4, 5, 6-H Cy), 1.46 (m, 1H, 1-H Cy), 1.28 (d, *J* = 7.2 Hz, 3H, β'-CH₃), 1.23-1.09 (m, 3H, 3, 4, 5-H Cy), 1.04 (s, 9H, γ-H Tbg), 1.00-0.86 (m, 2H 2, 6-H Cy). ¹³C NMR (125 MHz, CDCl₃): δ 178.1, 171.4, 171.0 (CO), 157.1 (q, *J* = 38.0 Hz, COCF₃), 118.2 (CN), 115.9 (q, *J* = 288.0 Hz, CF₃), 77.8 (Cα Aza), 56.0 (Cα Tbg), 55.0 (Cy' Aza), 42.7 (Cβ Aza), 40.4 (C5'), 39.5 (C1) 37.9 (C3'), 35.6 (Cβ Tbg), 35.2, 34.4 (C2-, C6- Cy), 34.1 (C2), 33.9 (CP'Aza), 33.8 (C1 Cy), 28.5 (C4'), 26.4 (Cγ Tbg), 26.4, 26.3, 26.2 (C3-, C4-, C5- Cy), 17.1 (β'-CH₃). MS (ES)⁺: 556.3 [M+H]⁺. Exact Mass calculated for C₂₇H₄₆F₃N₅O₄: 555.3032; found: 555.3031.

### EXAMPLE 39

### 2,2-Difluoroethyloxycarbonyl-L-Val-(αS,β'R)-Azf-NH-[(1S)-1-cyano-2-[(3S)-2-oxopyrrolidin-3-yl]ethane

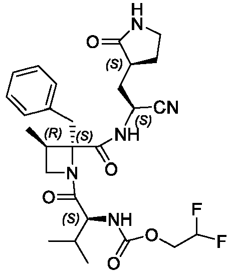

Syrup. Yield: 67% (method N, from **79**). Eluents: MeOH:DCM (1:20). HPLC: t_{R}= 7.58 min (gradient from 15 to 95% of A, in 10 min). [αD] = -85.54 (c 1.00, CHCl₃). ¹H NMR (400 MHz, CDCl₃): δ 8.78 (d, *J=* 7.4 Hz, 1H, 1-NH), 7.27-7.13 (m, 5H, Ph), 6.24 (br s, 1H, 1'-H),5.98 (tt, *J* = 55.1, 4.0 Hz, 1H, CHF₂), 5.67 (d, *J* = 9.4 Hz, 1H, α-NH Val), 4.98 (q, *J* = 7.8 Hz, 1H, 1-H), 4.30 (m, 2H, OCH₂), 3.82 (dd, *J* = 9.4, 7.1 Hz, 1H, α-H Val), 3.58 (d, *J* = 13.9 Hz, 1H, β-H Azf), 3.40-3.30 (m, 3H, 5'-H, γ'-H Azf), 3.24 (dd, *J* = 8.1, 4.3 Hz, 1H, γ'-H Azf), 3.07 (d, *J* = 13.9 Hz, 1H, β-H Azf), 2.65 (m, 1H, 3'-H), 2.59-2.42 (m, 2H, β'-H Azf, 4'-H), 2.28 (m, 1H, 2-H), 1.99-1.83 (m, 3H, β-H Val, 2-H, 4'-H), 1.22 (d, *J* = 7.0 Hz, 3H, β'-CH₃), 0.95 (d, *J* = 6.7 Hz, 3H, β-H Val), 0.93 (d, *J* = 6.7 Hz, 3H, β-H Val). ¹³C NMR (100MHz, CDCl₃): δ 178.6, 173.4, 171.5 (CO), 155.1 (NHCOO), 134.7, 130.7, 128.5, 127.6 (Ar), 118.2 (CN), 113.1 (t, *J* = 241.3 Hz, CHF₂), 77.6 (Cα Azf), 63.4 (t, *J=* 29.3 Hz, OCH₂), 56.4 (Cα Val), 53.9 (Cγ' Azf), 40.6 (C5'), 39.7 (Cβ Azf), 39.6 (C1), 38.1 (C3'), 34.2 (C2), 31.4 (CP' Azf), 31.1 (Cβ Val), 28.4 (C4'), 19.2, 17.8 (Cγ Val), 16.6 (β'-CH₃). MS (ES)⁺: 548.4 [M+H]⁺. Exact Mass calculated for C₂₇H₃₅F₂N₅O₅: 547.2606; found: 547.2607.

### EXAMPLE 40

### In vitro evaluation for the inhibition of the isolated SARS-CoV-2 M^{pro} protein

Compounds of the present patent have been evaluated in vitro for the inhibion of the isolated SARS-CoV-2 M^{pro} protein. The screening for the inhibitory concentration fifty (IC₅₀) of each compound against 3CL protease, 3CL Protease Untagged (SARS-CoV-2) Assay Kit (#78042-1, BPS Bioscience, San Diego, CA, USA) was used, according to manufacturer's instructions. Briefly, stock solutions at different concentration of new peptidomimetics were prepared using Assay buffer (20 mM Tris, 100 mM NaCl, 1 mM EDTA, and 1 mM DTT, pH 7.3). The final concentration of DMSO in this assay did not exceed 1%. 30 µL of diluted SARSCoV-2 3CL protease, at the final concentration of 15 ng was added to each well except "Blanks". GC376, a well known 3CL protease inhibitor, was used as inhibitor control. After adding 10 µL test inhibitor at different concentrations to each well, the mixtures were preincubate for 30 min at room temperature with slow shaking. Afterward, the reaction was started by adding the substrate (10 µL, 200 pM), dissolved in the reaction buffer to 50 µL final volume, at a concentration of 40 µM. Then the plates were sealed and incubated for 4 h at room temperature with slow shaking. Fluorescence intensity (Fi) was measured with a CLARIO star Plus, exciting at a wavelength of 360 nm and detecting at a wavelength of 460 nm. Each sample was assayed in three separate experiments performed in duplicate.
Known M^{pro} inhibitor (GC-376) and the antiviral nirmatrelvir (PF-07321332) have been used for obtaining comparative in vitro biological data between them and the compounds of the invention.

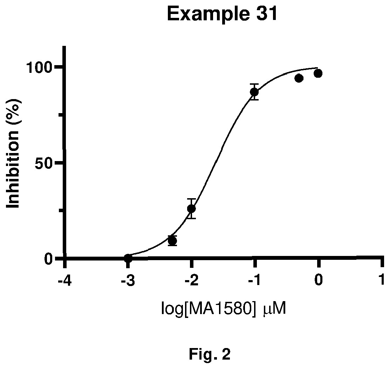

Figures 1 and 2 depicted isolated enzyme M^{pro} inhibition by two representative examples: example 18 and Example 31, respectively.

In vitro biological data of the compounds of the invention are summarized on following tables (table 1: assay in isolated SARS-CoV-2 M^{pro}; table 2: inhibition of proliferation of SARS-CoV2 in A549-ACE2 cells; and table 3: inhibition of proliferation of VSV; table 4: inhibition of proliferation of HCV).

**Table 1:**

| **EXAMPLE** | **IC₅₀, mean±SD, µM or (% of inhibition at 10 µM)** |
|---|---|
| **1** | 1.99 ± 1.00 |
| **2** | (0%) |
| **3** | 2.58 ± 0.48 |
| **4** | (0%) |
| **5** | 0.13 ± 0.03 |
| **6** | 0.07± 0.01 |
| **7** | 0.20 ± 0.03 |
| **8** | 0.39 ± 0.05 |
| **9** | 6.43± 0.53 |
| **10** | (9%) |
| **11** | 4.74±0.81 |
| **12** | 4.70±0.76 |
| **13** | 0.31±0,04 |
| **14** | 0.30± 0.03 |
| **15** | 2.40±0,93 |
| **16** | 0.94±0.17 |
| **17** | 1,03±0,03 |
| **18** | 0.54±0,12 |
| **19** | 0.51±0,09 |
| **20** | 0.42±0,08 |
| **21** | 3,66±1,59 |
| **22** | (16%) |
| **23** | (0%) |
| **24** | (10%) |
| **25** | (0%) |
| **26** | (28%) |
| **27** | 0.077±0.02 |
| **28** | 0.005± 0.002 |
| **29** | 0.027±0.006 |
| **30** | 0.048±0.004 |
| **31** | 0.024±0.002 |
| **32** | (40%) |
| **33** | 5.94 ± 0.69 |
| **34** | (0%) |
| **35** | 1,05 ± 0.12 |
| **36** | 0.19 ± 0.01 |
| **37** | 1,22 ± 0.08 |
| **38** | 2.36 ± 0.75 |
| **GC376** | 0.032 ± 0.02 |
| **PF-07321332** | 0.0037 ± 0.0008 |

### EXAMPLE 41

### Evaluation of the ability of compounds to inhibit SARS-CoV-2 replication.

Evaluation of the ability of compounds to inhibit SARS-CoV-2 replication in A549-ACE2 cell cultures was also measured. A549-ACE2 cells were seeded onto 96-well plates (2×10⁴ cells/well). Compounds were diluted from stock solutions in complete media (DMEM) supplemented with 10 mM HEPES, 1X non-essential amino acids (gibco), 100 U/ml penicillin-streptomycin (GIBCO) and 2 % fetal bovine serum (heat-inactivated at 56 °C for 30 min)) to achieve the desired concentration. SARS-CoV-2 stock strain NL/2020 (kindly provided by Dr. R. Molenkamp, Erasmus University Medical Center Rotterdam) was diluted to achieve a multiplicity of infection (MOI) of 0.005. A549-ACE2 cell monolayers were inoculated at MOI 0.005 in the presence of a wide range of compound concentrations from 50 to 0.78 µM. Forty-eight hours later cells were fixed by 20 minutes' incubation with a 4 % formaldehyde-PBS solution. After extensive washes with PBS, infection efficiency was evaluated by the detection of intracellular SARS-CoV-2 N protein accumulation by immunofluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated (DMSO) conditions was used as reference (100 %). EC₅₀ and EC₉₀ values were calculated from normalized data and are shown in Table 2.

To evaluate the cytotoxicity profile of the compounds MTT assays were performed in A549-ACE2 cells. Uninfected cells were incubated with a range of compound concentrations (from 100 to 1 µM) for forty-eight hours, time at which the MTT substrate [3-(4,5-dimethylthiazol-2-yl)-2,5-diphenyltetrazolium bromide] was added and the formation of formazan precipitates was evaluated two hours later, using a colorimetric readout. Vehicle-treated (DMSO) cells were used as reference (100 %). CC₅₀ values were calculated from normalized data and are shown in Table 2.

**Table 2:**

| **Example** | **EC₅₀ (µM)** | **EC₉₀ (µM)** | **CC₅₀ (µM) MTT** |
|---|---|---|---|
| **1** | 5 | 20 | >100 |
| **3** | 10 | 32 | 100 |
| **5** | 1 | 6 | >100 |
| **6** | 1 | 3 | >100 |
| **7** | 2 | 12 | >100 |
| **8** | 8 | 31 | >100 |
| **11** | 31 | 48 | >100 |
| **12** | 29 | >50 | 100 |
| **13** | 4 | 15 | >100 |
| **14** | 1 | 3 | >100 |
| **15** | 2 | 13 | >100 |
| **16** | 3 | 20 | >100 |
| **17** | 3 | 18 | 100 |
| **18** | 4 | 7 | >100 |
| **20** | 7 | 18 | 100 |
| **24** | 18 | 40 | >100 |
| **27** | 1 | 5 | >100 |
| **28** | 2 | 12 | >100 |
| **29** | 1 | 2 | >100 |
| **30** | 15 | 25 | >100 |
| **31** | 3 | 11 | >100 |
| **33** | 16 | >25 | >100 |
| **35** | 3 | 20 | >100 |
| **36** | 2 | 4 | >100 |
| **37** | 4 | 22 | >100 |
| **38** | 25 | >25 | >100 |
| **39** | 0,4 | 2 | >50 |
| **PF-07321332** | <0.2 | <0.2 | 100 |

### EXAMPLE 42

### Analysis of the effect on VSV infection

To characterize the antiviral spectrum of selected compounds of the family, A549-ACE2 cell monolayers were infected with a GFP-expressing recombinant vesicular stomatitis virus (VSV-GFP) at MOI 0.01 in the presence of a wide range of compound concentrations (50 to 0.4 µM). Brequinar, a DHODH inhibitor with broad-spectrum antiviral activity was used as positive inhibition control. Sixteen hours later cells were fixed by 20 minutes' incubation with a 4 % formaldehyde-PBS solution. After extensive washes with PBS, infection efficiency was evaluated by the detection of GFP accumulation by fluorescence microscopy. Nuclei were stained using DAPI (4',6-diamidino-2-phenylindole) dye. Automated imaging was carried out in a SparkCyto (Tecan) multimode plate reader. The total intensity signal present in vehicle-treated (DMSO) conditions was used as reference (100 %). EC₅₀ and EC₉₀ values were calculated from normalized data and are shown in Table 3. Results showed that compound concentrations that inhibit SARS-CoV-2 propagation are not able to control VSV-GFP infection suggesting that compounds display a selective antiviral activity towards SARS-CoV-2.

**Table 3:**

| Example | EC₅₀ (µM) | EC₉₀ (µM) | CC₅₀ (µM) |
|---|---|---|---|
| 6 | >50 | >50 | >50 |
| 14 | >50 | >50 | >50 |
| 25 | >50 | >50 | >50 |
| 27 | >50 | >50 | >50 |
| 29 | >50 | >50 | >50 |
| 27 | >50 | >50 | >50 |
| 36 | >50 | >50 | >50 |
| Brequinar | <0.6 | <0.6 | >50 |

### EXAMPLE 43

### Analysis of the effect on HCV infection

To further characterize the antiviral spectrum of the compounds, we tested their ability to interfere with hepatitis C virus infection, using a trans-complemented infectious particle reporter system (HCVtcp), which mimics a single cycle infection experiment and enables evaluating overall infection efficiency using a luciferase readout (10.1128/JVI.00118-08). Glecaprevir, a clinically approved HCV NS3/4A protease inhibitor was used as control to testify for the ability of the assay to reveal viral protease inhibitors.

**Table 4**

| Example | EC₅₀ (µM) | EC₉₀ (µM) | CC₅₀ (µM) |
|---|---|---|---|
| **6** | >50 | >50 | >50 |
| **14** | >50 | >50 | >50 |
| **25** | 42 | >50 | >50 |
| **29** | >50 | >50 | >50 |
| **34** | 45 | >50 | >50 |
| **36** | 42 | >50 | >50 |
| **Glecaprevir** | <0.06 | <0.06 | >50 |

## Claims

1. A compound of formula **I:** 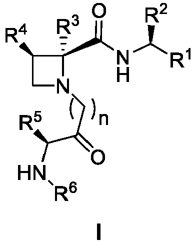 or a pharmaceutical acceptable salt thereof, wherein:
n is 0 or 1;
R¹ is a group selected from -CN, -CHO or -CH=CH-COOCH₂Ph;
R² is a -C₁-C₄ alkyl group which is optionally substituted by one -Cy₁;
Cy₁ is a 5 or 6 membered heterocycle, which is saturated, partially saturated, or aromatic, which comprises 1 or 2 heteroatoms in its structure selected from N, O or S, and which is optionally oxidized forming a -CO group in any available position.
R³ is selected from -CH₂-CH(Me)₂, -CH₂-Ph optionally substituted by -OH or -O-tBu, -C₁-C₄ alkyl substituted by -NHBoc or -NH₃Cl, 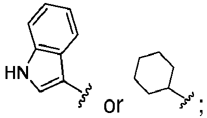
R⁴ is -C₁-C₄ alkyl;
R⁵ is a group selected from -CH(Me)₂, -C(Me)₃ or -C₁-C₄ alkyl substituted by -NHSO₂NHCO₂Me, -NHBoc or -NH₃Cl; and
R⁶ is selected from -COOMe, -COCH₂OPh-(4-Me), -COOCH₂CHF₂, -COCF₃, -COOCH₂Ph, -COOC (Me)₃, 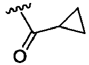 or -CONHC(Me)₃.

2. The compound of formula **I** according to claim 1, wherein R₂ is selected from a group methyl, ethyl, n-propyl, n-butyl,

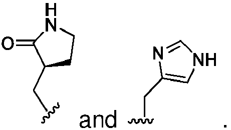

3. The compound of formula **I** according to any of claims 1 or 2, wherein R₄ is a methyl group.

4. The compound of formula **I** according to claim 1, selected from: 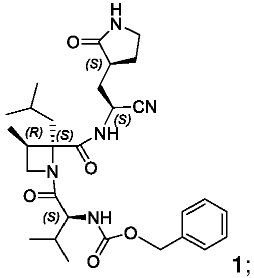 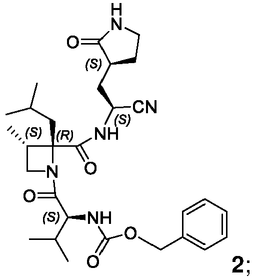 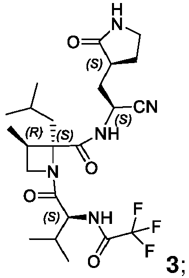 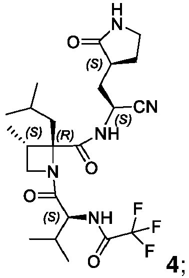 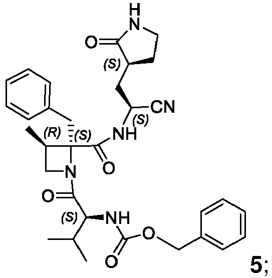 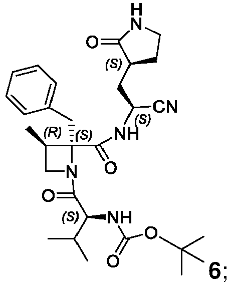 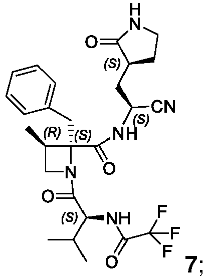 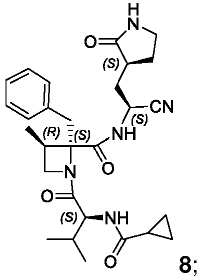 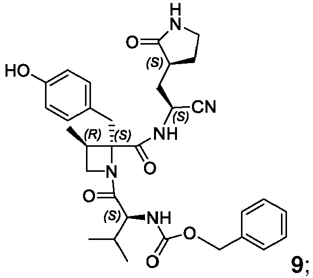 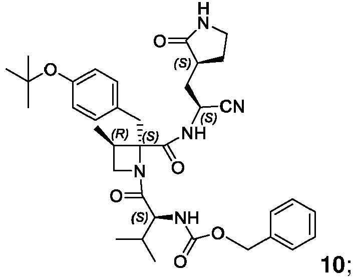 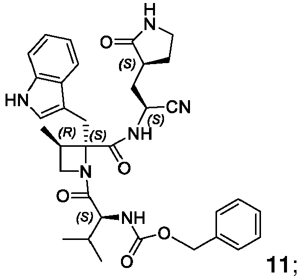 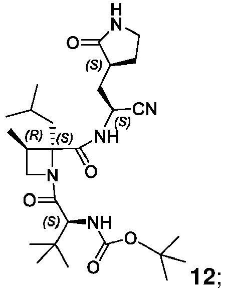 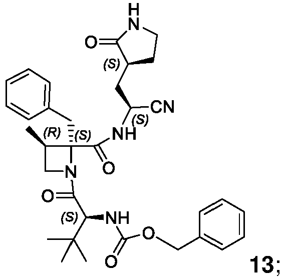 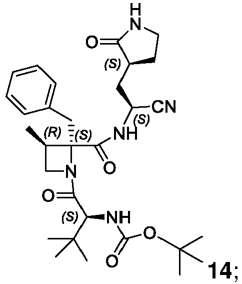 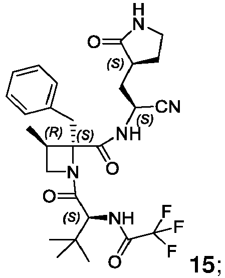 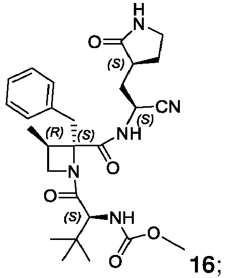 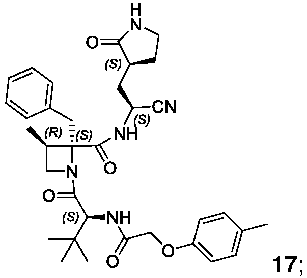 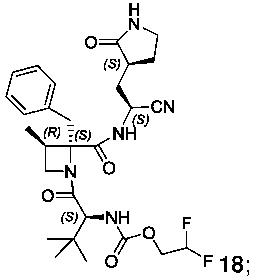 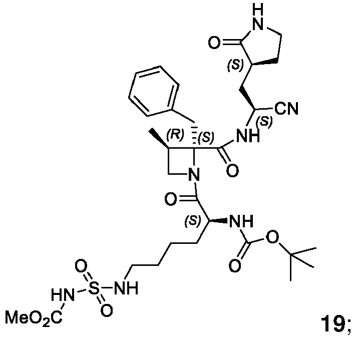 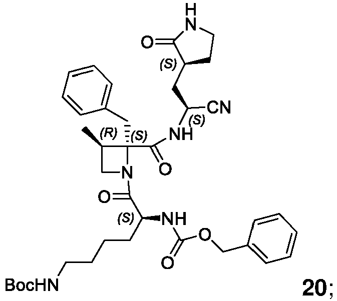 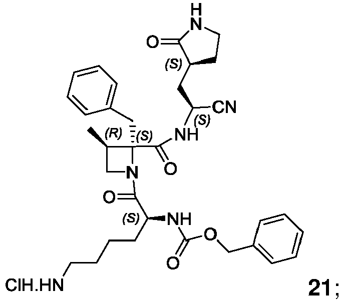 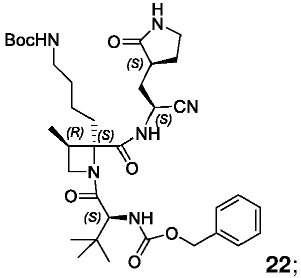 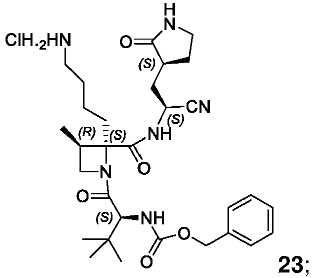 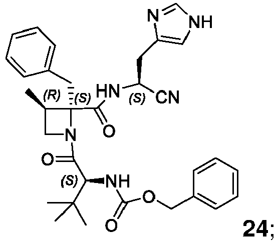 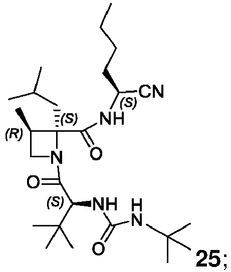 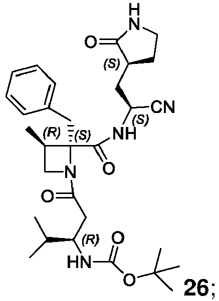 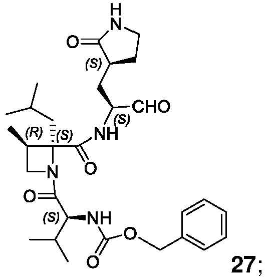 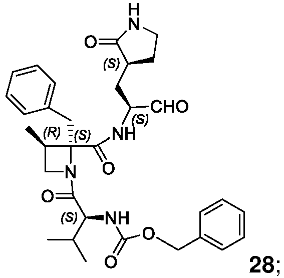 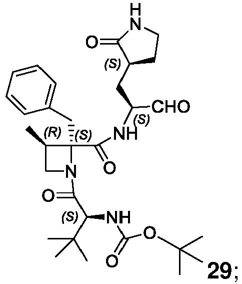 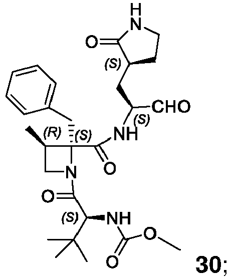 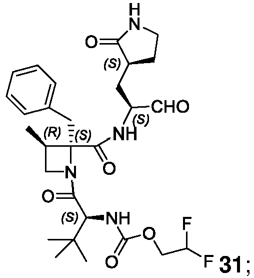 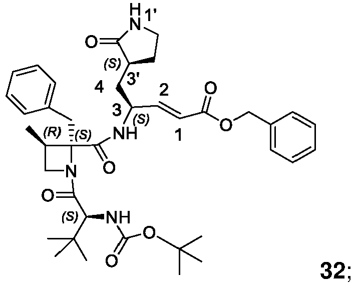 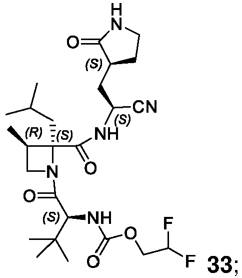 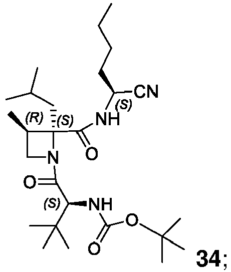 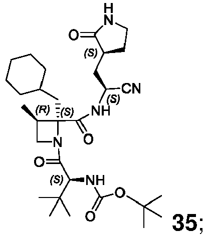 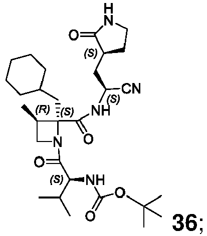 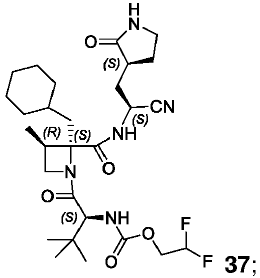 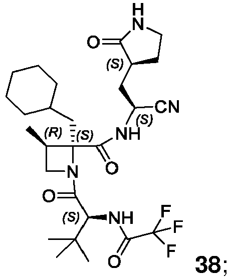 and

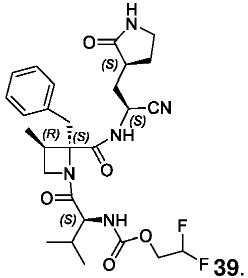

5. A pharmaceutical composition, which comprises a compound of formula I according to any of claims 1 to 4 or a pharmaceutically acceptable salt thereof and one or more pharmaceutically acceptable excipients.

6. A compound of formula **I** according to any of claims 1 to 4 or a pharmaceutical composition according to claim 5, for use as medicament.

7. A compound of formula **I** according to any of claims 1 to 4 or a pharmaceutical composition according to claim 5, for use for the treatment of a viral infection.

8. The compound or the pharmaceutical composition for their use according to claim 7, for the treatment of SARS-CoV-2 viral infection.
